# EUROPEAN PATENT APPLICATION

(11) **EP 4 763 969 A1**
(43) Date of publication of application: **24.06.2026**
(21) Application number: 24221097.9
(22) Date of filing: 18.12.2024
(51) Int. Cl.: C12M 3/06, C12M 1/00

(54) **BONE-ON-CHIP MICROFLUIDIC DEVICE**

(71) Applicant: CanChip GmbH, 14476 Potsdam (DE)
(72) Inventor: Madani, Ghazaleh, 14469 Potsdam (DE); Nejati, Omid, 14469 Potsdam (DE)
(74) Representative: Vossius & Partner Patentanwälte Rechtsanwälte mbB

(57) **Abstract**

The present invention relates to a microfluidic device comprising a main chamber configured to have a ring-like shape, at least one cell introduction port in fluidic connection with the main chamber, and at least one test port in fluidic connection with the main chamber. The present invention also relates to a method of producing the microfluidic device using digital light processing 3D printing, as well as methods and uses of the device for culturing cells and for generating bone micro tissue.

## Description

### Background

Organ-on-a-chip technologies have the potential to revolutionize preclinical drug testing by increasing throughput while minimizing the financial and ethical concerns associated with in vivo evaluation. Microfluidic-based tissue-on-a-chip devices are of considerable research interest and they have attracted attention for biomedical applications, such as drug development, because they can be used for small-volume, high-throughput studies of therapeutic effects on tissue mimics. From basic 2D cell cultures embedded in microfluidic devices microfluidic devices have evolved to utilize sophisticated 3D approaches with modern designs aimed at rewriting the dynamic and mechanical environment of the native tissue.

Since its inception, many efforts have been made to develop these devices for many organ types, but so far bone has been relatively neglected. Bone on-chip models are relevant for the study of bone physiology, diseases, and remodeling processes. While there are several bone-on-a-chip models that attempt to address the cellular components of bone, these models do not capture the chemical and structural properties of bone tissue. The bone on chip systems have employed different methods such as lithography and PDMS. However, PDMS has been shown to demonstrate drug absorption as a disadvantage. Further, microfluidic devices until now were not focused on the specific applications such as fraction healing and inflammation cocktail usage.

It is an object of the present invention to provide a microfluidic device which provides an improvement over known microfluidic devices described above and addresses the problems associated therewith.

### Summary

The present invention relates to a microfluidic device comprising a main chamber configured to have a ring-like shape, at least one cell introduction port in fluidic connection with the main chamber, and at least one test port in fluidic connection with the main chamber. The present invention also relates to a method of producing the microfluidic device using digital light processing 3D printing, as well as methods and uses of the device for culturing cells and for generating bone micro tissue.

### Brief Description of the Drawings

The subject matter of the invention will be explained in more detail in the following text with reference to preferred exemplary and non-limiting embodiments which are illustrated in the attached drawings. These figures disclose embodiments of the invention for illustrational purposes only. In particular, the disclosure provided by the figures and description is not meant to limit the scope of protection conferred by the invention.
**Fig. 1** illustrates an example of the microfluidic device according to a preferred embodiment of the present invention;
**Fig. 2** schematically depicts the fluidic pathways of the microfluidic device of Fig. 1;
**Fig. 3** schematically depicts the main chamber of the microfluidic device of Fig. 1;
**Fig. 4** is an example image taken of a prototype microfluidic device according to a preferred embodiment of the present invention;
**Fig. 5** is a schematic 3D rendering of the microfluidic device of Fig. 4 from an angled, top-down perspective;
**Fig. 6** is a schematic 3D rendering of the microfluidic device of Fig. 4 from an angled, bottom-up perspective;
**Fig. 7** is a timetable of Cells culture (0 - 35 days) and seeding in chip (ready to use chip);
**Fig. 8** illustrates culturing cells (Endothelial cells/HUVEC, Saos-2 cells or mesenchymal stem cells (MSC), and hematopoietic cells in the device to produce bone micro tissue. Subsequently, agents, such as an inflammatory cocktail, can be applied to the bone micro tissue to reflect bone fracture and/or further agents can be applied (such as immune cells/PBMC and/or (candidate) therapeutic agents); and
**Fig. 9** shows a workflow from producing the device (chip) to culturing cells in the device and assessing the cultured cells or cell culture.

### Detailed Description

The technical problem is solved by provision of the embodiments characterized in the claims and the embodiments described hereinbelow.

The present invention relates, inter alia, to the following items:
1. A microfluidic device comprising
   a main chamber configured to have a ring-like shape;
   at least one cell introduction port in fluidic connection with the main chamber; and
   at least one test port in fluidic connection with the main chamber.
2. The microfluidic device according to item 1, wherein the main chamber comprises two or more subchambers, the subchambers being arranged to form the ring-like shape.
3. The microfluidic device of item 2, wherein each of the subchambers has a polygonal shape, preferably wherein each of the subchambers has a hexagonal shape.
4. The microfluidic device of item 2 or item 3, wherein the main chamber comprises three or more subchambers, preferably four or more subchambers, more preferably six or more subchambers, most preferably six subchambers.
5. The microfluidic device of any one of items 2-4, wherein boundaries between adjacent subchambers are partially open allowing fluid and particle flow between adjacent subchambers.
6. The microfluidic device of any one of items 2-5, wherein each subchamber comprises a diameter of between 2 mm and 6 mm, preferably between 2.5 mm and 4 mm, most preferably between 3 mm and 3.5 mm.
7. The microfluidic device of any one of items 2-6, wherein each subchamber comprises a volume of between 10 mm³ and 94 mm³, preferably a volume of between 16 mm³ and 42 mm³, more preferably a volume of between 23 mm³ and 32 mm³.
8. The microfluidic device of any one of items 2-7, wherein each of the subchambers comprises multiple scaffold structures, each of the scaffold structures extending between a bottom surface of the subchamber to a top surface of the subchamber.
9. The microfluidic device of item 8, wherein each of the scaffold structures is shaped as a pillar having a polygonal cross-section, preferably wherein each of the scaffold structures is shaped as a pillar having a hexagonal cross-section.
10. The microfluidic device of item 8 or item 9, wherein each of the scaffold structures has a cross-sectional diameter within the range of 150 micrometers to 800 micrometers, preferably wherein each subchamber comprises at least one scaffold structure having a cross-sectional diameter within each of the following ranges: 150-250 µm, 250-450 µm, 450-650 µm; and 650-800 µm.
11. The microfluidic device of any of items 8-10, wherein each subchamber comprises multiple scaffold structures having different cross-sectional diameters, preferably at least six scaffold structures comprising at least two different cross-sectional diameters, preferably at least nine scaffold structures comprising at least three different cross-sectional diameters.
12. The microfluidic device of any of items 8-11, wherein each subchamber comprises between 3-18 scaffold structures, preferably between 6-15 scaffold structures, more preferably between 9-12 scaffold structures.
13. The microfluidic device of any one items 2-12, wherein each subchamber has a first fluid connection positioned in an outside wall of the ring-like main chamber and a second fluid connection positioned on an inside wall of the ring-like main chamber; wherein the first fluid connection fluidly connects the subchamber with the at least one cell introduction port and the at least one test port, and wherein the second fluid connection fluidly connects the subchamber with at least one further port.
14. The microfluidic device of any one of the previous items, wherein the total volume of the main chamber is between 62 mm³ and 562 mm³, preferably between 97 mm³ and 250 mm³, more preferably between 140 mm³ and 191 mm³.
15. The microfluidic device of any one of the previous items, wherein the main chamber comprises multiple scaffold structures, each of the scaffold structures extending between a bottom surface of the main chamber to a top surface of the main chamber.
16. The microfluidic device of any one of the previous items, wherein the device comprises at least two cell introduction ports.
17. The microfluidic device of any one of the previous items, wherein the device comprises at least two test ports.
18. The microfluidic device of any one of the previous items, wherein the device comprises at least one further port in fluidic connection with the main chamber, preferably wherein the at least one further port is positioned centrally within the ring-like shape of the main chamber.
19. The microfluidic device of any one of the previous items, wherein the microfluidic device comprises or is formed of biocompatible, transparent resin; preferably wherein the resin comprises at least one of the following: Asiga PlasCLEAR, Formlabs BioMed Clear, KeySplint Soft Clear, Loctite 3D IND405 Clear, Detax Freeprint^{®} Ortho Clear, NextDent Ortho Clear, Microfluidic Core Shell (µCS) Resin, Sartomer^{®} Clear Resins for Microfluidics, and Pro3dure Printodent GR-10 Resin; more preferably wherein the resin is Pro3dure Printodent GR-10 Resin.
20. The microfluidic device of any one of the previous items, wherein the microfluidic device is fabricated using 3D printing, preferably where the microfluidic device is fabricated using digital light processing (DLP) 3D printing.
21. A method of producing a microfluidic device, wherein the microfluidic device comprises a main chamber configured to have a ring-like shape, at least one cell introduction port in fluidic connection with the main chamber, and at least one test port in fluidic connection with the main chamber;
   preferably wherein the method includes 3D printing the microfluidic device using digital light processing (DLP) 3D printing.
22. The method of item 21, wherein the 3D printing method is performed using a biocompatible photocurable resin; preferably wherein the resin is completely transparent after curing; more preferably wherein the resin comprises at least one of the following: Asiga PlasCLEAR, Formlabs BioMed Clear, KeySplint Soft Clear, Loctite 3D IND405 Clear, Detax Freeprint^{®} Ortho Clear, NextDent Ortho Clear, Microfluidic Core Shell (µCS) Resin, Sartomer^{®} Clear Resins for Microfluidics, and Pro3dure Printodent GR-10 Resin; and most preferably wherein the resin is Pro3dure Printodent GR-10 Resin.
23. The method of item 21 or item 22, wherein the main chamber comprises two or more subchambers, the subchambers being arranged to form the ring-like shape.
24. The method of item 23, wherein each of the subchambers comprises multiple scaffold structures, each of the scaffold structures extending between a bottom surface of the subchamber to a top surface of the subchamber; and wherein the scaffold structures are DLP 3D printed.
25. The method of any one of items 21-24, wherein the microfluidic device is fabricated in a single DLP 3D printing process.
26. The method of any one of items 21-25, wherein the microfluidic device is unitarily formed.
27. A microfluidic device produced by the method of any one of items 21 to 25.
28. A method for culturing cells in the microfluidic device of any one of items 1-20 and 27.
29. The method of item 28, wherein the cells are cells of the bone tissue and/or cells which are capable of differentiating into cells of the bone tissue, preferably wherein the cells which are capable of differentiating into cells of the bone tissue are stem cells.
30. The method of item 28, wherein the cells are hematopoietic cells.
31. The method of any one of items 28 to 29, wherein the cells are bone cells or cells which are capable of differentiating into bone cells.
32. The method of item 31, wherein the bone cells are osteoblasts, osteocytes, osteoclasts and/or bone lining cells.
33. The method of item 31 or 32, wherein the cells which are capable of differentiating into bone cells are stem cells, preferably mesenchymal stem cells (MSCs).
34. The method of item 33, wherein the mesenchymal stem cells (MSCs) are obtained from bone marrow and/or from umbilical cord tissue (such as Wharton's jelly and/or the umbilical cord blood).
35. The method of item 31 or 32, wherein the cells which are capable of differentiating into bone cells are sarcoma osteogenic-2 (Saos-2) cells, preferably wherein the Saos-2 cells are capable of differentiating into osteocytes.
36. The method of any one of items 28 to 35, wherein the cells are endothelial cells or cells which are capable of differentiating into endothelial cells.
37. The method of item 36, wherein the cells which are capable of differentiating into endothelial cells are stem cells, preferably endothelial stem cells.
38. The method of item 36, wherein the cells which are capable of differentiating into endothelial cells are umbilical vein endothelial cells.
39. The method of item 38, wherein the umbilical vein endothelial cells are human umbilical vein endothelial cells (HUVECs).
40. The method of item 37, wherein the endothelial stem cells are obtained from bone marrow and/or from umbilical cord tissue (such as Wharton's jelly and/or the umbilical cord blood).
41. The method of any one of items 28 to 40, wherein the cells are hematopoietic cells or cells which are capable of differentiating into hematopoietic cells.
42. The method of item 41, wherein the cells which are capable of differentiating into hematopoietic cells are hematopoietic stem cells, preferably hematopoietic stem and progenitor cells (HSPCs).
43. The method of item 42, wherein the HSPCs are CD34⁺ HSPCs.
44. The method of any one of items 41 to 43, wherein the hematopoietic stem and progenitor cells (HSPCs) are obtained from (mobilized) peripheral blood, bone marrow and/or umbilical cord tissue (such as Wharton's jelly and/or the umbilical cord blood).
45. The method of any one of items 28 to 34 and 36 to 44, wherein the cells are obtained from a subject.
46. The method of item 45, wherein the subject is a human subject.
47. The method of any one of items 28 to 46, wherein the cells are human cells.
48. The method of any one of items 28 to 47,
   wherein the cells are healthy cells and/or obtained from a healthy subject; or
   wherein the cells are cells having characteristics of a bone disease or bone disorder and/or obtained from a subject suffering from a bone disease or bone disorder or suspected of suffering from a bone disease or bone disorder.
49. The method of any one of items 28 to 48, comprising the following steps in the order (A) to (C):
   (A) seeding the microfluidic device of any one of items 1-20 and 27 with cells as defined in any one of items 29 to 47;
   (B) culturing the cells; and
   Optionally (C) recovering the cells/cell culture and/or bone micro tissue or a cell culture having characteristics of bone micro tissue from the microfluidic device.
50. The method of item 49, wherein about 5,000-50,000 cells are introduced into the microfluidic device, thereby seeding the microfluidic device.
51. The method of item 49 or 50, wherein the microfluidic device is seeded with one or more cell type.
52. The method of item 51, wherein the microfluidic device is seeded solely with one cell type.
53. The method of any one of items 28 to 51, wherein one or more cell type is cultured in the microfluidic device.
54. The method of any one of items 28 to 53, wherein solely one cell type is cultured in the microfluidic device.
55. The method of any one of items 49 to 54, wherein
   bone cells or cells which are capable of differentiating into bone cells are seeded/cultured;
   wherein endothelial cells or cells which are capable of differentiating into endothelial cells are seeded/cultured; and/or
   wherein hematopoietic cells or cells which are capable of differentiating into hematopoietic cells are seeded/cultured.
56. The method of item 55, wherein the bone cells are osteoblasts, osteocytes, osteoclasts and/or bone lining cells.
57. The method of item 55 or 56, wherein the cells which are capable of differentiating into bone cells are stem cells, preferably mesenchymal stem cells (MSCs).
58. The method of item 57, wherein the mesenchymal stem cells (MSCs) are obtained from bone marrow and/or from umbilical cord tissue (such as Wharton's jelly and/or the umbilical cord blood).
59. The method of item 55 or 56, wherein the cells which are capable of differentiating into bone cells are sarcoma osteogenic-2 (Saos-2) cells, preferably wherein the Saos-2 cells are capable of differentiating into osteocytes.
60. The method of any one of items 55 to 59, wherein the cells are endothelial cells or cells which are capable of differentiating into endothelial cells.
61. The method of any one of items 55 to 59, wherein the cells which are capable of differentiating into endothelial cells are stem cells, preferably endothelial stem cells.
62. The method of item 61, wherein the wherein the cells which are capable of differentiating into endothelial cells are umbilical vein endothelial cells (UVECs).
63. The method of item 62, wherein the umbilical vein endothelial cells (UVECs) are human umbilical vein endothelial cells (HUVECs).
64. The method of any one of items 61 to 63, wherein the endothelial stem cells are obtained from bone marrow and/or from umbilical cord tissue (such as Wharton's jelly and/or the umbilical cord blood).
65. The method of any one of items 55 to 64, wherein the cells are hematopoietic cells or cells which are capable of differentiating into hematopoietic cells.
66. The method of item 65, wherein the cells which are capable of differentiating into hematopoietic cells are hematopoietic stem cells, preferably hematopoietic stem and progenitor cells (HSPCs).
67. The method of item 66, wherein the HSPCs are CD34⁺ HSPCs.
68. The method of any one of items 55 to 67,
   Wherein cells which are capable of differentiating into bone cells are seeded/cultured;
   wherein cells which are capable of differentiating into endothelial cells are seeded/cultured; and, optionally,
   wherein cells which are capable of differentiating into hematopoietic cells are seeded/cultured.
69. The method of item 68, wherein the cells which are capable of differentiating into bone cells are sarcoma osteogenic-2 (Saos-2) cells, preferably wherein the Saos-2 cells are capable of differentiating into osteocytes.
70. The method of item 68, wherein the cells which are capable of differentiating into bone cells are stem cells, preferably mesenchymal stem cells (MSCs).
71. The method of any one of items 55 to 67,
   Wherein bone cells are seeded/cultured;
   wherein cells which are capable of differentiating into endothelial cells are seeded/cultured; and
   wherein cells which are capable of differentiating into hematopoietic cells are seeded/cultured.
72. The method of item 71, wherein the bone cells are osteoblasts, osteocytes, osteoclasts and/or bone lining cells.
73. The method of any one of items 68 to 72, wherein the cells which are capable of differentiating into endothelial cells are endothelial stem cells, preferably human umbilical vein endothelial cells (HUVECs) and/or wherein the cells which are capable of differentiating into hematopoietic cells are hematopoietic stem cells, preferably hematopoietic stem and progenitor cells (HSPCs), more preferably CD34⁺ HSPCs.
74. The method of any one of item 29 to 73, wherein said cells capable of differentiating are cultured under conditions allowing differentiation.
75. The method of item 74,
   wherein said cells capable of differentiating differentiate into bone cells, endothelial cells and/or hematopoietic cells.
76. The method of any one of items 27 to 75, wherein the cells are cultured in the microfluidic device under conditions and/or for a time allowing the formation of bone micro tissue or a cell culture having characteristics of bone micro tissue.
77. The method of any one of items 27-76, comprising culturing the cells under conditions allowing attaching the cells to the (surface of the) microfluidic device.
78. The method of any one of items 27-76, comprising (preferably as step (B)(i)), culturing the cells for about 12 to about 48 hours, preferably for about 18 to about 48 hours, preferably about 24 hours in the microfluidic device.
79. The method of any one of items 27-78, wherein the cells are cultured in the microfluidic device over a constant flow of media, optionally supplemented with antibiotics, at a media flow rate of about 2 to about 10 µL/min, preferably about 4 µL/min.
80. The method of item 79, wherein the cells are cultured in the microfluidic device until bone micro tissue or a cell culture having characteristics of bone micro tissue has formed.
81. The method of any one of items 27-80, (preferably as step (B)(ii)), wherein the cells are cultured in the microfluidic device for about 1 week to about 6 weeks, preferably for about 1 week to about 2 weeks.
82. The method of any one of items 27to 81-30, wherein the method comprises (preferably prior to step (A)), a step (AA) comprising the following steps in the order (i) to (iii):
   (i) culturing the (to be seeded) cells in a medium in a humidified CO₂ buffered atmosphere,
   (ii) dissociating the (to be seeded) cells; and
   Optionally (iii) freezing the (to be seeded) cells.
83. The method of item 82, wherein step (i) is performed at a humidity of 85-95% and/or wherein step (i) is performed under a 4.5-6.5%, preferably 5% CO₂ buffered atmosphere and/or wherein step (i) is performed under a 20% O₂ atmosphere.
84. The method of item 82 or 83, wherein the cells are cultured in the at about 37°C for about 2 days to about 4 weeks (prior to seeding).
85. The method of any one of items 82 to 84, wherein the media is exchanged about every 2 days to about every 7 days.
86. The method of any one of items 82 to 85, wherein after step (iii) the cells are expanded and dissociated before being introduced into the microfluidic device (prior to seeding).
87. The method of any one of items 82 to 86, wherein the cells are dissociated using trypsin, preferably at a cell confluency of about 70 to about 85%.
88. Cells/cell culture and/or bone micro tissue or a cell culture having characteristics of bone micro tissue obtained or obtainable by the method of any one of items 27 to 87.
89. The method of any one of items 27 to 87, the method further comprising (preferably as step (CC)) contacting the cells/cell culture and/or bone micro tissue with a pro-inflammatory composition; or
   a method for producing cells/cell culture and/or bone micro tissue or a cell culture having characteristics of bone micro tissue, wherein the cells/cell culture and/or bone micro tissue has characteristics of a bone fracture, the method further comprising applying a pro-inflammatory composition to the cells/cell culture and/or bone micro tissue of item 88.
90. The method of item 89, comprising culturing the cells/cell culture and/or bone micro tissue under conditions and/or for a time allowing producing cells, bone micro tissue and/or a cell culture having characteristics of a bone fracture.
91. Cells, bone micro tissue and/or a cell culture having characteristics of a bone fracture obtained or obtainable by the method of item 89 or 90.
92. A method for analyzing a bone fracture and/or bone fracture healing comprising the method of any one of items 27 to 87 and 89 to 90.
93. A method for analyzing bone modeling and/or bone remodeling comprising the method of any one of items 27 to 87, 89 to 90 and 92.
94. The method of any one of items 27 to 87, 89 to 90 and 92, the method further comprising (preferably as step (D)) assessing the efficacy of a therapeutic agent or a candidate therapeutic agent comprising contacting the cells, bone micro tissue and/or cell culture with a therapeutic agent or a candidate therapeutic agent; or
   a method comprising assessing the efficacy of a therapeutic agent or a candidate therapeutic agent, the method comprising contacting the cells, bone micro tissue and/or a cell culture of item 91 with a therapeutic agent or a candidate therapeutic agent.
95. The method of item 94, wherein the therapeutic agent is an agent with known capacity to heal fracture or promote fracture healing; or wherein the candidate therapeutic agent is an agent with suspected capacity to heal fracture or promote fracture healing.
96. The method of item 94, wherein the therapeutic agent is an agent with known capacity to model and/or remodel bone; or wherein the candidate therapeutic agent is an agent with suspected capacity to model and/or remodel bone.
97. The method of any one of items 94 to 96, wherein the therapeutic agent or a candidate therapeutic agent is a drug and/or cell therapy.
98. The method of item 89 to 90 and 92 to 97, wherein the pro-inflammatory composition comprises or consists of cytokines or cytokine-like peptides in an aqueous solution, preferably wherein the pro-inflammatory composition comprises or consists of C3a, C5a, IL-1beta, IL-6, IL-8, and TNF-α in an aqueous solution, most preferably wherein the pro-inflammatory composition comprises or consists of 500 ng/mL C3a, 10 ng/mL C5a, 250 pg/mL IL-1beta, 500 pg/mL IL-6, 150 pg/mL IL-8, and 10 ng/mL TNF-α in an aqueous solution.
99. The method of item 97 or 98, wherein the cell therapy comprises stem cell therapy (iPSCs), CAR-T cell therapy, hematopoietic stem cell (HSC) therapy, mesenchymal stem cell (MSC) therapy, NK cell therapy, T-cell therapy and/or a cell therapy with human primary immune cells.
100. The method of any one of items 97 to 99, wherein the cell therapy comprises cells obtained from a subject.
101. The method of item 100, wherein the cells are allogenic, autologous or heterologous.
102. The method of any one of items 97 to 101, wherein the drug comprises an inhibitor or activator of bone formation, preferably wherein the drug comprises one or more of the bone formation inhibitors Sclerostin, Dkk1, C5a, a Histamin-receptor agonist, TNF-α, CCL3, or wherein the drug comprises one or more of the bone formation activators Wnt3a, Wnt1, PGE2, TGFb, or IL-17.
103. The method of any one of items 94 to 102, wherein the therapeutic agent or candidate therapeutic agent is a substance from a library.
104. The method of any one of items 94 to 103, wherein the therapeutic agent or candidate therapeutic agent is to be applied to the (cells, bone micro tissue and/or cell culture in the) microfluidic device.
105. The method of any one of items 27 to 87, 89 to 90 and 92 to 104, wherein the method is performed *in vitro.*
106. The method of any one of items 27 to 87, 89 to 90 and 92 to 105, wherein the method is not practiced on the human or animal body.
107. The method of any one of items 27 to 87, 89 to 90 and 92 to 106, wherein the method does not comprise a step of obtaining cells of a subject.
108. The method of any one of items 94, 97, 99 to 101 and 103 to 107, wherein the therapeutic agent is an agent with known efficacy against a bone disease; or wherein the candidate therapeutic agent is an agent with suspected efficacy against a bone disease.
109. The method of item 108, wherein the bone disease is selected from any one of bone fracture, osteoporosis, osteoarthritis, rheumatoid arthritis, Paget's disease of bone, osteogenesis imperfecta, rickets, osteomalacia, fibrous dysplasia, hyperparathyroidism, or bone cancer.
110. The method of item 109, wherein the bone cancer is selected from osteosarcoma, Ewing's sarcoma, chondrosarcoma, multiple myeloma, fibrosarcoma, chordoma.
111. Method for producing a microfluidic device comprising cells, bone micro tissue or a cell culture having characteristics of bone micro tissue, the method comprising the method of any one of items 27 to 87, 89 to 90 and 92 to 110.
112. A microfluidic device comprising cells, bone micro tissue or a cell culture having characteristics of bone micro tissue,
   wherein the microfluidic device comprising cells is produced by the method of item 111;
   or wherein the cells, bone micro tissue or a cell culture having characteristics of bone micro tissue are obtained or obtainable by the method of any one of items 27 to 87, 89 to 90 and 92 to 110.
113. A microfluidic device of any one of items 1-20 and 27, further comprising cells, bone micro tissue or a cell culture having characteristics of bone micro tissue.
114. Use of the microfluidic device of any one of items 1-20, 27, 112 or 113 for culturing cells.
115. Use of the microfluidic device of any one of items 1-20, 27, 112 or 113, or the cells, bone micro tissue or a cell culture having characteristics of bone micro tissue obtained or obtainable by the method of any one of items 27 to 87, 89 to 90 and 92 to 110 for establishing a model of bone micro tissue or a model of a bone disease or disorder or a model of a bone fracture.
116. Use of the microfluidic device of any one of items 1-20, 27, 112 or 113, or the cells, bone micro tissue or a cell culture having characteristics of bone micro tissue obtained or obtainable by the method of any one of items 27 to 87, 89 to 90 and 92 to 110 for assessing the effect of a therapeutic compound or a candidate therapeutic compound on bone modeling or bone remodeling, of a therapeutic compound or a candidate therapeutic compound on a bone disease or bone disorder, or of a therapeutic compound or a candidate therapeutic compound on a bone fracture.
117. Use of the microfluidic device of any one of items 1-20, 27, 75 or 76, or the cells of claim 73 for analyzing bone modeling or remodeling, for analyzing a bone disease or bone disorder, or for analyzing bone fracture and/or bone fracture healing.

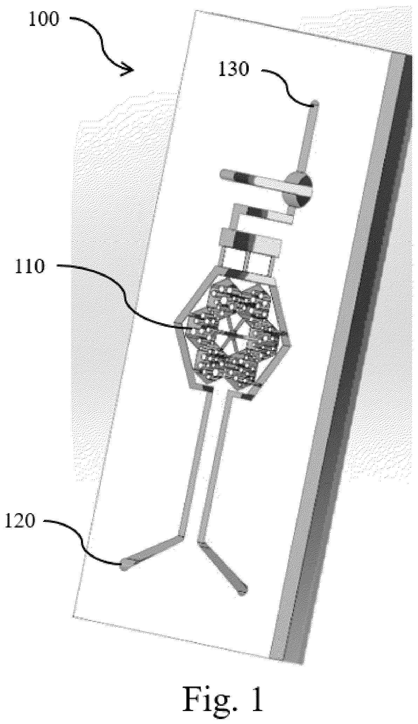

A microfluidic device according to a preferred embodiment of the present invention is depicted in Fig. 1. The microfluidic device 100 comprises a main chamber 110 which approximates a ring, at least one cell introduction port 120, and at least one test port 130, both of which are in fluidic connection with the main chamber 110. The microfluidic device 100 has been configured to have a 3D architecture and a physiologically relevant fluid shear stress (FSS) for an osteogenic device on a chip suitable for long-term culture.

This microfluidic device 100 can be used and simulated for studies of bone biology such as osteogenesis and diseases, for example, bone fracture, repair and bone healing, and osteoporosis studies. Within the microfluidic device 100 cells which are capable of differentiating into bone cells can be used to differentiate into bone cells, such as osteoblasts, osteocytes, osteoclasts and/or bone lining cells. By co-culturing bone cells (or cells which are capable of differentiating into bone cells) with endothelial cells (or cells which are capable of differentiating into endothelial cells) and, optionally, hematopoietic cells (or cells which are capable of differentiating into hematopoietic cells) in the device bone micro tissue can be formed. This can provide the ability to study the molecular processes of bone formation and their proliferation/differentiation into bone cells. Further, the present microfluidic device can be used in the studies of bone diseases by adding certain drugs to increase this differentiation or create stimuli such as creating inflammation with special cocktails/compositions and simulating fracture or osteoporosis. With this unique design, various cell compositions and different growth, inflammatory or immunological factors can be added and studied. This microfluidic device can be used to study the processes of bone repair with any type of disease background, to investigate the effects of drugs and immunotherapy on these processes.

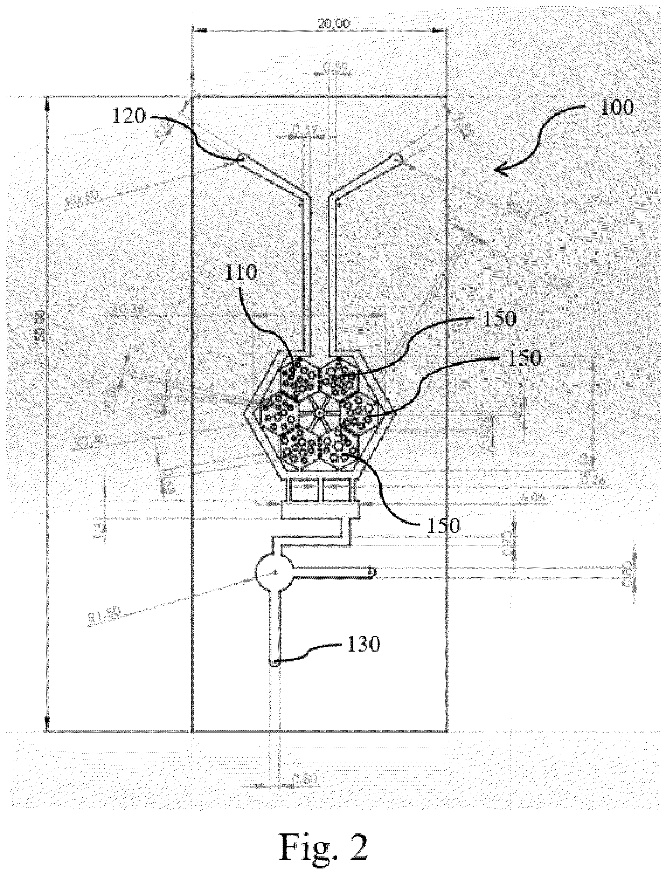

Fig. 2 shows a schematic depiction of the fluidic pathways of the microfluidic device 100. The main chamber 110 may be generally centrally located, with a cell introduction port 120 positioned on one side and the test portion 130 positioned on the opposite side of the main chamber 110. Microfluidic pathways connect the cell introduction port 120 with the main chamber, allowing a cell suspension to be introduced through the cell introduction port 120 into the main chamber 110. The microfluidic pathways in the proximity of the main chamber 110 are designed so as to equally introduce cells and media into a variety of portions of the main chamber 110. The microfluidic pathways may include a transport ring or partial transport ring positioned concentrically outside of the main chamber 110, to encourage a homogeneous introduction of cells and/or media into the main chamber 110.

A number of subchambers 150 are also depicted in Fig. 2. In this particular embodiment, the subchambers 150 have a hexagonal shape when viewed from above. In his embodiment, six hexagonal subchambers 150 are arranged to form the main chamber 110 having a ring-shape. The resulting polygonal ring-shape of the main chamber 110 contains an area equal to six hexagonal subchambers 150 as well as containing one, centrally-located hexagonal exclusion area within the middle of the main chamber 110. The total volume of the main chamber 110 is equivalent to total volume of all subchambers 150 added together. The total volume of the main chamber 110 may be between 62 mm³ and 562 mm³, preferably between 97 mm³ and 250 mm³, more preferably between 140 mm³ and 191 mm³. The main chamber can comprise a height of between 0.05 mm to 1.5 mm, for example between 0.06 mm to 1.4 mm, 0.07 mm to 1.3 mm, 0.08 mm to 1.2 mm, or 0.09 mm to 1.1 mm. Preferably, the main chamber comprises a height of 1.0 mm. The main chamber can comprise an area (including the area of the scaffold structures) of between 62 mm² and 562 mm², preferably between 97 mm² and 250 mm², more preferably between 140 mm² and 191 mm².While the subchambers 150 presented here are hexagonally shaped, the subchambers 150 can take on many other shapes and still achieve the same ring-shaped main chamber 110. The subchambers 150 may be polygonal, circular, or even irregularly shaped as long as, once assembled, the subchambers 150 form a ring-like shape. In the context of this invention a ring is defined as a roughly circular, toroidal shape or donut shaped, which does not include an area within the middle of the ring.

Although the present example has six hexagonal subchambers 150, the number of subchambers 150 is variable, and related to the shape of the subchambers 150. The main chamber 110 can comprise at least two subchambers, at least three subchambers, at least four subchambers, at least six subchambers, or up to eight subchambers. The hexagonal geometric shape of the subchambers does provide some unique advantages, as researchers can do more and better simulation in the 3D culture environment of the reaction chamber, to the trabeculae and bone spongy tissue for the implantation and niching of the primary cells. Also, this specific shape of the subchambers allows for more space on a micrometer scale for more implantation and contact to enable further studies, such as formation of six separate but related test spaces.

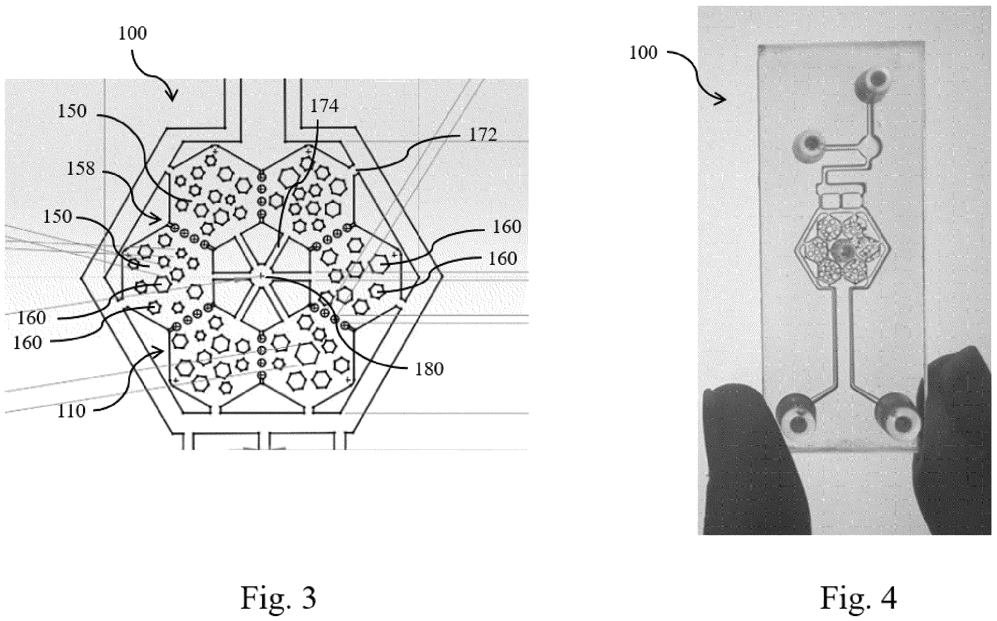

Fig. 3 provides a more detailed schematic view of the features of the main chamber 110. As with the previous embodiment, the main chamber 110 comprises a number of subchambers 150 which are arranged to form the ring-like shape of the main chamber 110. In some embodiments of the microfluidic device 100, the boundaries 158 between adjacent subchambers 150 are open, such that fluid and cellular material may pass between adjacent subchambers 150. Alternatively, the boundaries 158 between adjacent subchambers 150 may be partially open, such as depicted in Fig. 3, to allow a more limited passage of fluid and cellular material between adjacent subchambers 150. In this way fluid transport is enabled within and throughout the entire main chamber 110 to encourage equal distribution of materials. In a preferred aspect, all subchambers are identical, e.g. have the same shape (e.g. hexagonal), same diameter, same volume, same height and/or same area. The dimensions of each subchamber 150 can be defined by a diameter measured wall to wall across the center of the subchamber 150. Each subchamber 150 may comprise a diameter of between 2 mm and 6 mm, preferably between 2.5 mm and 4 mm, most preferably between 3 mm and 3.5 mm. Each subchamber can comprise a height of between 0.05 mm to 1.5 mm, for example between 0.06 mm to 1.4 mm, 0.07 mm to 1.3 mm, 0.08 mm to 1.2 mm, or 0.09 mm to 1.1 mm. Preferably, the subchamber comprises a height of 1.0 mm. Further, each subchamber 150 may comprise a volume of between 10 mm³ and 94 mm³, preferably a volume of between 16 mm³ and 42 mm³, more preferably a volume of between 23 mm³ and 32 mm³. Each subchamber can comprise an area (including the area of the scaffold structures) of between 10 mm² and 94 mm², preferably an area of between 16 mm² and 42 mm², more preferably an area of between 23 mm² and 32 mm².

The main chamber 110 or the subchambers 150, if present, may comprise a number of scaffold structures 160. The inclusion of scaffold structures 160 within microfluidic device 100 assists in mimicking the 3D microenvironment of bone tissue. In particular, the scaffold structure 160 may be sized and shaped to promote the development of an osteon-like structure within the microfluidic device 100. The scaffold structures 160 may be shaped as pillars which extend from the bottom surface to the top surface of the main chamber 120 or subchamber 150. A cross-section of the scaffold structure 160 taken in the plane of the microfluidic device 100 can be circular, polygonal, or preferably hexagonal. A diameter of the scaffold structure 160 is defined analogously to that of the subchamber, i.e. a distance measured in the cross-section of the scaffold structure 160, the distance being between two opposing surfaces and across the center of the scaffold structure 160. The diameter of the scaffold structures 160 may be within the range of 150 micrometers to 800 micrometers. For example, the diameter of the scaffold structures 160 may be within the range of 250 micrometers to 680 micrometers, within the range of 150-250 µm, within the range of 250-450 µm, within the range of 450-650 µm, within the range of 650-800 µm, and/or within the range of 400 micrometers to 500 micrometers. In one preferred embodiment each subchamber comprises at least one scaffold structure 160 having a diameter within each of the following ranges: 150-250 µm, 250-450 µm, 450-650 µm, 650-800 µm. When the scaffolding structures 160 are provided within subchambers 150, each of the subchambers 150 would comprise multiple scaffolding structure 160.

While all scaffolding structures 160 within the microfluidic device 100 may have the same dimensions, a preferred embodiment of the device 100 includes scaffolding structures 160 within each subchamber 150 which have a variety of different cross-sectional diameters. Such a configuration is evident within Fig. 3. Therein, the smallest scaffold structure is shown having wall-to-wall diameter of 250 micrometers, an intermediate scaffold structure is shown having a diameter of 360 micrometers, and the largest scaffold structure is shown having a diameter of 680 micrometers. One advantage of this configuration is that this type of design does not force the cells and their implantation to a regular and forced geometric growth, and instead by choosing small and large scaffold structure diameters in different places, it causes a difference in the volume of colonization, implantation and creation more akin to physiological bone structure.

It is envisaged that the scaffolding structures are randomly located within each subchamber, i.e. spatial distribution of the scaffolding structures in each subchamber are random. Also the dimensions of the distribution of the scaffolding structures in each subchamber can be random within the range of 150 micrometers to 800 micrometers.

For example, 30 % of the scaffolding structures in each subchamber may comprise a diameter of between 150-250 µm, 35 % of the scaffolding structures in each subchamber may comprise a diameter of between 250-450 µm, 25% of the scaffolding structures in each subchamber may comprise a diameter of between 450-650 µm and 10% of the scaffolding structures in each subchamber may comprise a diameter of between 650-800 µm.

For example, if the number of scaffolding structures is 9-12, 3 to 4 of the scaffolding structures in each subchamber may comprise a diameter of between 150-250 µm, 3 to 4 of the scaffolding structures in each subchamber may comprise a diameter of between 250-450 µm, 2 to 3 of the scaffolding structures in each subchamber may comprise a diameter of between 450-650 µm, and 1 scaffolding structure in each subchamber may comprise a diameter of between 650-800 µm. The number, dimension and/or spatial distribution of the scaffolding structures in each subchamber may be identical (i.e. may be the same) or may be different.

As also shown in Fig. 3, the number of scaffolding structures 160 within each subchamber 150 can be variable. Each subchamber 150 may comprise between 3 and 18 scaffold structures, preferably between 6 and 15 scaffold structures, more preferably between 6 and 14, 7 and 13, 8 and 13, most preferably between 9 and 12 scaffold structures, e.g. each subchamber 150 may comprise 1, 2, 3, 4, 5, 6, 7, 8, 9, 10, 11, 12, 13, 14, 15, 16, 17 or 18 scaffold structures, and preferably 9, 10, 11, or 12. The scaffold structures can be distributed somewhat randomly within the subchamber 150, as depicted within Fig. 3. However, it may be advantageous to ensure a certain minimum distance between two scaffold structures 160, such as 50 micrometers or 100 micrometers between scaffold structures 160. This separation allows for effective fluid and particle transportation throughout the main chamber 110 and the subchambers 150 as well as reinforcing physiological FSS. Similarly, it may be advantageous to ensure a certain minimum distance of around 50 to 100 micrometers between any scaffold structure and the walls of the subchambers 150 or main chamber 110.

In an embodiment of the present invention, such as in Fig. 3, there are six subchambers 150 arranged in a ring-like shape to form the main chamber 110. In this configuration each subchamber 150 comprises two boundary walls, which are shared with adjacent subchambers 150. These boundary walls may be partially open or permeable, or fully open. Each subchamber 150 also comprises one inside wall positioned closer to the center of the ring-like main chamber 110. Further, each subchamber 150 comprises three outside walls which are positioned further away from the center of the ring-like main chamber 110 than the inside wall.

A first fluid connection 172 may be positioned in an outside wall of the ring-like main chamber 110. In some configurations each subchamber 150 may comprise its own first fluid connection 172 positioned in an outside wall of the subchamber 150. A second fluid connection 174 may be positioned on an inside wall of the ring-like main chamber 110. In some configurations each subchamber 150 may comprise its own second fluid connection 174 positioned in an inside wall of the subchamber 150. The first fluid connection 172 may aid in connecting the main chamber 110 or each subchamber 150 to both the test port(s) 130 as well as the cell introduction port(s) 120.

The microfluidic device 100 may also include a further port 180 positioned centrally within the ring-like main chamber 110. The further port 180 can be used for matrigel, gelatin, hydrogel, or collagen injection to support cell proliferation and growth. A mixture including cells and protein scaffolding may also be introduced into the main chamber 110 through the further port 180. The main chamber 110 may include a second fluid connection 174 in the inside wall to provide the fluid connection to the further port 180. Alternatively, each subchamber 150 may include a second fluid connection 174 in the inside wall to provide the fluid connection to the further port 180.

Fig. 4 is an image of a prototype microfluidic device according to a preferred embodiment of the present invention. As can be noted therefrom, the entire microfluidic device is preferably transparent to promote use of the device for various imaging studies. In particular, the present microfluidic device may be used to perform fluorescence microscopy studies to follow up drug development, qPCR to detect genes of interest, flow cytometry to recognize biomarkers, live cell imaging for observing cells after inflammation cocktail injection as well as after drug injection, and MTT assay to test the toxicity of the drugs used.

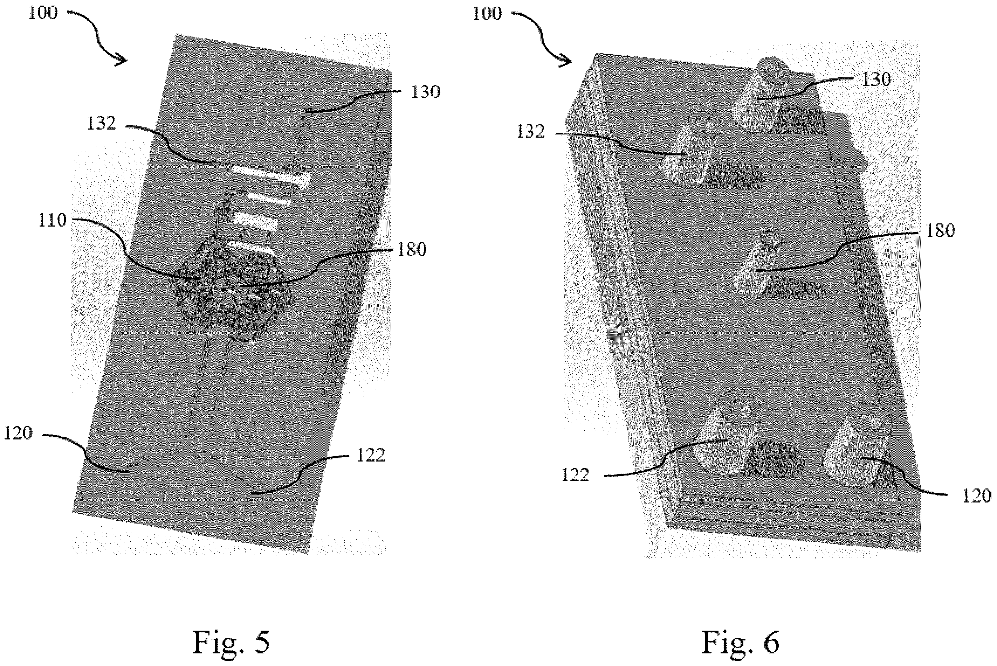

Fig. 5 and Fig. 6 are schematic 3D renderings of the microfluidic device 100 according to a preferred embodiment of the present invention. In some embodiments, the microfluidic device 100 includes a main chamber 110, a cell introduction port 120, a second cell introduction port 122, a further port 180, a test port 130, and a second test port 132.

While the microfluidic device 100 can be fabricated in a number of ways (such as molding, soft lithography, laser, Stereolithography (SLA)), it is preferable to form the microfluidic device 100 using 3D printing. In particular, the present device 100 can be fabricated using digital light processing (DLP) 3D printing methods. In contrast to other 3D printing methods, DLP 3D printing does not use a rastering method of the laser to polymerize the resin. Instead, a mask is used to polymerize each layer of the device simultaneously. This is particularly advantageous for the present device, given the level of detail of the main chamber 110 including the subchambers 150 as well as numerous scaffold structures 160 therewithin. As such, time and materials can be saved and the microfluidic devices 100 can be reliably produced.

DLP printing based on layer-by-layer vat photopolymerization can be used for microfabrication of the present device because of its high resolution of tens of microns, rapid processing speed, ease of operation, and versatility in resin materials. These advantages make the DLP technique favorable for the fabrication of the present microfluidic chip, which is expected to possess the desired resolution, optical transparency, smooth surfaces inside the channels, and sufficient mechanical stability to tolerate high pressure. The microfluidic chip devices as shown, for example, in Fig. 5 and Fig. 6 have been designed with SOLIDWORKS software, the design can be based on the needs of the study and how the microenvironment of the interested organ is in the body. The simulation has also been checked using SOLIDWORKS, to find the best flow for medium which does not harm the cells and at the same time is close to the body blood flow.

Resins particularly useful for producing the microfluidic device are biocompatible, transparent resins. Some resins useful for fabricating the microfluidic device include Asiga PlasCLEAR (clear photopolymer resin), Formlabs BioMed Clear (biocompatible clear resin), KeySplint Soft Clear (flexible splint resin), Loctite 3D IND405 Clear (industrial-grade clear resin), Detax Freeprint^{®} Ortho Clear (orthodontic clear resin), NextDent Ortho Clear (orthodontic clear resin), Microfluidic Core Shell (µCS) Resin (microfluidic resin), Sartomer^{®} Clear Resins for Microfluidics (clear microfluidic resin), and Pro3dure Printodent GR-10 Resin (biocompatible dental resin). In the present example provided in Fig. 4, the resin used was Pro3dure Printodent GR-10 Resin (biocompatible dental resin). This resin is completely transparent without any yellow tint and has not been shown to absorb any chemicals or drugs, which is not the case with PDMS, the resin most commonly used for microfluidic devices. The microfluidic device may be entirely produced in a single DLP 3D printing process. The microfluidic device can also be unitarily formed, and not formed of separate pieces which are subsequently joined together.

In the aforementioned figures and description, a microfluidic device according to the present invention has been discussed that includes a 3D architecture and physiologically relevant FSS for an osteogenic device on a chip suitable for long-term culture. It has the potential to provide more accurate, cheaper and faster methods to investigate new treatments for bone and bone fracture, compared to standard in vitro and in vivo tests and contribute to bone regeneration, which in turn can be used to develop bone regeneration strategies. While a number of unique features are presented herewith, some advantageous features of this microfluidic device include a unique chip design, integrated chip printing with an advanced DLP method, resin biomaterial with minimal drug absorption, and different biological applications to investigate bone fracture and its healing processes.

The invention also relates to a microfluidic device produced by the above described methods.

In one aspect, the present invention relates to a method for culturing cells in the microfluidic device as described herein.

In some embodiments, the cells in the method for culturing cells in the microfluidic device may be cells of the bone tissue and/or cells which are capable of differentiating into cells of the bone tissue, preferably wherein the cells which are capable of differentiating into cells of the bone tissue are stem cells.

In some embodiments, the cells in the method for culturing cells in the microfluidic device may be bone cells or cells which are capable of differentiating into bone cells.

In particular, the bone cells are osteoblasts, osteocytes, osteoclasts and/or bone lining cells.

Osteoblasts, as used herein, are cells involved in osteogenesis, remodeling, and healing. By synthesizing and secreting bone matrix osteoblasts maintain the structural integrity and shape of bone. Osteoblasts are derived from mesenchymal stem cells.

Osteocytes, as used herein, are cells derived from osteoblasts, or bone-forming cells, and are essentially osteoblasts surrounded by the products they secreted. Typical markers of osteoblasts and osteocytes include alkaline phosphatase, osteocalcin, and collagen type I.

Osteoclasts, as used herein, are cells involved in degradation of bone tissue to initiate bone remodeling and mediate bone loss. Osteoclasts are derived from precursors in the myeloid/monocyte lineage that circulate in the blood after their formation in the bone marrow.

Bone lining cells, as used herein, are resting, flat, spindle-shaped osteoblasts. Bone lining cells cover inactive (non-remodeling) bone surfaces and morphologically and physiologically separate the bone tissue from the bone marrow.

Osteogenesis, as used herein, relates to the two major modes of bone formation: intramembranous ossification and endochondral ossification.

Cells capable of differentiating into bone cells may be stem cells, preferably mesenchymal stem cells (MSCs). Mesenchymal stem cells (MSCs), also known as mesenchymal stromal cells, are cells capable of giving rise to multiple cell lineages including fibroblast, bone, adipocytes, and cartilage. The MSCs may be obtained from bone marrow and/or from umbilical cord tissue (such as Wharton's jelly and/or the umbilical cord blood). Preferably, they may be obtained from (adult) bone marrow. In one embodiment, the MSCs are human MSCs (hMSCs) and are obtained from human bone marrow aspirates, preferably of a healthy donor, with written informed consent.

Cells capable of differentiating into bone cells (or bone-like cells) may also be sarcoma osteogenic-2 (Saos-2) cells, preferably wherein the Saos-2 cells are capable of differentiating into osteocytes. Saos-2 cells are derived from an osteosarcoma patient. The cell-line shows an epithelial morphology and possesses osteoblastic features. Saos-2 cells can differentiate into osteocytes and/or osteocyte-like cells under certain conditions (e.g., by addition of Wnt3a and minerals to the cell culture media, such as McCoy's 5a Medium Modified) and as such produce a mineralized extracellular matrix, like natural bone tissue. Saos-2 are commercially available and/or deposited, e.g. at the DSMZ under DSMZ No. ACC243 or the ATCC under HTB-85.

In some embodiments, the cells in the method for culturing cells in the microfluidic device may be endothelial cells or cells which are capable of differentiating into endothelial cells. Endothelial cells are cells of mesodermal origin forming a single cell layer lining the interior walls of blood vessels, thereby regulating exchanges between the bloodstream and the surrounding tissues. The cells which are capable of differentiating into endothelial cells may be stem cells, preferably endothelial stem cells. Endothelial stem cells are multipotent stem cells capable of giving rise to endothelial cells. In one embodiment, the endothelial stem cells are obtained from the bone marrow and/or from umbilical cord tissue (such as Wharton's jelly and/or the umbilical cord blood). The cells which are capable of differentiating into endothelial cells may also be umbilical vein endothelial cells. In particular, the umbilical vein endothelial cells may be human umbilical vein endothelial cells (HUVECs).

In some embodiments, the cells in the method for culturing cells in the microfluidic device may be hematopoietic cells or cells which are capable of differentiating into hematopoietic cells. The cells which are capable of differentiating into hematopoietic cells may be hematopoietic stem cells, preferably hematopoietic stem cells and progenitor cells (HSPCs). The HSPCs may be CD34⁺ HSPCs. A population of HSPCs is considered to be CD34⁺ when ≥95% of cells express CD34 as assessed by flow cytometry using specific anti-CD34 antibodies. The HSPCs may be obtained from (mobilized) peripheral blood, bone marrow and/or umbilical cord tissue (such as Wharton's jelly and/or the umbilical cord blood). The term "hematopoietic stem and progenitor cells (HSPCs)" as used herein refers to cells capable of giving rise to different hematopoietic cell lineages. CD34 and CD45 are expressed by a majority of hematopoietic stem cells. The term "hematopoietic cells", as used herein, refers to differentiated, immune cells obtained from peripheral blood of subj ects, or to cells which have differentiated into hematopoietic cells, wherein the undifferentiated cells, which are capable of differentiating into hematopoietic cells, are obtained from bone marrow and/or umbilical cord tissue (such as Wharton's jelly and/or the umbilical cord blood). Hematopoietic cells in the context of this disclosure may be peripheral blood mononuclear cells (PBMC), such as lymphocytes, monocytes, natural killer cells (NK cells) or dendritic cells. In one embodiment, the hematopoietic cells may be hematopoietic CD45RA⁺ immune cells, in particular CD45RA⁺lymphocytes.

The term "differentiation", as used herein, relates to a change that occurs in cells to cause those cells to assume certain specialized functions and to lose the ability to change into certain other specialized functional units. Differentiation may be partial or complete with respect to mature adult cells. In the methods provided herein, the differentiation is preferably complete. Undifferentiated cells are cells which are capable of differentiating but have not yet undergone the differentiation process.

The term "stem cell", as used herein, relates to cells capable of self-renewal and of recreating functional tissues. Stem cells capable of differentiation may be any of totipotent, pluripotent, multipotent, oligopotent or unipotent stem cells. Totipotent stem cells are a stem cells capable of differentiating into any cell type of an organism's body, including germ line cells. Examples of totipotent cells include an embryonic stem cell, an embryonic germ cell, an inner cell mass (ICM)-derived cell, or a cultured cell from the epiblast of a late-stage blastocyst. A totipotent stem cell is able to develop into a complete organism. Pluripotent stem cells are stem cells capable of generating the three embryonic germ layers (endoderm, mesoderm, and ectoderm) and the cell lineages, tissues and organs originating from these layers. Multipotent stem cells are stem cells capable of forming multiple cell lineages generally derived from one embryonic germ layer. Oligopotent stem cells are a stem cells that can differentiate into only a few cells, such as lymphoid or myeloid stem cells. Unipotent stem cells are stem cells that can produce only one cell type, its own, but have the property of self-renewal which distinguishes it from non-stem cells (e.g., muscle stem cells). Induced pluripotent stem cell are pluripotent stem cells artificially derived from non-pluripotent cells. Non-pluripotent cells may be fully differentiated cells or cells whose potency to self-renew and differentiate is lower than that of pluripotent stem cells. Typically, induced pluripotent stem cells are derived from adult somatic cells.

The terms "culturing of cells" or "cell culture", as used herein, refer to a process of maintaining and growing cells under controlled conditions. The terms "culturing", "cultivating" or "growing" can be used interchangeably herein. Likewise, the term "cell"/"cells" includes the term "cell culture" and vice versa. The exact conditions may depend on the cell type. Conditions to be considered include specific growth media supplying essential nutrients (e.g., amino acids, vitamins, carbohydrates and/or minerals), growth factors, hormones, gases (e.g., O₂ and/or CO₂), temperature, humidity (85-95%), pH, osmotic pressure and/or sterile environment. Cells in culture may be adherent or in suspension. The cell types as provided herein may be cultured in conventional 2D culture or in 3D culture, i.e., in relation to 3D culture within the microfluid device. Cells in culture may proliferate and/or differentiate. Especially, (attached) cells cultured in conventional 2D culture may be expanded, dissociated (e.g., using trypsin), and subsequently harvested at 70-80% confluency. Suspension cells, e.g., lymphocytes, may also be expanded and harvested, however, they do not need dissociation. It is envisaged that cells capable of differentiation in conventional 2D culture can differentiate or start differentiating in 2D culture; however, it is preferred that the cells proliferate/expand in 2D culture without (substantial) differentiation and that the cells differentiate or start differentiation only in 3D culture (i.e. after seeding in the microfluidic device). In some embodiments, the cells capable of differentiating in the method for culturing cells in the microfluidic device are cultured under conditions allowing differentiation. The cells may be differentiated in conventional 2D culture and/or in the microfluid device, preferably in the microfluid device. Thus, the cells which are capable of differentiating into bone cells, the cells which are capable of differentiating into endothelial cells, and the cells which are capable of differentiating into hematopoietic cells can be introduced into the microfluid device as undifferentiated cells or as differentiated cells. If said cells are introduced into the microfluid device as differentiated cells, they are to be cultured under conditions allowing differentiation before introduction into the microfluid device. Preferably, the cells which are capable of differentiation are introduced into the microfluid device as undifferentiated cells. Said cells capable of differentiating differentiate into bone cells, endothelial cells and/or hematopoietic cells. Conditions allowing differentiation may, for example, be a cell culture medium supplemented with factors which induce differentiation or specific cell-cell-interactions.

The state, in which the growth area of the cell culture vessel is fully covered with cells is called "confluent". Therefore, 70% confluency refers to a state where the cells cover 70% of the growth area of a cell culture vessel. The cells may be frozen and stored at -80°C or used for further culture as described herein. The cell culture medium in which cells are cultured for cell expansion is referred to as (cell culture) growth medium. In contrast, cell culture medium which is used to induce differentiation of cells is referred to as differentiation medium.

"Expanding of the cells" as used herein, refers to maintaining the cells under conditions, as defined above, which allow proliferation of the cells, to obtain a larger and/or a certain number of cells. The process involves culturing and passaging the cells. "Passaging cells" is also known as subculturing and refers to a process of harvesting cells from a culture and transferring the cells to one or more culture vessels with fresh growth medium, which enables the further propagation of the cell line or cell strain. "Passage number" as used herein relates to the number of times the cells have been passaged in culture.

In general, the cell culture conditions described in the following apply to cells cultured in conventional 2D cell culture and to cells cultured within the microfluid device of the present invention (3D cell culture), unless specified otherwise.

Saos-2 cells may be cultured McCoy's 5a Medium Modified, (ATCC Catalog No. 30-2007). McCoy's 5A Medium is modified to contain 1.5 mM L-glutamine and 2200 mg/L sodium bicarbonate which is further supplemented with fetal bovine serum to a final concentration of 10%, penicillin/streptomycin (1%), and Glutamax^{™} (1%) (Glutamax is 200 mM L-alanyl-L-glutamine dipeptide in a 0.85% NaCl solution). This medium is a growth medium appropriate for Saos-2 cell culture. In a closed system (i.e., no media flow rate), the media may be refreshed every two to seven days, preferably every two days. Saos-2 cells are preferably maintained at 37°C, 5% CO₂, 85-95% humidity and 20% O₂.

Human bone marrow-derived mesenchymal stem cells (hBM-MSCs) or human mesenchymal stem cells (hMSC) generally may be cultured in in alpha-MEM media supplemented with platelet lysate (5%) (STEMCELL Technologies), penicillin/streptomycin (1%), and Glutamax^{™} (1%) (Glutamax is 200 mM L-alanyl-L-glutamine dipeptide in a 0.85% NaCl solution). This medium is a growth medium appropriate for hBM-MSCs cell culture. In a closed system (i.e., no media flow rate), the media may be refreshed every two to seven days, preferably every two days. hBM-MSCs are preferably maintained at 37°C, 5% CO₂, 85-95% humidity and 20% O₂. The terms "mesenchymal stem cells" and "mesenchymal stromal cells" can be used interchangeably herein. HUVECs may be cultured in EGM-2 media supplemented with 2% FBS, hFGF-B, VEGF, R3-IGF-1, ascorbic acid, hEGE, Heparin, and gentamicin (Endothelial Cell Growth Medium-2 (EGM-2); BulletKit, Lonza). This medium is a growth medium appropriate for HUVEC cell culture. In a closed system (i.e., no media flow rate), the media may be refreshed every two to seven days, preferably every three days. The cells are preferably maintained in a 2% gelatin-coated cell culture flask (Coming) at 37°C 5% CO₂, 85-95% humidity and 20% O₂.

Human CD34⁺ HSPCs may be cultured in serum-free HPC Expansion Medium XF (PromoCell GmbH, Heidelberg, Germany) supplemented with Cytokine-Mix E (containing TPO, SCF, Flt-3 ligand and IL-3) and 1% (v/v) penicillin/streptomycin. This medium is a growth medium appropriate for human CD34⁺ HSPCs cell culture. In a closed system (i.e., no media flow rate), the media may be refreshed every two to seven days, preferably every two days. Human CD34⁺ HSPCs are maintained at 37°C 5% CO₂, 85-95% humidity and 20% O₂.

Peripheral blood mononucleated cells (PBMCs) are isolated from peripheral blood of healthy donors or of subjects suffering from a bone fracture and/or a bone disease or disorder. Blood cells are separated from blood plasma by density gradient centrifugation, e.g., using Ficoll plague or the like. PBMCs may be cultured in RPMI 1640 supplemented with 10% FBS, 1% Penicillin-Streptomycin, and 2 mM L-glutamine. This medium is a growth medium appropriate for PBMC cell culture. In a closed system (i.e., no media flow rate), the media may be refreshed every two to seven days, preferably every two days. PBMCs are maintained at 37°C 5% CO₂, 85-95% humidity and 20% O₂. PBMCs may be sorted using markers specific for each blood cell type as described below.

In some embodiments, the cells in the method for culturing cells in the microfluidic device are obtained from a subject. For the purposes of the methods provided herein, the "subject" (or "patient") may be a mammal. In the context of the present invention, the term "subject" includes both humans and other mammals. Thus, the herein provided methods are applicable to both human and animal subjects, i.e., the method can be used for medical and veterinary purposes. Accordingly, said subject may be an animal such as a mouse, rat, hamster, rabbit, guinea pig, ferret, cat, dog, sheep, bovine species, horse, camel, or primate. Most preferably herein the subject is human.

In some embodiments, the cells in the method for culturing cells in the microfluidic device are human cells. The cells may be primary cells obtained from a subject, e.g., PBMCs, bone marrow-derived cells, or umbilical cord tissue-derived cells, and/or the cells may be established and commercially available cell-lines, e.g., Saos-2 cells. Preferably, the cells are obtained from a subject, most preferably from a human subject. In some embodiments, the cells are healthy cells and/or obtained from a healthy subject; or the cells are cells having characteristics of a bone dieses or bone disorder and/or obtained from a subject suffering from a bone disease or bone disorder or suspected of suffering from a bone disease or bone disorder. The bone disease and/or bone disorder from which a subject is suffering or is suspected of suffering, is not particularly limited and may be any one of bone fracture, osteoporosis, osteoarthritis, rheumatoid arthritis, Paget's disease of bone, osteogenesis imperfecta, rickets, osteomalacia, fibrous dysplasia, hyperparathyroidism, or (non-)metastatic bone cancer (osteosarcoma), wherein the bone cancer is selected from osteosarcoma, Ewing's sarcoma, chondrosarcoma, multiple myeloma, fibrosarcoma, chordoma.

The subject (preferably human subject) or cohorts of (preferably human) subjects may be of any kind of age or biological sex (i.e. male or female). The subject or cohorts of subject may, for example, be solely male or solely female subject(s) or may include both male and female subject(s). Further, the human subject or cohort of human subject(s) may have any age and may be (a) child/children (from birth to puberty, approx. 0 to 12 years), (a) youth(s) (from puberty to adulthood, approx. 13-18 years) or an adult(s) (i.e. 18 years and older), e.g. newborn (0-2 months), infant (3-11 months), toddler (1 to 2 or 3 years old), preschooler (3-4 years old), school aged child (5-12 years), youth/teenager (approx. 13-18/19 years) or adults of between 18 to 24, 25 to 34, 35 to 44, 45 to 54, 55 to 64 or 65 or over. It is envisaged that the subject(s) or cohort of subjects may have a different lifestyle (e.g. smoking or diet) and/or a different medical history (e.g. diabetes or osteoporosis).

In some embodiments, the methods provided herein comprise the following steps in the order (A) to (C): (A) seeding the microfluidic device of the present invention with cells as defined herein; (B) culturing the cells; and optionally (C) recovering the cells/cell culture and/or bone micro tissue or a cell culture having characteristics of bone micro tissue from the microfluidic device.

The term "seeding", as used herein, refers to the administration and/or introduction of the cells into the microfluidic device, wherein at least a part of the cells will attach themselves within the microfluidic device thereby resulting in a permanent cell culture. Before step (A), i.e., seeding the microfluid device of the present invention with cells, the main chamber including the subchambers of the microfluid device may be treated to prepare the microfluid device for cell culture applications, e.g., to sterilize the microfluid device and/or to enhance the attachment of the cells within the microfluid device. The microfluid device may be sterilized, by using UV-radiation and alcohol suitable for sterilization, for example. In one embodiment, microfluid device may be treated with, i.e., injected with, 150 µL of a 20 µg/mL streptavidin (SA) solution (in PBS) for one hour at room temperature, (and, optionally, 20 µg/mL biotinylated anti-CD34 antibody (in PBS), which binds to streptavidin), and 100 µL of collagen I at a concentration of 0.05 mg/mL or, optionally, 20 µg/mL poly D-Lysine. In a preferred embodiment, the microfluid device is treated with 150 µL of a 20 µg/mL streptavidin (SA) solution (in PBS) for one hour at room temperature and 100 µL of collagen I at a concentration of 0.05 mg/mL.

In some embodiments, about 5,000-50,000 cells are introduced into the microfluid device, preferably about 25,000 cells, thereby seeding the microfluid device. The exact number of cells seeded into the microfluid device may depend on several factors, such as on the cell type, the exact experimental setup, the exact dimensions of the main chamber and subchambers or the like. The microfluid device is seeded with cells by injection of the cells into the cell introduction port 120 of the microfluid device, wherein the cells are suspended in an appropriate volume of cell culture medium. An appropriate volume of cell culture medium may depend on the exact dimensions of the main chamber and subchambers. In some embodiments, the microfluid device is seeded with cells resuspended in 10 to 200 µL of cell culture medium, preferably in 10 to 70 µL of cell culture medium.

The cells can be introduced into the microfluid device on their own or in combination with a matrix, such as matrigel, gelatin, hydrogel, or collagen. The matrix may be constructed from synthetic or natural material, preferred herein are natural materials, which include in particular different derivates of the extracellular matrix, such as protein based materials (e.g., matrigel, gelatin, hydrogel, or collagen) and polysaccharidic materials (e.g., chitosan or glycosaminoglycans (GAGs)). Cells and the matrix (matrigel) can be mixed at a ratio of 3:1, 2:1, 1:1, 1:2, or 1:3, preferably at a ratio of 1:1 or 1:2, more preferably a ratio 1:1, cell solution and matrix (matrigel). Matrigel is a solubilized basement membrane preparation extracted from the Engelbreth-Holm-Swarm (EHS) mouse sarcoma, a tumor rich in such ECM proteins as laminin (a maj or component), collagen IV, heparan sulfate proteoglycans, entactin/nidogen, and a number of growth factors. Fibrin gel as such is a biodegradable polymer. It can be produced from the patient's own blood and used as an autologous matrix for the seeded fibroblasts without the potential risk of a foreign body reaction.

As used herein, a ratio of x:y refers to x units/parts of one material and y units/parts of another material, resulting in a total of x+y units/parts. For example, a 1:1 ratio of cells: matrix refers to 1 unit/part of cells and 1 units/parts of matrix or a 2:3 ratio of bone cells:endothelial cells refers to 2 parts of bone cells and three parts of endothelial cells. The conditions, e.g., passage of cells, volume of medium, appropriate cell culture growth medium, concentration of total cells in medium, and/or matrix may apply when one cell type and when more than one cell type is introduced into the microfluid device.

About 5,000-50,000 cells of the bone tissue and/or cells which are capable of differentiating into cells of the bone tissue, preferably Saos-2 cells, may be introduced/seeded into the microfluid device, preferably about 10,000 cells, preferably Saos-2 cells, are introduced/seeded into the microfluid device. The cells, preferably Saos-2 cells, are introduced into the microfluid device at passage 3 or 4. The cells, preferably Saos-2 cells, are introduced into the microfluid device in the appropriate cell culture growth medium, which is described above. Preferably 20 µL of cells, preferably Saos-2 cells, in appropriate cell culture growth medium at a concentration of about 500,000 cells per mL are introduced into the microfluid device. The cell culture growth medium may be replaced by an osteoblast mineralization medium about five to ten days, preferably seven days, after introduction of the cells, preferably Saos-2 cells, into the microfluid device. The osteoblast mineralization medium is for example the osteoblast mineralization medium C-27020 supplied by PromoCell.

About 5,000-50,000 cells of the bone tissue (bone cells) and/or cells which are capable of differentiating into cells of the bone tissue (bone cells), preferably human bone marrow mesenchymal stem cells (hBM-MSCs), may be introduced/seeded into the microfluid device, preferably about 10,000 cells, preferably hBM-MSCs, are introduced/seeded into the microfluid device. The cells, preferably hBM-MSCs, are introduced into the microfluid device at passage 3 to 5. The cells, preferably hBM-MSCs, are introduced into the microfluid device in the appropriate cell culture medium, which is described above. Preferably 20 µL of cells, preferably hBM-MSCs cells, in appropriate cell culture medium at a concentration of about 500,000 cells, preferably hBM-MSCs, per mL are introduced into the microfluid device. The cell culture growth medium may be replaced by an osteogenic medium about five to ten days, preferably seven days, after introduction of the cells, preferably hBM-MSCs, into the microfluid device. The osteogenic medium corresponds to the growth medium for hBM-MSCs (i.e., in alpha-MEM media supplemented with platelet lysate (5%) (STEMCELL Technologies), penicillin/streptomycin (1%), and Glutamax^{™} (1%) (Glutamax is 200 mM L-alanyl-L-glutamine dipeptide in a 0.85% NaCl solution), which is additionally supplemented with 0.1 mM of ascorbic acid, 10 mM of β-glycerophosphate, 0.1 µM of dexamethasone, and 10 nM of 1,25-dihydroxy vitamin D3 to initiate osteogenesis.

About 5,000-30,000 endothelial cells or cells which are capable of differentiating into endothelial cells, preferably HUVECs, may be introduced/seeded into the microfluid device, preferably about 15,000 endothelial cells or cells which are capable of differentiating into endothelial cells, preferably HUVECs, are introduced/seeded into the microfluid device. The cells, preferably HUVECs, are introduced into the microfluid device at passage 3 or 4. The cells, preferably HUVECs, are introduced into the microfluid device in the appropriate cell culture medium, which is described above. Preferably 50 µL of cells, preferably HUVECs, in appropriate cell culture medium at a concentration of about 300,000 cells per mL are introduced into the microfluid device. About 5,000-50,000 HSPCs (preferably CD34⁺ HSPCs) may be introduced/seeded into the microfluid device, preferably about 25,000, more preferably about 10,000 HSPCs (preferably CD34⁺ HSPCs) are introduced/seeded into the microfluid device. The CD34⁺ HSPCs are introduced into the microfluid device directly after isolation from the appropriate source and sorting. The CD34⁺ HSPCs are introduced into the microfluid device in the appropriate cell culture medium, which is described above. Preferably 10 µL of hematopoietic cells or cells which are capable of differentiating into hematopoietic cells (preferably HSPCs, more preferably CD34⁺ HSPCs) in appropriate cell culture medium at a concentration of about 1,000,000 cells (preferably HSPCs, more preferably CD34⁺ HSPCs) per mL are introduced into the microfluid device.

About 5,000-50,000 hematopoietic cells or cells which are capable of differentiating into hematopoietic cells (preferably PBMCs) may be introduced/seeded into the microfluid device, preferably about 25,000, more preferably about 10,000 cells (preferably PBMCs) are introduced/seeded into the microfluid device. Preferably 10 µL of cells (preferably PBMCs) in appropriate cell culture medium, as described above, at a concentration of 1,000,000 cells (preferably PBMCs) per mL are introduced into the microfluid device.

In some embodiments, the microfluid device is seeded solely with one cell type. In one embodiment, the microfluid device is seeded solely with one cell type, wherein the microfluid device is seeded with bone cells or cells which are capable of differentiating into bone cells. In one embodiment, the microfluid device is seeded solely with one cell type, wherein the microfluid device is seeded with endothelial cells or cells which are capable of differentiating into endothelial cells. In one embodiment, the microfluid device is seeded solely with one cell type, wherein the microfluid device is seeded hematopoietic cells or cells which are capable of differentiating into hematopoietic cells.

In some embodiments, one or more cell type is cultured in the microfluid device. In some embodiments, three cell types are cultured in the microfluid device. In some embodiments, two cell types are cultured in the microfluid device. This process of culturing two or more cell types simultaneously in the microfluid device is also referred to as co-culture. In the sense of the present invention, it is particularly advantageous to combine different cell types within the microfluid device to better mimic the situation of a physiological bone condition. In general, the cells co-cultured within a microfluid device, which is seeded in a method of seeding more than one cell type, may be cultured in a medium specific for only one cell type, or in a mixture of media, wherein each medium is specific for one cell type seeded into the microfluid device. If the cells are co-cultured in a mixture of media, the ratios are not particularly limited, the cells can be co-cultured in cell culture medium comprising equal parts of medium specific for a cell type. Or the ratio of the cell culture media specific for each cell type may correspond to the ratio of cells seeded into the microfluid device or any other ratio. For example, when Saos-2 cells and HUVECs are introduced/seeded into the microfluid device at a ratio of 2:3, the mixture of cell culture media comprises two parts of Saos-2 cell-specific medium and three parts of HUVEC-specific cell culture medium. Typically, the ratio of the cell-type specific media in the mixture of media (i.e. in the medium used for culturing the cells after seeding) corresponds to the ratio of the volume of the cell culture used for seeding. For example, if 20 µl Saos-2 cell culture, 50 µl HUVEC cell culture and, optionally, 10 µl HSPCs cell culture is used for seeding, the mixture of cell culture media may comprise two parts of Saos-2 cell-specific medium and five parts of HUVEC-specific cell culture medium (and optionally one part of HSPC specific cell culture medium). In one aspect, the mixture of cell culture media may comprise two parts of Saos-2 cell-specific medium and five parts of HUVEC-specific cell culture medium (and optionally one part of HSPC specific cell culture medium).

The terms "seeded" and "introduced" may be used interchangeably herein.

When two cell types (e.g. (i) bone cells or cells which are capable of differentiating into bone cells; and (ii) endothelial cells or cells which are capable of differentiating into endothelial cells) are co-cultured, the cells can be introduced/seeded into the microfluid device at any ratio, for example, at a ratio of 3:1, 2:1,1:1, 1:2, or 1:3, or 2:3, preferably at a ratio of 2:3 (e.g. ratio of bone cells or cells which are capable of differentiating into bone cells : endothelial cells or cells which are capable of differentiating into endothelial cells). The ratio can be calculated based on the cell number to be introduced/seeded.

When three cell types (e.g. (i) bone cells or cells which are capable of differentiating into bone cells; (ii) endothelial cells or cells which are capable of differentiating into endothelial cells; and (iii) hematopoietic cells or cells which are capable of differentiating into hematopoietic cells) are co-cultured, the cells can be introduced/seeded into the microfluid device at any ratio, for example, at a ratio of 3:1:1, 2:1:1,1:1:1, 1:2:1, or 1:3:1, or 2:3:2, preferably at a ratio of 2:3:2 (e.g. ratio of bone cells or cells which are capable of differentiating into bone cells : endothelial cells or cells which are capable of differentiating into endothelial cells : hematopoietic cells or cells which are capable of differentiating into hematopoietic cells). The ratio can be calculated based on the cell number to be introduced/seeded.

When two, three or more cell types are co-cultured, the term "ratio" can mean that the cell types are mixed and subsequently the mixture is introduced/seeded.

As mentioned herein above, terms like ratio of 2:3 or ratio of 2:3:2 as used herein refer to e.g. 2+3 parts or 2+3+2 parts, respectively, and the like.

The terms "cells of the bone tissue" and "bone cell" can be used interchangeably herein. Likewise, the terms "cells which are capable of differentiating into bone cells" and "cells which are capable of differentiating into cells of the bone tissue" can be used interchangeably herein.

In some embodiments, solely one cell type is cultured in the microfluid device. In one embodiment, solely one cell type is cultured in the microfluid device, wherein bone cells or cells which are capable of differentiating into bone cells are cultured in the microfluid device. In one embodiment, solely one cell type is cultured in the microfluid device, wherein hematopoietic cells or cells which are capable of differentiating into hematopoietic cells are cultured in the microfluid device.

In some embodiments, in the methods for culturing cells in the microfluidic device as provided herein, bone cells or cells which are capable of differentiating into bone cells are seeded/cultured; endothelial cells or cells which are capable of differentiating into endothelial cells are seeded/cultured; and/or hematopoietic cells or cells which are capable of differentiating into hematopoietic cells are seeded/cultured. In some embodiments, bone cells or cells which are capable of differentiating into bone cells are seeded/cultured; endothelial cells or cells which are capable of differentiating into endothelial cells are seeded/cultured; and/or hematopoietic cells or cells which are capable of differentiating into hematopoietic cells are seeded/cultured, wherein the bone cells are osteoblasts, osteocytes, osteoclasts and/or bone lining cells. In some embodiments, bone cells or cells which are capable of differentiating into bone cells are seeded/cultured; endothelial cells or cells which are capable of differentiating into endothelial cells are seeded/cultured; and/or hematopoietic cells or cells which are capable of differentiating into hematopoietic cells are seeded/cultured, wherein the cells capable of differentiating into bone cells are stem cells, preferably mesenchymal stem cells (MSCs). In some embodiments, the mesenchymal stem cells (MSCs) are obtained from bone marrow and/or from umbilical cord tissue (such as Wharton's jelly and/or the umbilical cord blood). In some embodiments, bone cells or cells which are capable of differentiating into bone cells are seeded/cultured; endothelial cells or cells which are capable of differentiating into endothelial cells are seeded/cultured; and/or hematopoietic cells or cells which are capable of differentiating into hematopoietic cells are seeded/cultured, wherein the cells which are capable of differentiating into bone cells are sarcoma osteogenic (Saos-2) cells, preferably wherein the Saos-2 cells are capable of differentiating into osteocytes.

In some embodiments, bone cells or cells which are capable of differentiating into bone cells are seeded/cultured; endothelial cells or cells which are capable of differentiating into endothelial cells are seeded/cultured; and/or hematopoietic cells or cells which are capable of differentiating into hematopoietic cells are seeded/cultured, wherein the cells are endothelial cells or cells which are capable of differentiating into endothelial cells. In some embodiments, bone cells or cells which are capable of differentiating into bone cells are seeded/cultured; endothelial cells or cells which are capable of differentiating into endothelial cells are seeded/cultured; and/or hematopoietic cells or cells which are capable of differentiating into hematopoietic cells are seeded/cultured, wherein the cells which are capable of differentiating into endothelial cells are stem cells, preferably endothelial stem cells. In some embodiments, the cells which are capable of differentiating into endothelial cells are umbilical vein endothelial cells (UVECs). Preferably, the umbilical vein endothelial cells (UVECs) are human umbilical vein endothelial cells (HUVECs). In some embodiments, the endothelial stem cells are obtained from bone marrow and/or from umbilical cord tissue (such as Wharton's jelly and/or the umbilical cord blood).

In some embodiments, bone cells or cells which are capable of differentiating into bone cells are seeded/cultured; endothelial cells or cells which are capable of differentiating into endothelial cells are seeded/cultured; and/or hematopoietic cells or cells which are capable of differentiating into hematopoietic cells are seeded/cultured, wherein the cells are hematopoietic cells or cells which are capable of differentiating into hematopoietic cells. In some embodiments, the cells which are capable of differentiating into hematopoietic cells are hematopoietic stem cells, preferably hematopoietic stem and progenitor cells (HSPCs). In some embodiments, the HSPCs are CD34⁺ HSPCs.

In some embodiments, in the methods for culturing cells in the microfluidic device as provided herein, cells which are capable of differentiating into bone cells are seeded/cultured; cells which are capable of differentiating into endothelial cells are seeded/cultured; and cells which are capable of differentiating into hematopoietic cells are seeded/cultured. In some embodiments, cells which are capable of differentiating into bone cells and cells which are capable of differentiating into endothelial cells are co-cultured in the microfluidic device under conditions and/or for a time allowing the formation of bone micro tissue or a cell culture having characteristics of bone micro tissue, wherein after formation of said bone micro tissue or a cell culture having characteristics of bone micro tissue, hematopoietic cells or cells which are capable of differentiating into hematopoietic cells are introduced into said microfluid device. In some embodiments, the cells which are capable of differentiating into bone cells are sarcoma osteogenic-2 (Saos-2) cells, preferably wherein the Saos-2 cells are capable of differentiating into osteocytes. In some embodiments, the cells which are capable of differentiating into bone cells are stem cells, preferably mesenchymal stem cells (MSCs). In some embodiments, the cells which are capable of differentiating into endothelial cells are endothelial stem cells, preferably human umbilical vein endothelial cells (HUVECs) and/or the cells which are capable of differentiating into hematopoietic cells are hematopoietic stem cells, preferably hematopoietic stem and progenitor cells (HSPCs), more preferably CD34⁺ HSPCs.

In some embodiments, in the methods for culturing cells in the microfluidic device as provided herein, bone cells are seeded/cultured; cells which are capable of differentiating into endothelial cells are seeded/cultured; and cells which are capable of differentiating into hematopoietic cells are seeded/cultured. In some embodiments, bone cells and cells which are capable of differentiating into endothelial cells are co-cultured in the microfluidic device under conditions and/or for a time allowing the formation of bone micro tissue or a cell culture having characteristics of bone micro tissue, wherein after formation of said bone micro tissue or a cell culture having characteristics of bone micro tissue, hematopoietic cells or cells which are capable of differentiating into hematopoietic cells are introduced into said microfluid device. In some embodiments, the bone cells are osteoblasts, osteocytes, osteoclasts and/or bone lining cells. In some embodiments, the cells which are capable of differentiating into endothelial cells are endothelial stem cells, preferably human umbilical vein endothelial cells (HUVECs) and/or the cells which are capable of differentiating into hematopoietic cells are hematopoietic stem cells, preferably hematopoietic stem and progenitor cells (HSPCs), more preferably CD34⁺ HSPCs.

The terms "bone micro tissue" and "bone tissue" can be used interchangeably herein. The term "bone micro tissue" as used hereinrefers to a three-dimensional (3D) construct that mimics the structural, mechanical, and biological properties of native bone on a micro scale. These tissues are often developed using advanced bioengineering techniques, such as organ-on-chip technology, which incorporates microfluidics to simulate the dynamic physiological environment of bone. This includes replicating critical features like the mineralized matrix, mechanical stress (e.g., fluid shear stress), and cellular interactions between osteoblasts (bone-forming cells), osteoclasts (bone-resorbing cells), and osteocytes (mature bone cells embedded within the matrix). Bone micro-tissues are particularly valuable in studying bone-related diseases (e.g., osteoporosis) and drug responses in a controlled and reproducible environment, offering a more accurate alternative to traditional 2D cultures and animal models

It is understood that if "bone micro tissue" forms in the device, cells/cell cultures may still be present which are not "bone micro tissue" or which do not have characteristics of "bone micro tissue", for example, cells that have not yet differentiated into bone cells, endothelial cells and/or hematopoietic cells. For the purposes of the invention, it suffices that "bone micro tissue" has formed to an extent sufficient for analyzing the bone micro tissue and/or the effect/efficacy of (candidate) compounds, e.g. (candidate) therapeutic compounds, as described herein. Preferably, solely/only "bone micro tissue" is present in the in the device, e.g. after "bone micro tissue" has formed. In other words, cells/cell cultures are not present (or present only *de minimis*) which are not "bone micro tissue" or which do not have characteristics of "bone micro tissue".

In some embodiments, the microfluid device is seeded with one or more cell type. In particular, the microfluid device may be seeded with bone cells or cells which are capable of differentiating into bone cells; endothelial cells or cells which are capable of differentiating into endothelial cells and hematopoietic cells or cells which are capable of differentiating into hematopoietic cells wherein the different cell types may be seeded at the same time, two cell types may be seeded at the same time and one cell type at a later time, or all cell types may be seeded one after the other. In particular, the microfluid device may be seeded with Saos-2 cells or hBM-MCSs, HUVECs and CD34⁺ HSPCs, wherein the different cell types may be seeded at the same time, two cell types may be seeded at the same time and one cell type at a later time, or all cell types may be seeded one after the other. In a preferred embodiment, the microfluid device is seeded with cells which are capable of differentiating into bone cells, such as Saos-2 cells or hBM-MCSs, and cells which are capable of differentiating into endothelial cells, such as HUVECs, at the same time and seeded with cells which are capable of differentiating into hematopoietic cells, such as CD34⁺ HSPCs, at a later time, preferably after bone micro tissue has formed. When the cells are seeded into the microfluid device at the same time, the cells may be premixed and injected simultaneously through one cell introduction port, or the cells may be injected individually through different cell introduction ports.

In one embodiment, bone cells or cells which are capable of differentiating into bone cells and endothelial cells or cells which are capable of differentiating into endothelial cells are seeded into and co-cultured in the microfluid device at the same time. In particular, Saos-2 cells and HUVECs are introduced into the microfluid device at the same time, preferably wherein a total of 25,000 cells is seeded into the microfluid device. Or hBM-MSCs and HUVECs are introduced into the microfluid device at the same time, preferably wherein a total of 25,000 cells is seeded into the microfluid device. The ratio between bone cells or cells which are capable of differentiating into bone cells and endothelial cells or cells which are capable of differentiating into endothelial cells is not limited and may be 4:3, 4:1, 3:2, 3:1, 2:1, 1:1, 1:2, 1:3, 2:3, 1:4 or 3:4. In a preferred embodiment, the ratio between bone cells or cells which are capable of differentiating into bone cells and endothelial cells or cells which are capable of differentiating into endothelial cells is 2:3. In particular, Saos-2 cells : HUVECs are introduced into the microfluid device at a ratio of 2:3 at the same time, wherein a total of 25,000 cells is introduced into the microfluid device (i.e., 10,000 Saos-2 cells and 15,000 HUVECs are introduced into the microfluid device). The Saos-2 cells and the HUVECs at a ratio of 2:3 may be introduced into the microfluid device alone or in combination with matrigel. Preferably, the Saos-2 cells and the HUVECs at a ratio of 2:3 are introduced into the microfluid device in a 1:1 solution of matrigel and cells. Or hBM-MSCs:HUVECs are introduced into the microfluid device at a ratio of 2:3 at the same time, wherein a total of 25,000 cells is introduced into the microfluid device (i.e., 10,000 hBM-MSCs and 15,000 HUVECs are introduced into the microfluid device). The hBM-MSCs and the HUVECs at a ratio of 2:3 may be introduced into the microfluid device alone or in combination with matrigel. Preferably, the hBM-MSCs cells and the HUVECs at a ratio of 2:3 are introduced into the microfluid device in a 1:1 solution of matrigel and cells.

The seeding and co-culturing of bone cells or cells which are capable of differentiating into bone cells, endothelial cells or cells which are capable of differentiating into endothelial cells, and optionally a matrix, into the same microfluid device results in the formation of vascularized bone micro tissue or a cell culture having the characteristics of bone microtissue.

In one embodiment, bone cells or cells which are capable of differentiating into bone cells and endothelial cells or cells which are capable of differentiating into endothelial cells are seeded into the microfluid device sequentially. In one embodiment, bone cells or cells which are capable of differentiating into bone cells are introduced into the microfluid device according to the methods described herein, the cells are differentiated into bone cells for about one to three weeks, preferably for about two weeks, according to the methods described herein, and after said about one to three weeks, preferably for about two weeks, of differentiation, endothelial cells or cells which are capable of differentiating into endothelial cells, preferably HUVECs, are introduced into the microfluid device. The HUVECs may be introduced into the microfluid device comprising bone cells in a 1:1 solution of fibrin gel and gelMA.

In some embodiments, bone cells or cells which are capable of differentiating into bone cells and endothelial cells or cells which are capable of differentiating into endothelial cells are seeded and co-cultured in the microfluid device in cell culture media specific for bone cells or cells which are capable of differentiating into bone cells, in cell culture media specific for endothelial cells or cells which are capable of differentiating into endothelial cells or in a mixture of cell culture media, wherein one medium is specific for bone cells or cells which are capable of differentiating into bone cells and one cell culture medium is media specific for endothelial cells or cells which are capable of differentiating into endothelial cells. In some embodiments, the cell culture medium, which specific for bone cells or cells which are capable of differentiating into bone cells, and the cell culture medium, which is specific for endothelial cells or cells which are capable of differentiating into endothelial cells, are mixed at a ratio of 4:3, 4:1, 3:2, 3:1, 2:1, 1:1, 1:2, 1:3, 2:3, 2:4, or 3:4. The cell culture medium specific for bone cells or cells which are capable of differentiating into bone cells can be growth medium, osteoblast mineralization medium, or osteogenic medium.

In some embodiments, the cells are cultured in the microfluidic device under conditions and/or for a time allowing the formation of bone micro tissue or a cell culture having characteristics of bone micro tissue.

Bones are made up of compact bone, spongy bone, and bone marrow. Compact bone makes up the outer layer of the bone. Spongy bone is found mostly at the ends of bones and contains red marrow. Bone marrow is found in the center of most bones and has many blood vessels. Trabeculae are the thin columns and plates of bone that create a spongy structure in a cancellous bone, which is located at the ends of long bones and in the pelvis, ribs, skull, and vertebrae. Bone is a mineralized connective tissue comprising four cell types: osteoblasts, bone lining cells, osteocytes, and osteoclasts. Additionally, hematopoietic stem cells and bone marrow stem cells can be found in the bone marrow, which is highly vascularized. Bone marrow refers to the soft, spongy tissue found in the center of most bones. The red bone marrow comprises hematopoietic stem cells which differentiate into blood cells, e.g., erythrocytes, immune cells (e.g., monocytes, lymphocytes, neutrophils, eosinophils, basophils, and macrophages) or platelets. The yellow bone marrow comprises fat and bone marrow stem cells which can differentiate into various cell types such, as adipocytes, bone cells or cartilage. The bone extracellular matrix (ECM), which is mainly synthesized and secreted by osteoblasts, contains organic, mainly collagens and other non-collagenous proteins involved in regulatory and structural functions, and inorganic compounds, mainly calcium-deficient apatite and trace elements. The bone micro tissue or the cell culture having characteristics of bone micro tissue in the microfluid device recreates the physiological structure and characteristics of bone and bone tissue.

Bone micro-tissue refers to a three-dimensional (3D) construct that mimics the structural, mechanical, and biological properties of native bone on a micro scale.

These tissues are often developed using advanced bioengineering techniques, such as organ-on-chip technology, which incorporates microfluidics to simulate the dynamic physiological environment of bone. This includes replicating critical features like the mineralized matrix, mechanical stress, and cellular interactions between osteoblasts (bone-forming cells), osteoclasts (bone-resorbing cells), and osteocytes (mature bone cells embedded within the matrix). Bone micro-tissues are particularly valuable in studying bone-related diseases (e.g., osteoporosis) and drug responses in a controlled and reproducible environment, offering a more accurate alternative to traditional 2D cultures and animal models.

The cells are cultured under conditions allowing attaching the cells to the (surface of the) microfluid device.

In some embodiments, the methods for culturing cells in the microfluidic device provided herein further comprise (i.e. following seeding) culturing the cells for about 12 to 48 hours, preferably for about 18 to about 48 hours, preferably for about 24 hours in the microfluidic device. This culturing of the cells in the microfluidic device for about 12 to 48 hours, preferably for about 18 to about 48 hours, preferably for about 24 hours, corresponds to a step (B)(i) after step (B), in a method comprising the following steps in the order (A) to (C): (A) seeding the microfluidic device as described herein with the cells as defined herein; (B) culturing the cells; and optionally (C) recovering the cells/cell culture and/or bone micro tissue or a cell culture having characteristics of bone micro tissue from the microfluidic device.

During this period of about 12 to 48 hours, preferably about 18 to about 48 hours, preferably about 24 hours, the cells attach to (the surface of) the microfluid device. The cells are cultured in the microfluid device in an appropriate volume of an appropriate medium, wherein the media may be refreshed if necessary. During attaching the cells are cultured in the microfluid device without a constant flow of the media.

In some embodiments, the cells are cultured in the microfluidic device for about one week to about six weeks, preferably for about one week to about two weeks. This culturing of the cells in the microfluid device for about one week to about six weeks, preferably for about one week to about two weeks, corresponds to a step (B)(ii), in a method comprising the following steps in the order (A) to (C): (A) seeding the microfluidic device as described herein with the cells as defined herein; (B) culturing the cells in the microfluid device, optionally (B)(i) culturing the cells for about 12 to 48 hours, preferably for about 18 to about 48 hours, preferably for about 24 hours, (B)(ii) culturing the cells for about one week to about six weeks, preferably for about one week to about two weeks; and optionally (C) recovering the cells/cell culture and/or bone micro tissue or a cell culture having characteristics of bone micro tissue from the microfluidic device. As mentioned, culturing for about 12 to 48 hours (step (B)(i)) allows attaching of the cells. Subsequently the cells may be (further) cultured for about one week to about six weeks (step (B(ii)). Culturing the cells in the microfluid device for another one to six weeks, preferably one to two weeks, (step (B)(ii)) allows for differentiation of the cells and/or allows for the formation of bone micro tissue or of a cell culture having characteristics of bone micro tissue. During the period of differentiation and/or formation of bone micro tissue or of a cell culture having characteristics of bone micro tissue, the cells are cultured in the microfluidic device over a constant flow of media. For the decision over which period the cells are to be cultured in step (B)(ii), quantitative and qualitative tests for endpoint analysis can be used. The primary endpoint outcomes are cell viability, cell differentiation, bone matrix deposition and mineralization. Those can be assessed, e.g., via flow cytometry, and staining, as described below.

In some embodiments, the cells are cultured in the microfluidic device over a constant flow of media, optionally supplemented with antibiotics, at a media flow rate of about 2 to about 10 µL/min. In a preferred embodiment, the cells are cultured in the microfluidic device over a constant flow of media, optionally supplemented with antibiotics, at a media flow rate of about 4 µL/min. The supplemented antibiotics are not limited, suitable examples include the combination of penicillin/streptomycin, wherein volume of the penicillin/streptomycin combination is 1% of the total media volume. The cells cultured in the microfluidic device over a constant flow of media are attached cells, e.g., during step (b)(ii). Thus, the cells are not cultured in the microfluid device over a constant media flow rate of about 2 to about 10 µL/min, preferably about 4 µL/min during the first 12 to 48 hours, preferably for the first 18 to about 48 hours, preferably for the first 24 hours (i.e., the cells are not cultured in the microfluid device over a constant media flow rate during step (B)i)). The media may be growth medium specific for one cell type, differentiation medium for one cell type and/or a mixture of media, wherein each medium in the mixture of media is growth or differentiation medium specific for one cell type.

In some embodiments, the cells are cultured in the microfluid device over a constant media flow rate of about 2 to about 10 µL/min, preferably about 4 µL/min until bone micro tissue or a cell culture having characteristics of bone micro tissue has formed. For formation of bone micro tissue or a cell culture having characteristics of bone micro tissue, often, a differentiation medium is suitable.

In some embodiments, the methods for culturing cells in the microfluidic device provided herein further comprise (preferably prior to step (A)), a step (AA) comprising the following steps in the order (i) to (iii): (i) culturing the (to be seeded) cells in a medium in a humidified CO₂ buffered atmosphere, (ii) dissociating the (to be seeded) cells; and optionally (iii) freezing the (to be seeded) cells. In some embodiments, step (i) is performed at a humidity of 85-95% and/or step (i) is performed under a 4.5-6.5%, preferably 5% CO₂ buffered atmosphere, and/or step (i) is performed under a 20% O₂ atmosphere. Accordingly, provided herein is a method comprising the following steps in the order (AA) to (C): (AA) (i) culturing the (to be seeded) cells in a medium in a humidified CO₂ buffered atmosphere, (ii) dissociating the (to be seeded) cells; and optionally (iii) freezing the (to be seeded) cells; (A) seeding the microfluidic device as described herein with the cells as defined herein; (B) culturing the cells in the microfluidic device, optionally (B)(i) culturing the cells for about 12 to 48 hours, preferably for about 18 to about 48 hours, preferably for about 24 hours, further optionally (B)(ii) culturing the cells for about 1 week to about 6 weeks, preferably for about 1 week to about 2 weeks; and optionally (C) recovering the cells/cell culture and/or bone micro tissue or a cell culture having characteristics of bone micro tissue from the microfluidic device. Optionally, the method further comprises a step (AA)(iv) after step (AA)(iii) and before step (A), wherein step (AA)(iv) comprises thawing, expanding, and dissociating the cells before introducing the cells into the microfluid device (prior to seeding), i.e., before step (A).

In some embodiments, step (AA)(i) is performed at a humidity of 85-95% and/or step (AA)(i) is performed under a 4.5-6.5%, preferably 5% CO₂ buffered atmosphere and/or step (AA)(i) is performed under a 20% O₂ atmosphere. The cells may be maintained under a 15-30% O₂ atmosphere, preferably under a 20% O₂ atmosphere. In some embodiments, steps (AA)(i) and/or (B) are performed at a humidity of 85-95% and/or steps (AA)(i) and/or (B) are performed under a 4.5-6.5%, preferably 5% CO₂ buffered atmosphere and/or steps (AA)(i) and/or (B) are performed under a 20% O₂ atmosphere. In some embodiments, in step (AA)(i) the cells are cultured in the at about 37°C for about 2 days to about 4 weeks (prior to seeding).

In general, the preferred temperature for culturing cells, particularly in steps (AA)(i) and/or (B) is 37°C. In some embodiments, in step (AA)(i) the media is exchanged about every 2 days to about every 7 days, preferably about every two or three days. The preferred timing of media exchange for every cell type is described above. In step (AA)(i), the cells may be cultured in cell culture vessels, such as T25 or T75 flasks. The flasks may be coated to improve culture conditions for the cells, e.g., HUVECs are maintained in 2% gelatin-coated flasks. Usually step (AA)(i) is performed in a conventional 2D culture. In principle, the cells are cultured in their respective media as described above and the respective media are exchanged/refreshed as described above. The cells are expanded until they are in a passage in which they can be used for the respective experiments. Which cell type is used in which passage is indicated above. In general, the attached cells can be dissociated using any appropriate enzymatic solution, such as a Trypsin/EDTA solution or the like. The cells are dissociated at a confluency of 60-85%, preferably of about 70-85%. Saos-2 and MSCs are usually dissociated and/or harvested at a confluency of about 70%. HUVECs are usually dissociated using trypsin and/or harvested at a confluency of about 80%. In a preferred embodiment, the cells are dissociated using trypsin at a cell confluency of about 70 to about 85%, preferably Saos-2 and/or MSCs are dissociated using trypsin at a cell confluency of about 70 % and/or HUVECs are dissociated using trypsin at a confluency of about 80%. The harvested cells may be frozen and stored at -80°C or immediately used for further culture as described herein. It is also possible to split the cells, e.g., seed microfluid device with one part, keep one part of the cells in conventional 2D culture and/or freeze one part of the cells, optionally wherein the parts do not need to be equal parts. In case, the cells are frozen and store at -80°C, the cells need to be thawed prior to seeding the microchip with the cells. After thawing, the cells are cultured according to step (AA)(i) and dissociated according to step (AA)(ii). Step (B) may not be performed immediately after step (AA)(iii) without an additional step (AA)(iv) of thawing, expanding and dissociating the to be seeded cells.

In one aspect, the present invention relates to cells/cell culture and/or bone micro tissue or a cell culture having characteristics of bone micro tissue obtained or obtainable by the methods provided herein.

In one aspect, the method for culturing cells in the microfluidic device provided herein further comprises (preferably as step (CC)) contacting the cells/cell culture and/or bone micro tissue with a pro-inflammatory composition (also termed herein "cocktail"); or it further comprises a method for producing cells/cell culture and/or bone micro tissue or a cell culture having characteristics of bone micro tissue, wherein the cells/cell culture and/or bone micro tissue has characteristics of a bone fracture, the method further comprising applying a pro-inflammatory composition to the cells/cell culture and/or bone micro tissue obtained or obtainable by the methods provided herein.

Step (CC) of the method follows step (B) and precedes step (C). Accordingly, provided herein is a method comprising the following steps in the order (AA) to (C): optionally (AA)(i) culturing the (to be seeded) cells in a medium in a humidified CO₂ buffered atmosphere, optionally (AA)(ii) dissociating the (to be seeded) cells; and optionally (AA)(iii) freezing the (to be seeded) cells; (A) seeding the microfluidic device of the present invention with cells as defined herein; (B) culturing the cells in the microfluidic device, optionally (B)(i) culturing the cells for about 12 to 48 hours, preferably for about 18 to about 48 hours, preferably for about 24 hours, further optionally (B)(ii) culturing the cells for about 1 week to about 6 weeks, preferably for about 1 week to about 2 weeks; (CC) contacting the cells/cell culture and/or bone micro tissue with a pro-inflammatory composition; or it further comprises a method for producing cells/cell culture and/or bone micro tissue or a cell culture having characteristics of bone micro tissue, wherein the cells/cell culture and/or bone micro tissue has characteristics of a bone fracture, the method further comprising applying a pro-inflammatory composition to the cells/cell culture and/or bone micro tissue obtained or obtainable by the methods provided herein; and optionally (C) recovering the cells/cell culture and/or bone micro tissue or a cell culture having characteristics of bone micro tissue having characteristics of bone fracture from the microfluidic device. In the method provided herein comprising steps (A) to (C), usually the cells are cultured in the microfluidic device over a constant flow of media at a media flow rate of about 2 to about 10 µL/min, preferably at a media flow rate of about 4 µL/min, during steps (B)(ii) and/or step (CC).

A proinflammatory composition as used herein comprises or consists of cytokines or cytokine-like peptides in an aqueous solution, preferably wherein the pro-inflammatory composition comprises or consists of C3a, C5a, IL-1beta, IL-6, IL-8, and TNF-α in an aqueous solution, most preferably wherein the pro-inflammatory composition comprises or consists of 500 ng/mL C3a, 10 ng/mL C5a, 250 pg/mL IL-1beta, 500 pg/mL IL-6, 150 pg/mL IL-8, and 10 ng/mL TNF-α in an aqueous solution. A pro-inflammatory composition comprising or consisting of 500 ng/mL C3a, 10 ng/mL C5a, 250 pg/mL IL-1beta, 500 pg/mL IL-6, 150 pg/mL IL-8, and 10 ng/mL TNF-alpha in an aqueous solution may mimic the physiological microenvironment of a bone fracture. The proinflammatory composition may mimic the physiological microenvironment of a bone disease and/or disorder and/or of a bone fracture. For example, the proinflammatory composition may mimic the physiological microenvironment of osteoporosis. These cocktails typically include cytokines like TNF-α, IL-6, and IL-1β, which mimic inflammatory environments, as well as glucocorticoids or other agents that disrupt bone remodeling by inhibiting osteoblast activity or promoting osteoclast genesis. The aqueous solution is/comprises water (H₂O) or PBS (phosphate buffer saline). In other words, the aqueous component of the solution is/comprises water (H₂O) or PBS (phosphate buffer saline).

In some embodiments, the method comprises culturing the cells/cell culture and/or bone micro tissue under conditions and/or for a time allowing producing cells, bone micro tissue and/or a cell culture having characteristics of a bone fracture.

In one aspect, the present invention relates to cells, bone micro tissue and/or a cell culture having characteristics of a bone fracture obtained or obtainable by the methods of the disclosure. For example, cells, bone micro tissue and/or a cell culture having characteristics of a bone fracture can be obtained by contacting the cells/cell culture and/or bone micro tissue with a pro-inflammatory composition; or by a applying a pro-inflammatory composition to the cells/cell culture and/or bone micro tissue produced as described herein; wherein the pro-inflammatory composition comprises or consists of 500 ng/mL C3a, 10 ng/mL C5a, 250 pg/mL IL-1beta, 500 pg/mL IL-6, 150 pg/mL IL-8, and 10 ng/mL TNF-α in an aqueous solution.

In one aspect, the present invention relates to a method for analyzing a bone fracture and/or bone fracture healing comprising the method of the present invention as described above.

In one aspect, the present invention relates to a method for analyzing bone modeling and/or bone remodeling comprising the method of the present invention as described above. In particular when the bone micro tissue reflects healthy bone tissue (e.g. when the cells are healthy cells or are obtained or derived/originate from healthy subjects) the method may be used to analyze bone modeling and/or bone remodeling, e.g. by contacting the bone micro tissue with (candidate) compounds and assessing the efficacy of (candidate) compounds on enhancing or decreasing modeling/remodeling.

The method may also be useful when the bone micro tissue reflects bone tissue having characteristics of a bone disease or bone disorder (e.g. when the cells are bone disease/disorders cells (or cells having characteristics of a bone disease/disorder or obtained or derived/originate from subjects having a bone disease or disorder). Also in this case the method may be used to analyze bone modeling and/or bone remodeling, e.g. by contacting the bone micro tissue with (candidate) compounds and assessing the efficacy of (candidate) compounds on enhancing or decreasing modeling/remodeling.

Remodeling of bone tissue is a continuous process in organisms aiming to maintain bone homeostasis by shaping and reshaping bones. *In vivo,* bone remodeling is orchestrated through the actions of bone cells, i.e., osteoblasts, osteocytes, osteoclasts and bone lining cells, in combination with local (e.g., growth factors and cytokines) and systemic (e.g., calcitonin and estrogens) factors. Bone remodeling is a highly complex process by which old bone is replaced by new bone, in a cycle comprised of three phases: (1) initiation of bone resorption by osteoclasts, (2) the transition (or reversal period) from resorption to new bone formation, and (3) the bone formation by osteoblasts. Bone modeling also refers to the process of increasing bone mass and maintaining or altering bone shape. This process of bone remodeling can be studied *in vitro* by analyzing the cells/cell culture and/or bone micro tissue or a cell culture having characteristics of bone micro tissue obtained cultured in the microfluid device of the present invention. Furthermore, the development of bone formation can be studied with the microfluid device, and the methods escribed herein, such that the present invention is also a suitable tool in the field of developmental biology.

The methods for analyzing a bone fracture and/or bone fracture healing and/or for analyzing bone modeling and/or bone remodeling are useful to study the cells within the chip and to allow a conclusion, e.g., they are useful when studying fracture healing using an inflammatory cocktail or when studying the effect of a drug on the cells. The methods for analyzing a bone fracture and/or bone fracture healing and/or for analyzing bone modeling and/or bone remodeling include but are not limited to gene expression profiling (e.g., transcriptomics, i.e., quantitative real-time polymer chain reaction (qRT-PCR) or (RNA)-sequencing), protein expression profiling (e.g., proteomics including mass spectrometry, flow cytometry, immunofluorescence staining or immunohistochemistry), cell matrix and mineralization staining and/or quantification of calcium deposition (e.g., Alizarin Red S (ARS) staining, von Knossa staining, alcian blue staining, or alkaline phosphatase activity (ALP) staining), quantification of cytokines and growth factors (e.g., enzyme-linked immunosorbent assay (ELISA) or multiplex detection assay, or Alamar blue assay using Deep blue cell viability reagent), cell proliferation studies (e.g., lactate dehydrogenase (LDH) assay, cell proliferation reagent (MTS assay)), cell viability assay (e.g., water soluble tetrazolium salt (WST)-assay or Calcein-AM/PI double staining), cell adhesion assay (e.g., CCK-8 solution), cellular morphology analysis, quantification of collagen synthesis (e.g., Direct Red 80 (DR80) staining), and live cell tracking and imaging by automated live cell imaging system (AutoLCI).

Cells/cell culture and/or bone micro tissue or a cell culture having characteristics of bone micro tissue can be characterized by analysis of specific marker expression. The expression of the cell type specific markers can be performed using flow cytometry, immunofluorescence staining, q(RT)-PCR, RNA sequencing or the like. This analysis may be performed to confirm whether the differentiation of the cells, which are capable of differentiating into bone cells, endothelial cells or hematopoietic cells, has occurred and, thus, to confirm whether bone micro tissue or a cell culture having characteristics of bone micro tissue has developed.

Specific markers for HUVECs, whose expression can be detected by flow cytometry, include but are not limited to, CD31 as a positive marker and CD34, CD14, and CD90 as negative markers. Thus, HUVECs may be CD31⁺ CD34⁻ CD14⁻ and CD90⁻ cells. The expression of HUVEC specific markers is preferably analyzed by flow cytometry and Immunohistochemistry (IHC).

Specific markers for Saos-2 cells, whose expression can be detected by flow cytometry, include, but are not limited to, epidermal growth factor (EGF) and transforming growth factor beta (type 1 and type 2). Thus, bone micro tissue or a cell culture having characteristics of bone micro tissue should not express the specific marker pattern of undifferentiated HUVECs and/or of Saos-2 cells.

Specific markers, whose expression can be detected by flow cytometry, for T cells include CD3, CD4 and CD8, for B cells they include CD19, and for NK cells they include CD16 and CD56.

Flow cytometry can be used to determine the expression of cell type specific markers. "Flow cytometry" as used herein is a technology that provides rapid multi-parametric analysis of single cells in solution. Flow cytometers utilize lasers as light sources to produce both scattered and fluorescent light signals that are read by detectors such as photodiodes or photomultiplier tubes. These signals are converted into electronic signals that are analyzed by a computer and written to a standardized format (.fcs) data file. Cell populations can be analyzed and/or purified based on their fluorescent or light scattering characteristics.

Enzyme-linked immunosorbent assay (ELISA) is a solid-phase type of enzyme immunoassay (EIA) to detect the presence of a ligand (commonly a protein) in a liquid sample using antibodies directed against the ligand to be measured. The quantification of bone morphogenetic protein-2 (BMP-2) and vascular endothelial growth factor (VEGF) can be performed by enzyme-linked immunosorbent assay (ELISA). A multiplex assay is a type of immunoassay that uses magnetic beads to simultaneously measure multiple analytes in a single experiment. A multiplex assay is a derivative of an ELISA using beads for binding the capture antibody. A multiplex bone panel for detection of multiple bone related marker genes can be used to quantify the levels of osteopontin (OPN) and osteoprotegerin (OPG). A human hematopoietic stem cell panel can be used to quantify the levels of IL-6, FLT3L, GM-CSF, IL-3, IL-34, IL-11, SCF, LIF, CXCL12 (SDF-1), IL-15, M-CSF, and/or IL-7.

The term "immunofluorescence" or "immunofluorescence staining" refers to the process of detecting and localizing of a wide variety of target biomolecules, in particular proteins and/or peptides, within a cell or tissue at a quantitative level. The process utilizes the specific pairing between antigen and antibody. By conjugating the antibody to a fluorophore, the position of the target biomolecule is visualized by exciting the fluorophore and measuring the emission of light in a specific predefined wavelength using a fluorescence microscope. Either the primary antibody is directly couple to a fluorophore or a primary antibody binding the antigen, is bound by a secondary antibody coupled to a fluorophore. The fluorescence staining may be visualized using a suitable microscope, e.g., a fluorescence microscope, a spinning disc confocal microscope, a laser scanning confocal microscope or the like. For immunofluorescence staining, the samples are fixed using fixatives, e.g, 4% v/v paraformaldehyde or 100% methanol. The fixing, immunofluorescence staining and imaging of the cells/cell culture and/or bone micro tissue or a cell culture having characteristics of bone micro tissue can be performed within the microfluid device. The term "immunohistochemistry" or IHC refers to the process of detecting and localizing proteins in cells of a tissue section exploiting the principle of antibodies binding specifically to antigens in biological tissues.

Specific markers, whose expression can be detected by immunofluorescence staining or IHC, of bone micro tissue or a cell culture having characteristics of bone micro tissue include, but are not limited to, osteopontin (OPN), osteocalcin (OCN), bone morphogentic protein (BMP-2), Collagen 1A (COL-1), Collagen IV, Fibronectin, CD34, CD31, actinin-associated LIM protein (ALP), or runt-related transcription factor 2 (RUNX2). These markers are specific for differentiated cells and are preferably analyzed by immunofluorescence staining or IHC using marker-specific primary antibodies.

Osteopontin (OPN), also designated bone sialoprotein 1, urinary stone protein, spp-1, Eta-1, nephropontin and uropontin, is an extracellular matrix cell adhesion phosphoglycoprotein. OPN is deposited into unmineralized matrix prior to calcification leading to localization at various tissue interfaces including cement lines, lamina limitans and between collagen fibrils of fully matured hard tissues. OPN functions as a substrate for transglutaminase and is involved in cell adhesion, chemoattraction and immunomodulation.

Osteocalcin, is an abundant, non-collagenous protein component of bone that is produced by osteoblasts. Osteocalcin appears transiently in embryonic bone at the time of mineral deposition, where it binds to hydroxyapatite in a calcium-dependent manner. In addition, osteocalcin is one of the most abundant, non-collagenous proteins found in mineralized adult bone.

BMP-2 promotes osteogenic differentiation of mesenchymal stem cells by enhancing mitochondrial activity.

Collagen type I alpha 1 chain antibodies, pro-alpha1 chain of type I collagen antibodies, alpha-1 type I collagen antibodies are specific for cartilage, bone, tendon, skin related structural protein antibody, or COL1A1 gene products. Collagen type I alpha is part of the bone extracellular matrix. Collagen IV is a crucial component of the extracellular matrix (ECM) that forms a specialized structure known as the basement membrane, which is essential for various physiological processes. It is a network-forming collagen, distinct in its ability to assemble into a sheet-like mesh, making it fundamental to the structural integrity and filtration properties of the basement membranes found in endothelial and epithelial tissues. Scientifically, Collagen IV has been extensively studied for its role in cellular behavior, tissue development, and morphogenesis. Its unique triple-helical structure, composed of three alpha chains (α1 through α6), enables the formation of a scaffold that supports cell adhesion, migration, and differentiation. Collagen IV is often utilized to create biomimetic environments for in vitro studies tissue engineering, and organotypic culture models. The ability of Collagen IV to interact with other ECM proteins and cellular receptors such as integrins has been a focal point in studies examining cell-ECM interactions, angiogenesis, and barrier function in both normal and pathological states.

Fibronectin is an extracellular matrix glycoprotein present on most cell surfaces, in extracellular fluids and in plasma and it has been shown to be involved in various functions including cell adhesion, cell motility and wound healing.

CD34 is primarily known as a biomarker for hematopoietic stem cells (HSCs) and hematopoietic stem precursor cells, but it has also been identified as a marker for several non-hematopoietic cells.

For instance, CD34 expression has been observed on endothelial precursors, which are responsible for the formation of blood vessels during development and in response to injury. Additionally, CD34 has been found on fibroblast progenitors, which are involved in the formation and maintenance of connective tissue.

CD31/PECAM-1 is a heavily glycosylated transmembrane homophilic adhesion protein that is highly expressed on endothelial cells and at a lower level on platelets, granulocytes, macrophages, dendritic cells, T and B cells, and NK cells.

ALP (actinin-associated LIM protein), ALP interacts with α-actinin-2 and, via this interaction, is thought to play a role in Actin filament organization, specifically regulating the association of Actin filaments arrays with muscle cells.

RUNX2 is essential for skeletal mineralization in that it stimulates osteoblast differentiation of mesenchymal stem cells, promotes chondrocyte hypertrophy and contributes to endothelial cell migration and vascular invasion of developing bones.

Additional to the specific marker proteins, cell nuclei may be visualized using 4',6-diamidino-2-phenylindole (DAPI) or Hoechst 33342 staining, preferably DAPI staining. Furthermore, the cytoskeleton of the differentiated cells may be analyzed using F-actin staining. Actin filaments, which often constitutes up to 50% of total cellular protein in eukaryotes, form both stable and labile structures and are crucial components of microvilli and the contractile apparatus of muscle cells. Members of the small GTPase family regulate the organization of the Actin cytoskeleton. Rho controls the assembly of Actin stress fibers and focal adhesion. Rac regulates Actin filament accumulation at the plasma membrane. Cdc42 stimulates formation of filopodia.

Cell matrix and mineralization staining allow to study the cell matrix and mineralization that occurs during the formation of the bone microtissue or of the cell culture having characteristics of bone micro tissue within the microfluid device. This is an important indication for studying how bone heals and regrows after, e.g., in an inflammatory situation mimicking a fracture or in response to a drug. Alican blue staining (e.g., Sigma-Aldrich) can be used for the detection of proteoglycans. Alizarin Red S (ARS) staining and/or von Knossa staining (5% silver nitrate) can be used for the detection of mineralization and bone formation. In particular, matrix calcium deposition can be measured using Alizarin Red S (ARS) staining. Alkaline phosphatase activity (ALP) staining can be used for the detection of alkaline phosphatase which is highly expressed in cells of mineralized tissue and plays a critical function in the formation of hard tissue.

Gene expression in cells/cell culture and/or bone micro tissue or a cell culture having characteristics of bone micro tissue can be studied following RNA extraction from these cells, wherein the gene expression can be studied via a PCR-based method and/or a sequencing-based method, for example. Total RNA may be extracted from the cells/cell culture and/or bone micro tissue or a cell culture having characteristics of bone micro tissue grown within the microfluid device by pulverizing the samples by hammering in a liquid nitrogen-cooled biopulverizer and adding the obtained powder to a solution suitable for RNA extraction, such as Trizol or the like. RNA purification may be performed using commercially available RNA purification kits, such as the RNeasy Plus mini kit (Qiagen, Hilden), according to the manufacturer's instructions. The term "a PCR based method" as used herein refers to methods comprising a polymerase chain reaction (PCR). This is a method of exponentially amplifying nucleic acids, e.g., DNA by enzymatic replication in vitro. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR).

Moreover, PCR-based methods comprise e.g., real time PCR, and, particularly suited for the analysis of expression levels, kinetic or quantitative PCR (qPCR). Real time PCR and quantitative PCR can be combined as quantitative real time PCR (qRT-PCR). As used herein, the term "primer" refers to the ordinary meaning of this term which is well known to the person skilled in the art of molecular biology. Primers shall be understood as being polynucleotide molecules having a sequence identical, complementary, homologous, or homologous to the complement of the regions of a target molecule, which is to be detected or quantified.

The term "sequencing method" or "next generation sequencing methods" as used herein refers to high-throughput methods to provide insights in the transcriptome of a cell with high resolution and coverage. Using next generation sequencing-based techniques, such as RNA-Seq, the whole transcriptome may be analyzed. Sequencing methods as used herein include but are not limited to Sanger sequencing or next generation sequencing, such as RNA-Seq. RNA-Seq refers to method for determining the nucleotide sequences of RNA, which is based on high throughput methods, such as NGS. RNA-Seq includes reverse transcription of RNA to cDNA and subsequent DNA-sequencing. As used herein "quantitative transcriptomics" refers to a method for quantification of the transcriptome, e.g., by using RNA-Seq-based approaches. Sequencing can be applied to investigate mRNA, total RNA, pre-mRNA, and noncoding RNA, such as microRNA and long ncRNA. In a preferred embodiment, mRNA expression is determined using sequencing methods. Commonly used sequencing platforms which can be used for determining gene expression levels include, but are not limited to, Massive Analysis of cDNA Ends (MACE) Sequencing, Illumina-based NGS technology, Oxford Nanopore Sequencing, ThermoFisher patho sequencing, 10X Visium Spatial Gene Expression, Twist Bioscience, Lexogen RNA-Seq, HTG Edge, Roche 454, GS FLX Titanium, Life Technologies SOLiD4, Complete Genomics, MGI, BGI. When it comes to the determination of expression levels, a sequencing- or next generation sequencing-based method may for example be used to detect the presence of a given mRNA by (1) library preparation, including cDNA synthesis, e.g., reverse transcription of the complete mRNA pool (the so-called transcriptome) into cDNA with help of a reverse transcriptase enzyme, (2) the sequencing step, and (3) data analysis.

In the context of the present invention, the following genes are of particular interest for gene expression studies:
The expression of cell adhesion genes *CD44,* integrin subunit beta 1 (*ITGB1*/*CD 29*)*,* cell surface antigen (*Thy1*/*CD90*), activated leukocyte adhesion molecule (*ALCAM*/*CD166*) and vascular cell adhesion molecule-1 (*VCAM1*/*CD106*) are indicative of the development of bone micro tissue or a cell culture having characteristics of bone micro tissue.

The expression levels of osteogenic genes are indicative of the development of bone micro tissue or a cell culture having characteristics of bone micro tissue and can be determined, e.g., by qRT-PCR and/or sequencing. Examples of osteogenic lineage markers include, but are not limited to, runt-related transcription factor (*RUNX2*/*CBFA1*)*,* which is key to osteoblastic differentiation and is preferably determined in bone mesenchymal stem cells (BMSCs), alkaline phosphatases (*ALP*)*,* collagen type 1 (*COL-1*)*,* secreted phosphoprotein 1 (*SPP1*), osteocalcin (*OCN*)*,* osteopontin (*OPN*)*,* bone morphogenetic protein-2 (*BMP*-*2*)*,* endothelial nitric-oxide synthase (*eNOS*)*,* vascular endothelial growth factor (*VEGF*) Chemokine Receptor 7 (*CCR7*)*, iNOS,* Arginase-1 *(ARG-1), CD206, SP7,* and Integrin Binding Sialoprotein (*IBSP*/*BSP2*)*.* The addition of Wnt3a to the osteogenic differentiation medium increases upregulation of the osteogenic marker genes *RUNX2, SP7,* and *IBSP.* Wnt3a is a protein that plays a crucial role in the Wnt signaling pathway, which is important for regulating cell growth and differentiation. The osteogenic differentiation medium is a specialized culture medium used to induce stem cells to become osteoblasts. In one embodiment, Wnt3a is added to the cell culture media to enhance differentiation into bone cells of cells which are capable of differentiating into bone cells. Specifically, Wnt3a increases the expression of genes associated with osteoblasts. This means that the cells are more effectively turning into bone-forming cells, which is beneficial for research and therapeutic applications related to bone regeneration and repair.

The expression of stem cell factor (SCF) can also be determined by qRT-PCR and/or RNA sequencing and is indicative of undifferentiated cells. SCF plays a role in the formation of blood cells. SCF is the ligand of the c-Kit oncogene and is expressed by various structural and inflammatory cells in the airways. Binding of SCF by the c-Kit receptor leads to homodimerization of the receptor and the activation of signaling pathways such as PI-3, PLC-gamma, Jak/STAT, and MAP kinase pathways. SCF expression leads to the induction of mast cell survival and the expression and release of histamine, pro-inflammatory cytokines and chemokines.

The expression levels of the pluripotency genes SRY (sex determining region Y)-box-2 (*SOX2*) and Nanog determined by qRT-PCR and/or RNA sequencing. High expression of pluripotency genes is indicative for undifferentiated cells.

Additional genes whose expression may be assessed in the context of the present invention include, but are not limited to, *18S,* Distal-Less Homeohox 5 (*DLX5*)*,* Elongation factor 1-alpha (*EF1A*)*,* Matrix metalloprotease-2 (*MMP2*)*,* Interleukin-6 (IL-6), Interleukin-8 (IL-8), Receptor Activator of NEκB Ligand (*RANKL*)*,* SRY (sex determining region Y)-box-9 (*SOX9*)*,* Phosphoglycerate kinase 1 (*PGK1*)*,* Lactate dehydrogenase A (*LDHA*)*,* Hypoxia-inducible factor 1-alpha (*HIF1A*), and Endothelial PAS domain-containing protein 1 (*EPAS*)*.*

Cell proliferation can be studied using a lactate dehydrogenase (LDH) assay (e.g., by Sigma-Aldrich, ref. nr. TOX7-1 KT) and/or using a CellTiter 96^{®} AQueous Non-Radioactive Cell Proliferation Assay (MTS) (Promega) and can be assessed by a Spectrophotometer.

In addition to gene expression analysis and immunofluorescence staining, collagen synthesis may also be quantified by Direct Red 80 (DR80) staining.

Live cell tracking and imaging of the cells/cell culture and/or bone micro tissue or a cell culture having characteristics of bone micro tissue within the microfluid device can be performed. This allows for visualization of bone micro tissue development, cell morphology, pathological changes, healing processes, cell migration, cell viability, or effects of drugs. An advantage of the microfluid device of the present invention is thus, that the bone micro tissue or cell culture having the characteristics of bone micro tissue can be directly imaged within the microfluid device, wherein the imaging includes real-time cell monitoring and time-lapse imaging. Live cell tracking and imaging can be carried out using the Staining with CellTrackerTM Red CMTPX (Thermo Fisher Scientific, United Kingdom), and/or Live/dead Fixable Far Red Dead Cell Stain (Thermo Fisher Scientific) according to manufacturer's instructions. AutoLCI is an automated live cell imaging system that is equipped with an advanced fluorescence and bright field microscopy, autofocusing and real time multi-position imaging technology for a microfluid device. This device allows positioning the microfluid device in an incubator, thereby providing improved cell viability and reducing chances of cellular abnormality by minimizing disturbances during the course of an experiment.

The above analytical methods are used to analyze the cells/cell culture and/or bone micro tissue or a cell culture having characteristics of bone micro tissue within the microfluid device or extracted from the microfluid device. These analytical methods provide invaluable input regarding culture conditions, as well as the effect of drugs or an inflammatory situation mimicking fracture healing on cells in the chip. Thus, they are useful for studying bone diseases, such as bone fracture or bone cancer or osteoporosis, within the microfluidic device and asses the efficacy of cell therapy or drug therapy on those diseases.

In some embodiments, the method further comprises (preferably as step (D)) assessing the efficacy of a therapeutic agent or a candidate therapeutic agent comprising contacting the cells, bone micro tissue and/or cell culture with a therapeutic agent or a candidate therapeutic agent. Alternatively, in some aspects the invention relates toa method comprising assessing the efficacy of a therapeutic agent or a candidate therapeutic agent, the method comprising contacting the cells, bone micro tissue and/or a cell culture as provided herein with a therapeutic agent or a candidate therapeutic agent. Step (D) is preferably performed after step (C). Thus, in one aspect, the method provided herein comprises the following steps in the order (AA) to (D): optionally (AA)(i) culturing the (to be seeded) cells in a medium in a humidified CO₂ buffered atmosphere, optionally (AA)(ii) dissociating the (to be seeded) cells; and optionally (AA)(iii) freezing the (to be seeded) cells; (A) seeding the microfluidic device of the present invention with cells as defined herein; (B) culturing the cells in the microfluidic device, optionally (B)(i) culturing the cells for about 12 to 48 hours, preferably for about 18 to about 48 hours, preferably for about 24 hours, further optionally (B)(ii) culturing the cells for about 1 week to about 6 weeks, preferably for about 1 week to about 2 weeks; optionally (CC) contacting the cells/cell culture and/or bone micro tissue with a pro-inflammatory composition; or it further comprises a method for producing cells/cell culture and/or bone micro tissue or a cell culture having characteristics of bone micro tissue, wherein the cells/cell culture and/or bone micro tissue has characteristics of a bone fracture, the method further comprising applying a pro-inflammatory composition to the cells/cell culture and/or bone micro tissue obtained or obtainable by the methods provided herein; optionally (C) recovering the cells/cell culture and/or bone micro tissue or a cell culture having characteristics of bone micro tissue from the microfluidic device; and (D) assessing the efficacy of a therapeutic agent or a candidate therapeutic agent comprising contacting the cells, bone micro tissue and/or cell culture with a therapeutic agent or a candidate therapeutic agent; or a method comprising assessing the efficacy of a therapeutic agent or a candidate therapeutic agent, the method comprising contacting the cells, bone micro tissue and/or a cell culture as provided herein with a therapeutic agent or a candidate therapeutic agent.

Assessing the efficacy of a therapeutic agent or a candidate therapeutic agent refers to the ability of a therapeutic agent or a candidate therapeutic agent to heal and/or to contribute to the healing of a bone fracture and/or of a bone disease or disorder.

In particular when the bone micro tissue reflects bone tissue characteristic of a bone disease or disorder (e.g. when the cells are healthy cells or are obtained or derived/originate from healthy subjects, but treated with a composition inducing a disease state, e.g. fracture by applying the proinflammatory composition), or when the cells are bone disease/disorders cells (or cells having characteristics of a bone disease/disorder) or are obtained or derived/originate from subjects having a bone disease or disorder) the method may be used to analyze the efficacy of (candidate) compounds on the bone disease or disorder. This may be done by contacting the bone micro tissue with (candidate) compounds and assessing the efficacy of (candidate) compounds on enhancing or decreasing bone modeling/remodeling (e.g. increase bone strength/ bone density). The (candidate) compound can be immune cells, such as PBMCs, wherein the efficacy and/or effect of immune cells on bone fracture healing is assessed.

In some embodiments, the therapeutic agent is an agent with known capacity to heal fracture or promote fracture healing; or wherein the candidate therapeutic agent is an agent with suspected capacity to heal fracture or promote fracture healing.

In some embodiments, the therapeutic agent is an agent with known capacity to model and/or remodel bone; or wherein the candidate therapeutic agent is an agent with suspected capacity to model and/or remodel bone.

In some embodiments, the therapeutic agent or a candidate therapeutic agent is a drug and/or cell therapy.

In some embodiments, the therapeutic agent is an agent with known efficacy against a bone disease; or wherein the candidate therapeutic agent is an agent with suspected efficacy against a bone disease.

In some embodiments, the bone disease is selected from any one of bone fracture, osteoporosis, osteoarthritis, rheumatoid arthritis, Paget's disease of bone, osteogenesis imperfecta, rickets, osteomalacia, fibrous dysplasia, hyperparathyroidism, or bone cancer. In a preferred embodiment, the bone disease is bone fracture. In another preferred embodiment, the bone disease is osteoporosis. In another preferred embodiment, the bone disease is bone cancer, wherein the bone cancer is selected from osteosarcoma, Ewing's sarcoma, chondrosarcoma, multiple myeloma, fibrosarcoma, chordoma.

Known drugs for use in the treatment of bone fractures and/or bone diseases/bone disorders, such as osteoporosis, and/or drugs having an effect on bone modeling/bone remodeling include but are not limited to Bisphosphonates, Denosumab, Selective Estrogen Receptor Modulators (SERMs), Calcitonin, Calcium and Vitamin D Supplements. However, the methods disclosed herein may also be used to identify new drugs for the treatment of bone fractures and/or bone diseases/disorders, i.e., the methods disclosed herein may be methods of drug screening.

In some embodiments, the drug comprises an inhibitor or activator of bone formation, preferably wherein the drug comprises one or more of the bone formation inhibitors Sclerostin, Dkk1, C5a, a Histamin-receptor agonist, TNF-α, CCL3, or wherein the drug comprises one or more of the bone formation activators Wnt3a, Wnt1, PGE2, TGFb, or IL-17.

In some embodiments, the therapeutic agent or candidate therapeutic agent is a substance from a library. In some embodiments, the therapeutic agent or candidate therapeutic agent is to be applied to the (cells, bone micro tissue and/or cell culture in the) microfluidic device. In particular, the therapeutic agent or candidate therapeutic agent is to be applied to the cells, bone micro tissue and/or cell culture cultured in the microfluidic device (i.e., in an *in vitro* method), wherein the cells are obtained from a subject suffering from a bone fracture and/or a bone disease or disorder, to identify a therapeutic agent or candidate therapeutic agent which has efficacy in treatment of said bone fracture and/or bone disease or disorder in said subject.

"Cell therapy" as used herein refers to a therapeutic approach that aims to prevent or treat diseases, e.g., bone diseases and/or bone disorders, by introducing carefully chosen, expanded, and modified healthy cells into the body to replace or repair resident cell populations. Cell therapies constitute one possible clinical treatment for cancer, such as bone cancer, but they also hold promise in the regenerative medicine field, e.g., for healing bone fractures. Various types of cells can be utilized in cell therapy, including hematopoietic stem cells (HSC), and mesenchymal stem cells. Hematopoietic stem cells can generate blood cells and are commonly employed in treatments such as bone marrow transplants. Mesenchymal stem cells have the potential to differentiate into various cell types, including bone cells, and exhibit immunomodulatory effects, making them suitable for treating inflammatory and autoimmune conditions. Lymphocytes, specifically T cells, and dendritic cells are vital components of the immune system and can be manipulated to enhance immune responses against cancers or infections.

In some embodiments, the cell therapy comprises stem cell therapy (iPSCs), CAR-T cell therapy, hematopoietic stem cell (HSC) therapy, mesenchymal stem cell (MSC) therapy, NK cell therapy, T-cell therapy and/or a cell therapy with human primary immune cells.

In one aspect, the method provided herein comprises the following steps in the order (AA) to (D): optionally (AA)(i) culturing the (to be seeded) cells in a medium in a humidified CO₂ buffered atmosphere, optionally (AA)(ii) dissociating the (to be seeded) cells; and optionally (AA)(iii) freezing the (to be seeded) cells; (A) seeding the microfluidic device of the present invention with bone cells or cells which are capable of differentiating into bone cells and endothelial cells or cells which are capable of differentiating into endothelial cells; (B) co-culturing the cells in the microfluidic device, optionally (B)(i) culturing the cells for about 12 to 48 hours, preferably for about 18 to about 48 hours, preferably for about 24 hours, further optionally (B)(ii) culturing the cells for about 1 week to about 6 weeks, preferably for about 1 week to about 2 weeks to allow formation of bone micro tissue or a cell culture having the characteristics of bone micro tissue; (CC) contacting the cells/cell culture and/or bone micro tissue with a pro-inflammatory composition; or it further comprises a method for producing cells/cell culture and/or bone micro tissue or a cell culture having characteristics of bone micro tissue, wherein the cells/cell culture and/or bone micro tissue has characteristics of a bone fracture, the method further comprising applying a pro-inflammatory composition to the cells/cell culture and/or bone micro tissue obtained or obtainable by the methods provided herein; and (D) assessing the efficacy of a therapeutic agent or a candidate therapeutic agent comprising contacting the cells, bone micro tissue and/or cell culture with a therapeutic agent or a candidate therapeutic agent; or a method comprising assessing the efficacy of a therapeutic agent or a candidate therapeutic agent, the method comprising contacting the cells, bone micro tissue and/or a cell culture as provided herein with a therapeutic agent or a candidate therapeutic agent, wherein the therapeutic agent or the candidate therapeutic agent are hematopoietic cells or cells which are capable of differentiating into hematopoietic cells, preferably PBMCs, and said hematopoietic cells or cells which are capable of differentiating into hematopoietic cells, preferably PBMCs are introduced into the microfluid device. This method allows to study the impact that patient derived primary immune cells have on bone fracture healing.

In some embodiments, the cell therapy comprises cells obtained from a subject. In a preferred embodiment, the cell therapy comprises cells obtained from a human subject.

In some embodiments, the cells (for cell therapy) are allogenic, autologous or heterologous. "Allogenic" cells are cells which are obtained from a (human) donor other than the subject suffering from a bone disease/bone disorder, preferably wherein the donor and the subject from a bone disease/bone disorder are human leukocyte antigen (HLA)-matched. "Autologous" cells are cells which are obtained from the same subject suffering from a bone disease/bone disorder. "Heterologous" cells are cells obtained from a mixed population of donors.

In some embodiments, the method as disclosed herein is performed *in vitro.*

In some embodiments, the method as disclosed herein is not practiced on the human or animal body.

In some embodiments, the method does not comprise a step of obtaining cells of a subject.

In one aspect, the present invention relates to a method for producing a microfluidic device comprising cells, bone micro tissue or a cell culture having characteristics of bone micro tissue, the method comprising the methods as provided herein.

In one aspect, the present invention relates to a microfluidic device comprising cells, bone micro tissue or a cell culture having characteristics of bone micro tissue, wherein the microfluidic device comprising cells is produced by the methods provided herein; or wherein the cells, bone micro tissue or a cell culture having characteristics of bone micro tissue are obtained or obtainable by the methods provided herein.

In one aspect, the present invention relates to a microfluidic device as provided herein further comprising cells, bone micro tissue or a cell culture having characteristics of bone micro tissue.

In one aspect, the present invention relates to the use of the microfluidic device provided herein for culturing cells.

In one aspect, the present invention relates to the use of the microfluidic device provided herein, or of the cells, bone micro tissue or a cell culture having characteristics of bone micro tissue obtained or obtainable by the methods provided herein for establishing a model of bone micro tissue or a model of a bone disease or disorder or a model of a bone fracture.

In one aspect, the present invention relates to the use of the microfluidic device provided herein, or of the cells, bone micro tissue or a cell culture having characteristics of bone micro tissue obtained or obtainable by the methods provided herein for assessing the effect of a therapeutic compound or a candidate therapeutic compound on bone modeling or bone remodeling, of a therapeutic compound or a candidate therapeutic compound on a bone disease or bone disorder, or of a therapeutic compound or a candidate therapeutic compound on a bone fracture.

In one aspect, the present invention relates to the use of the microfluidic device provided herein, or of the cells for analyzing bone modeling or remodeling, for analyzing a bone disease or bone disorder, or for analyzing bone fracture and/or bone fracture healing, wherein said cells are cells which are capable of differentiating into bone cells, preferably wherein the cells are cells which are capable of differentiating into bone cells are stem cells, preferably mesenchymal stem cells (MSCs) or wherein the cells which are capable of differentiating into bone cells are sarcoma osteogenic-2 (Saos-2) cells, preferably wherein the Saos-2 cells are capable of differentiating into osteocytes and/or wherein said cells are cells which are capable of differentiating into endothelial cells, preferably wherein the cells which are capable of differentiating into endothelial cells preferably are human umbilical vein endothelial cells (HUVECs) and/or wherein said cells are cells which are capable of differentiating into hematopoietic cells, preferably wherein the cells which are capable of differentiating into hematopoietic cells are hematopoietic stem cells, preferably hematopoietic stem and progenitor cells (HSPCs), more preferably CD34⁺ HSPCs.

The present invention also relates to the field of personalized medicine. Personalized medicine involves the systematic use of information about each individual patient to select or optimize the patient's preventative and therapeutic care. In the modern conception of personalized medicine, the tools provided to the physician are more precise, probing not just the obvious, such as cells under a microscope, but the very molecular makeup of each patient. The ambition of personalized medicine is to be able to provide a patient with a more precise therapy, a therapy that matches the individual's properties down to the molecular properties. In the context of this invention, this refers to cells/cell culture and/or bone micro tissue or a cell culture having characteristics of bone micro tissue in a microfluid device, wherein the cells within the microfluid device are obtained from a subject suffering from a bone fracture and/or a bone disease, and wherein the efficacy of therapeutic agents or candidate therapeutic agents are assessed by contacting the cells, bone micro tissue and/or cell culture in said microfluid device with said therapeutic agents or said candidate therapeutic agents, and wherein the therapeutic agent or the candidate therapeutic agent showing the better efficacy compared to the remaining therapeutic agents or candidate therapeutic agents is identified as best drug for said subject. In other words, the cells/cell culture and/or bone micro tissue or a cell culture having characteristics of bone micro tissue in the microfluid device can be used in a drug screening approach to identify therapeutic agents or candidate therapeutic agents which could be suitable for the treatment of a bone fracture and/or a bone disease. The microfluid device and the methods of (co-)culturing cells in said microfluid device of the present invention can in principle be used as a model for any bone disease or bone disorder. The cells/cell culture and/or bone micro tissue or a cell culture having characteristics of bone micro tissue seeded into and (co-)cultured in the microfluidic device can reflect donor-derived variables, i.e., age, sex, lifestyle (e.g. smoking or diet), medical history (e.g. diabetes or osteoporosis).

For example, when the bone micro tissue reflects bone tissue characteristic of a bone disease or disorder (e.g. when the cells are bone disease/disorders cells (or cells having characteristics of a bone disease/disorder), or in particular when the cells (to be seeded) are obtained or derived/originate from a subject having a bone disease or disorder) the method may be used to analyze the efficacy of (candidate) compounds on the bone disease or disorder. This may be done by contacting the bone micro tissue with (candidate) compounds and assessing the efficacy of (candidate) compounds, e.g. on enhancing or decreasing bone modeling/remodeling (e.g. increase bone strength/ bone density). For illustration, a number of candidate drugs and/or approved drugs may be available for the treatment of a bone disease or disorder. However, it is desired to identify the therapy/drug that provides optimal efficacy before the patient is treated. In such a situation, the efficacy of the candidate drugs and/or approved drugs can be assessed using the microfluidic device of the invention in accordance with the above, e.g. by contacting the bone micro tissue with (candidate) compounds and assessing the efficacy of (candidate) compounds. The compound or compounds with the assessed best efficacy may be used in the treatment of the patient.

In some embodiments, the bone disease is a bone fracture. For example, when the bone micro tissue reflects bone tissue characteristic of a bone fracture, wherein the characteristics of the bone fracture are induced by a proinflammatory cocktail as defined above, the method may be used to analyze the efficacy of (candidate) compounds, such as immune cells, on bone fracture healing. This may be done by (1) contacting the bone micro tissue with a proinflammatory cocktail to induce the characteristics of a bone fracture and (2) by contacting the bone micro tissue having the characteristics of a bone fracture with (candidate) compounds, such as immune cells, preferably wherein the immune cells and the cells forming the bone micro tissue are obtained from the same subject (or from (a) different subject(s)), and (3) assessing the efficacy of (candidate) compounds on bone fracture healing.

This may of course also be done in patient cohorts, for example, in the preparation of clinical trials, i.e. prior to starting the clinical trial. By assessing the efficacy of (candidate) compounds in different patient cohorts it may be assessed which cohort(s) is/are more amenable to treatment with the (candidate) compound and the clinical trial may be tailored to target these specific cohort(s).

The cells/cell culture and/or bone micro tissue or a cell culture having characteristics of bone micro tissue in the microfluid device as described herein can be used for drug screening. Thus, provided herein is a method of screening drugs in the cells/cell culture and/or bone micro tissue or a cell culture having characteristics of bone micro tissue cultured in the microfluid. "Drug screening" as used herein refers to a method of iterative testing a variety of therapeutic agents or candidate therapeutic agents in a common (high-throughput) assay, i.e., in a microfluid device comprising bone micro tissue or a cell culture having characteristics of bone micro tissue, with the aim to identify and evaluate therapeutic agents or candidate therapeutic agents for treatment of bone fractures and/or bone diseases. The screened drugs may have efficacy for the treatment of bone fractures and/or bone diseases in subjects suffering from said bone fracture and/or bone disease in general or they may be used to identify the best treatment for a specific subject. Currently, due to the limitations of 2D cultures and *in vivo* models, as well as the poor translation of preclinical efficacy from animal models to human trials, the need for an alternative method for screening and evaluating new treatments for bone fractures and/or bone diseases or disorders is indicated. The microfluid device with its applications as described herein can provide a suitable platform for this work to reduce financial and ethical costs compared to *in vivo* models and can fill the gap between clinical laboratory screenings in 2D cultures and clinical trials of patients.

The microfluid device described herein, is an organ-on-a-chip models developed using microengineering and microfluidic technologies, which aims to recreate the physiological microenvironments of bone or bone tissue, can be used as a tool to study (patho)physiological of bone (tissue) processes *in vitro.* The method for culturing cells in the microfluidic device, wherein the cells are cells capable of differentiating into cells of the bone tissue, thereby forming a bone micro tissue or a cell culture having characteristics of a bone micro tissue, allow studying of bone physiology, diseases, and remodeling processes due to its 3D structural model of bone and its trabeculae that can be created and simulated by injecting a variety of cells and extracellular matrix.

As used herein, the term "same" refers preferably to an identical value e.g. for a diameter, volume and/or area, but can include minor variations of ± 5 %, ± 4 %,± 3 %,± 2 %,± 1 %,± 0.5 %, or less. It the values are identical, the variation is 0 % and/or within the scope of measurement accuracy.

As used herein the term "about" refers to ± 10%, preferably ± 5%, ± 4%,± 3%,± 2%, more preferably ± 1%.

As used herein, the terms "comprising", "including", "having" or grammatical variants thereof are to be taken as specifying the stated features, integers, steps or components but do not preclude the addition of one or more additional features, integers, steps, components or groups thereof. The terms "comprising"/"including"/"having" encompass the terms "consisting of' and "consisting essentially of'. Thus, whenever the terms "comprising"/"including"/"having" are used herein, they can be replaced by "consisting essentially of' or, preferably, by "consisting of'.

The terms "comprising"/"including"/"having" mean that any further component (or likewise features, integers, steps and the like) can be present.

The term "consisting of" means that no further component (or likewise features, integers, steps and the like) can be present.

The term "consisting essentially of' or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed product, composition, device or method and the like.

Thus, the term "consisting essentially of" means that specific further components (or likewise features, integers, steps and the like) can be present, namely those not materially affecting the essential characteristics of the product, composition, device or method. In other words, the term "consisting essentially of" (which can be interchangeably used herein with the term "comprising substantially"), allows the presence of other components in the product, composition, device or method in addition to the mandatory components (or likewise features, integers, steps and the like), provided that the essential characteristics of the product, composition, device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

While the invention has been illustrated and described in detail in the drawings and foregoing description, such illustration and description are to be considered illustrative or exemplary and non-restrictive; the invention is thus not limited to the disclosed embodiments. Variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality and may mean "at least one".

The invention is illustrated in the following Examples:

### Example 1: Production of a microfluidic device (chip) of the present invention

Bone is an important part of human body structure and plays a vital role in human health. A human-based in vitro model for bone regeneration is an exciting prospect because of the wide range of advantages it could offer. Currently, due to the limitations of 2D cultures and in vivo models, as well as the poor translation of preclinical efficacy in animal models to human trials, which indicates the need for an alternative method for screening and evaluating new treatments for bone disorders. Such a model can provide a suitable platform for this work to reduce financial and ethical costs compared to in vivo models and fill the gap between clinical laboratory screenings and clinical trials of patients. Organ-on-a-chip models developed using microengineering and microfluidic technologies aim to recreate the physiological microenvironments of organs or tissues as a tool to study (patho)physiological processes in vitro. Bone-on-a-chip models are relevant for the study of bone physiology, diseases, and remodeling processes. While there are a few bone-on-a-chip models that summarize the cellular components of bone, most of these models do not capture the chemical and structural properties of bone tissue. Here, the development of a human bone-on-a-chip (hBONEoC) platform is reported, which includes a 3D structural model of bone and its trabeculae that can be created and simulated by injecting a variety of cells and extracellular matrix.

We have successfully developed a microfluidic platform that includes a 3D architecture and physiologically relevant for an osteogenic device on a chip suitable for long-term culture. It has the potential to provide more accurate, cheaper and faster methods to investigate new treatments for bone and bone fracture, compared to standard in vitro and in vivo tests. and contribute to bone regeneration, which in turn can be used to develop bone regeneration strategies. Further, to realize the fabrication of biomimetic structures, a series of biocompatible materials, including Matrigel and synthesized alternative hydrogels, are used to support the self-organization of bone organoids. The features of this hBone-on-a-chip (hBONEoC) can be described from 4 perspectives:
1. Unique chip design.
2. Integrated chip printing with advanced DLP method, saving time and production costs.
3. Resin biomaterial with minimal drug absorption instead of PDMS.
4. Different biological application to investigate bone fracture and its healing process.

Organoids, as a new concept built in vitro with the help of organ-on-a-chip technology based on biological theory, can simulate the complex biological functions of organs inside the body. Once proposed, it shows a wide application prospect in organ development research, drug screening, mechanism study, etc. As a complex and special organ, making bone organoid itself is very challenging. This chip (hBONEoC) introduces the characteristics of the bone microenvironment, the concept of organoids, focuses on the progress of bone organoid research, and suggests strategies for bone organoid fabrication, study directions, and application prospects. Also, they can originate from the patient's tissue, which can help personalized medicine to optimize treatment.

The microfluidic device (herein also termed chip) of the present invention is particularly advantageous to study the interaction of bone cells and cells within the bone environment. The microfluidic device has been specifically designed to best accommodate such cells, and to best mimic a bone environment close to a natural setting. Advantages are the unique design of the chip, the advanced printing technique (DLP method), and the type of biocompatible material suitable for printing that does not absorb drugs and biomedical application of this chip in studies and research of bone disorders and biology. Thus, the microfluidic device is specifically advantageous to study bone modeling and bone remodeling, bone diseases and bone healing processes, e.g. fracture healing and/or healing of an inflammation event, as well as to study how bone cells and cells of the bone environment react to an inflammation or drugs. The microfluidic device thus provides unprecedented opportunities to study the effect of an individual patient's immune cells and/or drugs on bone cells cultivated in the chip. Said cells may represent a disease state, e.g. mimic the inflammatory situation of a bone fracture, and/or comprise cancer cells, such as sarcoma cells. Thereby, the chip contributes to a personalized medicine approach by using cells that have been obtained from the patient. The unique structure of the herein provided microfluidic device allows for the above mentioned advantages. Furthermore, they are demonstrated in Examples 2 to 7 below.

The chip was designed with SOLIDWORKS software (Dassault Systèmes SolidWorks Corp.) and analysed by that simulation to best represent an environment as close as possible to the situation in nature. In the next step, using DLP technology and ASIGA MAX UV 3D printing, it was prepared according to manufacturer's instructions. Biocompatible Pro3dure Printodent GR-10 resin was used in the fabrication of this chip and each chip was washed with a cleaning solution through this protocol, then thoroughly rinsed with 70% alcohol. After drying, it was irradiated in a UV device for 20 seconds. After this step, it was washed with sterile distilled water and washed twice with sterile normal saline under a biological hood. Then, it was sterilized for 20 minutes under UV under a hood for the final step and after drying in the hood, the sterile chip is finished. To complete the sterilization process, 150 µL of a 20 µg/mL streptavidin (SA) solution (in PBS) was injected into each chip and incubated for 1 hour at room temperature. Finally, 100 µL of collagen I was injected into each chip to form a treated chip. The modified chips were then stored in a refrigerator for later use. For testing, they were placed in a sterile rubber seal and stored in a refrigerator at 4°C.

### Example 2: Establishing a cell culture and seeding the microfluidic device (chip) with cells

As a proof of concept, and to test viability of cells within the microfluidic device of the present invention, different cell lines are seeded in the chip to study if a permanent culture within the chip is successful. For this, the following cell lines are used:

### Sarcoma osteogenic (Saos-2) cells:

Saos-2 cells is a cell line that has been originally obtained from an osteosarcoma patient. The cell line shows an epithelial morphology and possesses osteoblastic features. Saos-2 cells are capable of differentiating into osteocytes. Saos-2 cells are thus used as a proof of principle for culturing and seeding osteosarcoma cells on the chip. Saos-2 cell are cultivated in McCoy's 5a Medium Modified, (ATCC Catalog No. 30-2007). McCoy's 5A Medium is modified to contain 1.5 mM L-glutamine and 2200 mg/L sodium bicarbonate and that it is further complemented with fetal bovine serum to a final concentration of 10%, penicillin/streptomycin (1%), and Glutamax^{™} (1%) (Glutamax is 200 mM L-alanyl-L-glutamine dipeptide in a 0.85% NaCl solution). Cells between passages 3 and 5 are used in the further experiments.

Saos-2 cells are cultured according to the following steps:
1. Saos-2 are cultured in complete McCoy's 5a Medium Modified as described above in an appropriate container (Filter cap T25 cell culture Flask Thermo Scientific^{™} 174951 or Filter cap T75 cell culture Flask Thermo Scientific^{™} 174952).
2. Cells are maintained at 37°C and 5% CO₂; The media is refreshed medium every 2 days. The cells can be stored by freezing and storing at -80°C or can be immediately used as described herein below.
3. After step 2 or after thawing the frozen cells from passage 2, the cells are expanded in the same medium and dissociated using trypsin and subsequently harvested at 70% confluency.
4. 20 µL of cells (same medium) are seeded into the chip at a concentration of 500,000 cells/mL (10,000 Saos-2 cells),. For seeding Saos-2 cells and HUVEC cells are mixed at a ratio of 2:3 and 25,000 cells in total (HUVEC+Saos-2) are seeded per chip). Then 50 µL of this mix is injected to the channel with 50 µL of matrigel.

### Human umbilical vein endothelial cells (HUVECs):

HUVECs are a standard model cell line that shows endothelial morphology. HUVECs are thus used as a proof of principle for culturing and seeding endothelial cells on the chip. HUVECs are cultured in EGM-2 media supplemented with 2% FBS, hFGF-B, VEGF, R3-IGF-1, ascorbic acid, hEGE, Heparin, and gentamicin (Endothelial Cell Growth Medium-2 (EGM-2); BulletKit, Lonza).

The cells are maintained in a 2% gelatin-coated cell culture flask (Corning); cells between passages 3 and 4 are used in the further experiments.

HUVECs are cultured according to the following steps:
1. HUVECs are cultured in EGM-2 media with supplements as described above in gelatin-coated flasks.
2. Cells are maintained at 37°C and 5% CO₂. The media are refreshed every 3 days. The cells can be stored by freezing and storing at -80°C or can be immediately used as described herein below.
3. After step 2 or after thawing the frozen cells, cells are expanded and dissociated using trypsin and subsequently harvested at 80% confluency.
4. Cells between passages 3 and 4 are used for seeding the chip. 15,000 cells are seeded into the chip.

### Human CD34⁺ hematopoietic stem and progenitor cells (HSPCs, also hematopoietic stem cells (HSC)):

CD34⁺ HSPCs can be differentiated into osteoclasts which is a type of bone cell that breaks down bone tissue, e.g. during maintenance, repair, and remodeling of bones of the vertebral skeleton. Thus, this cell line is of particular interest to study the role of the osteoclast lineage in bone diseases. Human CD34⁺ HSPCs are thus used as a proof of principle for culturing and seeding HSPCs on the chip. Cells are selected to be CD34 positive via flow cytometry and the cells are only used if a minimum of 95% of the cells are positively stained for CD34⁺. HSPCs are maintained in serum-free HPC Expansion Medium XF (PromoCell GmbH, Heidelberg, Germany) supplemented with Cytokine-Mix E (containing TPO, SCF, Flt-3 ligand and IL-3) and 1% (v/v) penicillin/streptomycin. Cells between passages 3 and 5 are used in the further experiments.

Human CD34⁺ HSPCs are cultured according to the following steps:
1. The CD34⁺ cells with a purity of >95% are cultured in the XF media described above.
2. Cells are maintained at 37°C and 5% CO₂. The media are refreshed every 2 days. The cells can be stored by freezing and storing at -80°C or can be immediately used as described herein below.
3. After step 2 or after thawing the frozen cells, cells are expanded and dissociated and subsequently harvested at 80% confluency.
4. Cells between passages 3 and 4 are used for seeding the chip (10,000 cell/chip).

### Human Bone Marrow Mesenchymal Stem Cells (hBM-MSCs):

hBM-MSCs can e.g. differentiate into osteoblasts, osteocytes, osteoclasts and/or bone lining cells and can thus be used to study the role of healthy osteoblasts in the following experiments. hBM-MSCs are thus used as a proof of principle for culturing and seeding human mesenchymal stem cells on the chip. The cell culture is carried out in alpha-MEM media supplemented with platelet lysate (5%) (STEMCELL Technologies), penicillin/streptomycin (1%), and Glutamax^{™} (1%) (Glutamax is 200 mM L-alanyl-L-glutamine dipeptide in a 0.85% NaCl solution). Cells between passages 3 and 5 are used in the further experiments.

hBM-MSCs are cultured according to the following steps:
1. hBM-MSCs are cultured in the α-MEM media with supplements as described above.
2. Cells are maintained at 37°C and 5% CO₂. The media is refreshed every 2 days. The cells can be stored by freezing and storing at -80°C or can be immediately used as described herein below.
3. After step 2 or after thawing the frozen cells, cells are expanded in the same culture media as above and dissociated using trypsin and subsequently harvested at 70% confluency.
4. Cells between passages 3 and 5 are used for further experiments. 20 µL of cells are seeded into the chip at a concentration of 500,000 cells/mL (10,000 cells/chip).

After seeding of any of the above-mentioned cell lines into the chip, the inlets of the chip are blocked to prevent evaporation. Subsequently, the cells are allowed to attach for 24 hours in a humidified atmosphere in an incubator at 37°C with 5% CO₂.

After seeding of any of the above-mentioned cell lines into the chip, the inlets are blocked, e.g. with a pipette tip, to prevent medium evaporation, and the cells are allowed to attach for 24 hours in a humidified atmosphere (85-95% relative humidity) in an incubator at 37°C with a 5% CO₂ atmosphere. After attachment, the chips containing the cells are connected via Tygon tubing (Ultrainfuse, Harvard Instruments) to a microfluidic pump. This pump provided a continuous flow of the cell specific media described above to the respective cells at a flow rate of 4 µL/min. Viability of the cells is be assessed via flow cytometry or preferably live cell imaging

### Results

A stable culture after seeding the chip is achieved for cell lines mentioned above. Thus, the microfluidic device of the present invention is suitable for examining bone diseases and cell reactions to fractures or inflammations within the chip, e.g. using the above mentioned cell lines.

### Example 3: Establishing a cell culture and seeding the microfluidic device with Saos-2 cells or hMSCs until formation of bone micro tissue

In the sense of the present invention, it is particularly advantageous to combine different cell lines within the chip to better mimic the situation of a bone condition in nature. In general, all mentioned cell lines might be combined to study bone diseases. As a proof of concept, the osteosarcoma cell line Saos-2 and the HUVEC cell line or, in the alternative the hMSCs and the HUVEC cell line, are introduced into a single microfluidic device of the present invention. This has the advantage, that both, Saos-2 derived osteocytes and HUVEC derived endothelial cells; or, in the alternative, hMSC derived osteoblasts and HUVEC derived endothelial cells, can be studied within the chip, e.g. together during an inflammatory situation (such as during fracture healing) or during treatment with a drug.

In general, the cells can be cultured, and the chip can be seeded as described in Example 2 above. In brief:
The Saos-2 cells are cultured in a basal medium consisting of McCoy's 5a Medium Modified without nucleosides, 0.2 mM ascorbic acid 2-phosphate, and 10% fetal bovine serum, in a humidified atmosphere. The cultures are maintained in an incubator at 37°C with 5% CO₂, with the medium being refreshed every two days. At passage 2, the cells were trypsinized and stored in liquid nitrogen for future use. Upon thawing, the cells are re-expanded in the basal culture medium and trypsinized at 70% confluency for seeding on chips. For seeding, 20 µL of medium containing 500,000 cells per mL (10,000 cells per chip) is slowly introduced with a micropipette.

Human mesenchymal stem cells (hMSCs) are obtained from human bone marrow aspirates of a healthy donor, with written informed consent. These cells are cultured as described in Example 2. The cells are maintained in an incubator at 37°C with 5% CO₂, with the medium being refreshed every two days. At passage 2, the cells are trypsinized and stored in liquid nitrogen for future use. Upon thawing, the cells are re-expanded in the medium of Example 2 and dissociated with trypsin at 70% confluency for seeding on the chip.

For seeding, 20 µL of Saos-2 at a concentration of 500,000 cells per mL and 50 µl HUVEC cells at a concentration of 300,000 cells per mL (25,000 cells of Saos-2 and HUVEC in total per chip) are slowly introduced into the same subchamber with a micropipette.

Alternatively, 20 µL of hMSCs at a concentration of 500,000 cells per mL and 50 µl HUVEC cells at a concentration of 300,000 cells per mL (25,000 cells of hMSCs and HUVEC in total per chip) are slowly introduced into the same subchamber with a micropipette.

After seeding the inlets are blocked, e.g. with a pipette tip, to prevent medium evaporation, and the cells are allowed to attach for 24 hours in a humidified atmosphere (85-95% relative humidity) in an incubator at 37°C with a 5% CO₂ atmosphere. After attachment, the chips containing the cells are connected via Tygon tubing (Ultrainfuse, Harvard Instruments) to a microfluidic pump. As a flow media during culturing in the chip either the cell-specific media of Example 2 for Saos2 and HUVEC or, alternatively for hMSCs and HUVEC ,is injected via separate channels into the chip, or both media for Saos2 and HUVEC or, alternatively for hMSCs and HUVEC, are mixed. Each cell consumes the preferred media. The pump provides a continuous flow of media or media mix to the respective cells at a flow rate of 4 µL/min.

The cells/cell culture in cultivated until bone micro tissue forms or cell/cell culture having characteristics of bone micro tissue.

The primary endpoint analysis is a cell viability assay by live cell imaging. The culturing conditions can be optimized to ensure maximum cell viability for the mix of different cell types.

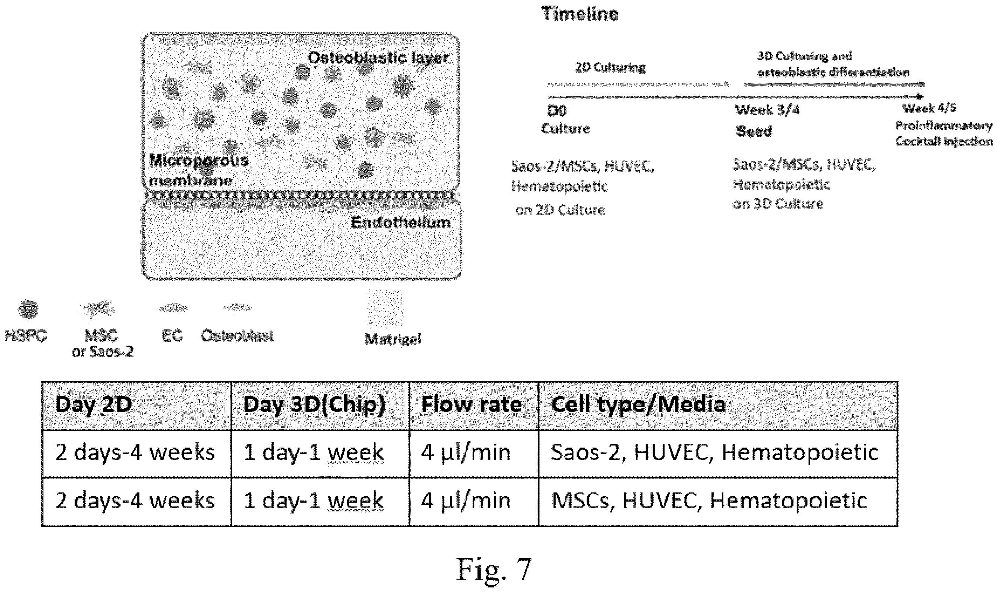

Fig. 7 is a timetable of Cells culture (0 - 35 days) and seeding in chip (ready to use chip).

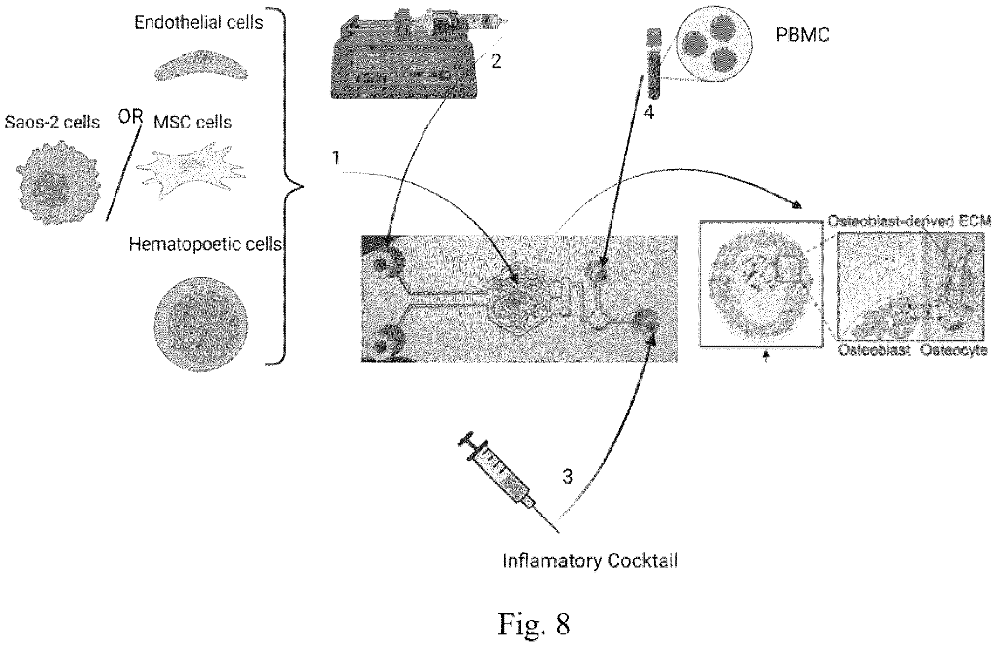

Fig. 8 illustrates culturing cells (Endothelial cells/HUVEC, Saos-2 cells or mesenchymal stem cells (MSC), and hematopoietic cells in the device to produce bone micro tissue. Subsequently, agents, such as an inflammatory cocktail, can be applied to the bone micro tissue to reflect bone fracture and/or further agents can be applied (such as immune cells/PBMC and/or (candidate) therapeutic agents).

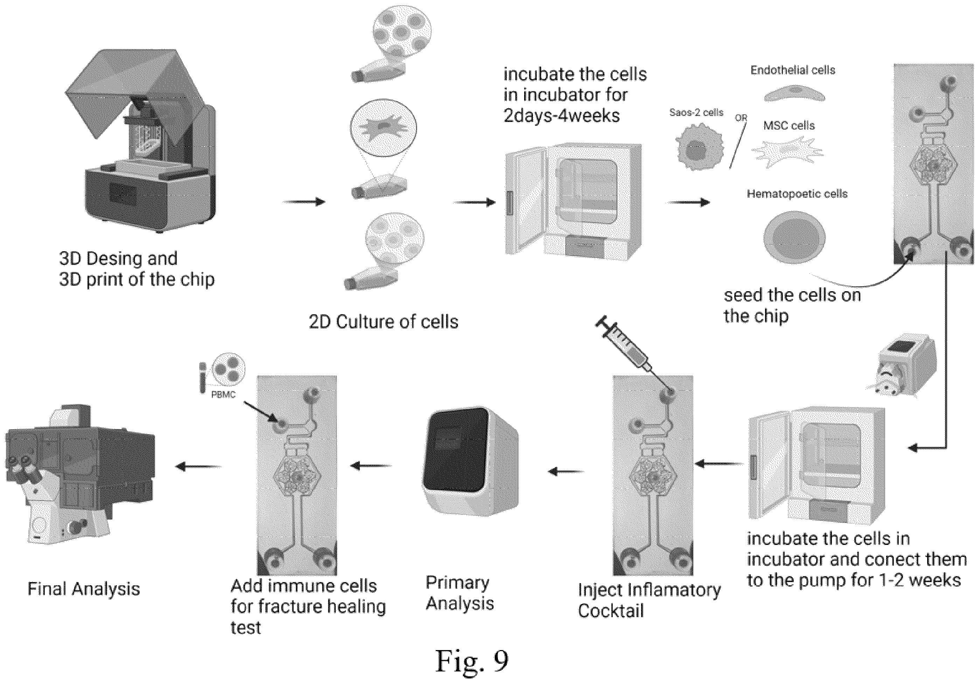

Fig. 9 shows a workflow from producing the device (chip) to culturing cells in the device and assessing the cultured cells or cell culture.

### Example 4: Establishing a high-throughput platform: Chips maintenance, monitoring and establishment of endpoint analysis tests

Example 4 discloses cell maintenance within the chip and subsequent analysis of cell morphology and transcriptomics.

The optimal time of cultivation of the cells in the chip is assessed, e.g. how long cells need to be cultivated in the chip to have desired properties and/or how long the cells can be maintained in the chips without losing the desired properties. This is tested by incubation and monitoring of cells cultivated in the chips according to Examples 2 or 3 for 1 week, 2 weeks, 3 weeks and 4 weeks in 5% CO₂ and 37 C° incubator and under the conditions described in Example 2 and 3 above. Quantitative and qualitative tests for endpoint analysis are used. The primary endpoint outcomes are cell viability, cell differentiation, bone matrix deposition and mineralization. Those can be assessed e.g. via flow cytometry, live cell imaging, ELISA and staining as described below.

The following test is useful to study the cells within the chip and to allow a conclusion, e.g. when studying fracture healing using an inflammatory cocktail or when studying the effect of a drug on the cells.

### Cell characterization by Flow cytometry and qPCR

Cells can be characterized by the following cell line specific markers. HUVECs (CD31 as positive marker) and (CD34, CD14, and CD90 as negative markers) (0.5 mg/mL, 5 µL of antibody per 1 × 106 of cells, 1:20). Saos-2 cells Epidermal growth factor (EGF); transforming growth factor beta (type 1 and type 2) Expression markers

### Immunofluorescence Staining

- Osteopontin, Osteocalcin, BMP-2, Collagen IV, Fibronectin, CD34, CD31 antibodies. These antibodies allow for detection of specifically differentiated.
- The cells are incubated with primary antibodies against ALP ((C-8): sc-373737, Santa Cruz), COL1A ((COL-1): sc-59772, Santa Cruz), RUNX2 ((27-K): sc-101145, Santa Cruz), OCN ((ABOC-5021): sc-73464, Santa Cruz) or OPN ((LFMb-14): sc-73631, Santa Cruz) at 4 °C overnight and then incubated with a donkey anti-mouse IgG H&L secondary antibody (Alexa Fluor^{®} 647, ab 150107) for 1 h at room temperature.
- F-actin are used to stain the cytoskeleton (green) for 1 h, and DAPI will be used to stain the nucleus (blue) for 5 min at room temperature. The cells will be washed gently with PBS and then observed under a laser confocal microscope (TCS SP8, Leica, Germany). This allows to study cell morphology.

### Cell matrix and mineralization staining

These stainings allow to study the cell matrix and mineralization that takes place inside the chip. This is an important indication for studying how bone heals and regrows after, e.g. an inflammatory situation mimicking a fracture or in response to a drug.
- Alcian blue staining (Sigma) for detection of proteoglycans
- Von Kossa staining (5% silver nitrate) and alizarin red staining for detection of mineralization and bone formation
- ALP staining for detection of alkaline phosphatase which is highly expressed in cells of mineralized tissue and plays a critical function in the formation of hard tissue

### Molecular studies

- RNA extraction from cells for studying changes in the transcriptome of cells, e.g. via RNA sequencing
- RNA may also be extracted to study expression of individual genes via quantitative real-time PCR analysis
- The following genes are of particular interest for gene expression studies:
   ∘ Cell adhesion genes
      ▪ Expression levels of cell adhesion genes CD 44, integrin subunit beta 1 (ITGB1) (CD 29), cell surface antigen (Thy1) (CD 90), activated leukocyte adhesion molecule (ALCAM) (CD 166) and vascular cell adhesion molecule-1 (VCAM1)/(CD106)
   o Osteogenic genes
      ▪ The expression of runt-related transcription factor (RUNX2), key to osteoblastic differentiation,
      ▪ The expression of other osteogenic lineage markers alkaline phosphatases (ALP)
      ▪ COL1A1, and phosphoprotein 1 (SPP1) expression.
   ∘ Osteogenic-related mRNA expression of BMSCs
      ▪ Including runt-related transcription factor 2 (Runx-2)
   o Osteocalcin (OCN), collagen type 1 (Col-1), and Osteopontin (OPN),
      ▪ On the scaffolds can determine by real-time quantitative PCR
   o CCR7, iNOS, ARG-1, and CD206, related osteogenic genes
      ▪ Like bone morphogenetic protein-2 (BMP-2), collagen 1 (COL1), endothelial nitric-oxide synthase (eNOS), and vascular endothelial growth factor (VEGF) can be detected using RT-qPCR.
   o Expression of SCF
      ▪ SCF plays a role in the formation of blood cells. Stem cell factor (SCF) is the ligand of the c-Kit oncogene and is expressed by various structural and inflammatory cells in the airways. Binding of SCF by the c-Kit receptor leads to homodimerization of the receptor and the activation of signalling pathways such as PI-3, PLC-gamma, Jak/STAT, and MAP kinase pathways. SCF expression leads to the induction of mast cell survival and the expression and release of histamine, pro-inflammatory cytokines and chemokines.
   ∘ Pluripotency genes Sox2 and Nanog and of the osteogenic marker genes Cbfa1, Sp7, and Ibsp
      ▪ The addition of Wnt3a to the osteogenic differentiation medium increases their upregulation. Wnt3a is a protein that plays a crucial role in the Wnt signaling pathway, which is important for regulating cell growth and differentiation and Osteogenic differentiation medium is a specialized culture medium used to induce stem cells to become osteoblasts. When Wnt3a is added to this medium, it enhances the differentiation process. Specifically, it increases the expression (or upregulation) of genes associated with osteoblasts. This means that the cells are more effectively turning into bone-forming cells, which is beneficial for research and therapeutic applications related to bone regeneration and repair.
   ∘ RUNX-2 and SPP1 (osteopontin)

### Quantification of cytokines and growth factors

- Enzyme-linked immunosorbent assay (ELISA)
   o The quantification of bone morphogenetic protein-2 (BMP-2) and vascular endothelial growth factor (VEGF) are assessed by ELISA assays. Commercially available human BMP-2 and VEGF ELISA kits (Abcam) are available and are performed according to the manufacturer's recommendations.
- Multiplex detection assay
   o A bone panel for detection of multiple bone related marker genes was used to quantify the levels of osteopontin (OPN) and osteoprotegerin (OPG) and a human hematopoietic stem cell panel, both from BioLegend. The LegendPlex panels were performed according to the manufacturer's protocols, and the measurements were read in a Flow Cytometer. Alternatively, expression levels can be assessed with QPCR.

### Cell Proliferation

Cell proliferation is studied using a lactate dehydrogenase (LDH) assay (Sigma-Aldrich, ref. nr. TOX7-1 KT) and can be assessed by a Spectrophotometer.

The cell proliferation reagent (MTS assay) is be used to study cell proliferation which can be assessed by a spectrophotometer and e.g. the kit CellTiter 96_AQueous Non-Radioactive.

### Calcium Deposition

Matrix calcium deposition is measured using Alizarin Red S (ARS) staining.

### Collagen Synthesis

Total collagen content is detected using Direct Red 80 (DR80) staining.

### Live Cell Tracking and imaging by Automated Live Cell Imaging System (AutoLCI)

Live cell tracking and imaging can be carried out using the Staining with CellTracker^{™} Red CMTPX (Thermo Fisher Scientific, United Kingdom), and/or Live/dead Fixable Far Red Dead Cell Stain (Thermo Fisher Scientific) according to manufacturer's instructions.

The above analytical methods that are carried out on the cells within the chip, or cells extracted from the chip provides invaluable input regarding culture conditions, as well as the effect of drugs or an inflammatory situation mimicking fracture healing on cells in the chip. Thus, they are useful for studying bone diseases, such as bone fracture or bone cancer or osteoporosis, within the microfluidic device and asses the efficacy of cell therapy or drug therapy on those diseases.

### Example 5: Establishing a cell culture of primary immune cells on the microfluidic device

One aim of the invention is to provide a microfluidic chip in which the effect of a patient's own immune cells can be studied on a bone condition, e.g. an inflammatory situation mimicking a fracture healing of a bone or bone cancer and the like. For this, primary human blood-derived immune cells are cultured and seeded into the microfluidic device (chip) without any other cells as a proof of concept that the patient derived immune cells can establish a viable culture within the chip. Thus, they are seeded into chips which were not previously seeded with other cells like Human mesenchymal stem cells (hMSCs) or Saos-2 cells yet (which may give rise to/differentiate into osteoblasts and endothelial cells). Blood which has been obtained from different donors is used. First, the different isolation methods of the blood which has been obtained from different blood donors (density gradient centrifugation, cell sorting, magnetic bead associated sorting) is compared. In general, the establishing of a cell culture and seeding the same into the microfluidic chip, does not need to comprise a blood draw from a patient. The already drawn blood may be provided by a hospital. The amount and viability of the derived immune cells (PBMC) is analysed by FACS. The next step is to establish the seeding of the cells to the chip to ensure maximum survival. For that step, different cell culture mediums and environmental conditions (humidity, temperature, CO₂, O₂) for the patient derived blood cells is tested. The most appropriate culture media and environment is determined and analysed by FACS by studying the activity, viability and amount of immune cells after 1 day, 3 days, 7 days, 14 days and 21 days. Possible appropriate media that can be used in this context are standard media like RPMI1640 and DMEM high glucose. The cells are then cultured and seeded into the chip as explained in Example 2.

After successfully seeding and formation of bone micro tissue (see Example 3 and 4) a pro inflammatory "fracture" cocktail is introduced to the cells in the chip for 24h. This pro-inflammatory cocktail simulates the inflammatory situation present at a bone fracture healing environment in vivo. This allows to investigate the impact of e.g. therapeutic agents, such as patient derived immune cells, on the inflammatory situation at a bone fracture site. Cells can be analyzed by by RNASequencing and endpoint mineralization as described in Example 4. This verifies the functionality of the chips for accommodating patient derived primary immune cells. Patient derived primary immune cells may be obtained from peripheral blood mononuclear cell (PBMC) of a patient. The inflammatory cocktail comprises 500 ng/mL C3a, 10ng/mL C5a, 250 pg/mL Il-1beta, 500 pg/mL IL-6, 150 pg/mL IL-8, 10 ng/mL TNF-α. Individual cytokines is be included as controls.

The role of immune cells (CD45RA⁺) and mesenchymal stem cells during the different phases of fracture healing is described in the art; see, inter alia Ehnert et al. (2021). Effects of immune cells on mesenchymal stem cells during fracture healing. World Journal of Stem Cells, 13(11), 1667.

### Example 6: Establishing and seeding a co-culture of primary immune cells with cell lines of Example 2

The microfluidic device of the present invention is particularly advantageous, since it allows for culturing patient derived primary immune cells with bone cell lines, e.g. those of Example 2. The advantage of this is that the impact of the patient's own immune cells can be studied for different bone disease. For example, the impact of patient's own immune cell on a bone fracture healing is studied. Accordingly, chips which have already been seeded with the human osteoblasts (hBM-MSCs) and endothelial cell (HUVEC) or (Saos-2 cell line with endothelial cells (HUVEC)) as explained in Example 2 or 3, is contacted with the inflammatory cocktail of Example 5 for 24h. Afterwards, the chip is seeded with patient primary immune cells as described in Example 5. This allows to study how the patient's own immune cells contribute to bone fracture healing. Furthermore, as an optional step one or more drugs can be provided before, together or after, or instead of seeding the chip with the primary immune cells of a patient. This additionally allows to study the contribution of a drug and/or the patient's own primary immune cells to fracture healing. The outcome is measured as explained in Example 4 and/or 5. For example, the immune cell-bone cell interactions can be studied by RNASequencing analysis and endpoint mineralization. As controls, chips comprising human osteoblasts (hBM-MSCs) and endothelial cell or (Saos-2 cell line) and endothelial cells as explained in Example 2 which have been contacted with the pro-inflammatory cocktail but without patient derived primary immune cells are used. Individual cytokines may also be included as controls.

Additionally, to the above, the variability of the impact that patient derived primary immune cells have on fracture healing is studied. The aim is to gain knowledge about the influence of donor-derived variables on the functionality of the chips, the immune cell-bone cell interactions and the endpoint mineralization. For this, isolated immune cells from males vs. females; young vs. old (18-40 years vs. 65-99 years); and from donors with different comorbidities (e.g. diabetes, osteoporosis, smoking) are used. Cell survival and cell-cell interactions in the different donors as well as endpoint analysis in the different donors (mineralization) are investigated for example under the conditions explained above, i.e. fracture healing and/or drug provision. Furthermore, the effects of the pro-inflammatory cocktail and its influence of donor variability is tested. The outcome is measured as explained in Example 4 and/or 5. For example immune cell-bone cell interactions verified by RNASequencing analysis and endpoint mineralization.

### Example 7: Drug testing

As briefly mentioned above, an advantage of the microfluidic device of the invention is that drugs can be provided to the cells in the chip for example allowing to investigate their impact and efficacy on bone diseases, such as fracture healing. The aim of this example is to establish a streamlined drug testing platform with the seeded chips. For that, the chips of Example 2, 3, 5 and 6 may be used, i.e. chips comprising bone cell lines and/or patient derived primary immune cells.

Furthermore, the knowledge about donor variability obtained in Example 6 can be incorporated. To study for example fracture healing, a drug comprising substances which are known to inhibit/activate bone formation by interacting with osteoimmunological mechanisms (inhibition e.g.: Sclerostin, Dkk1, C5a, Histamin-receptor agonist, TNF-α, CCL3; activation e.g. Wnt3a, Wnt1, PGE2, TGFb, IL-17) can be used.

Following this approach also the efficacy of a drug e.g. an anti-cancer drug may be assessed. For this, patient derived cancer cells can be seeded into a chip and the efficacy of an anti-cancer, specifically a bone cancer, can be tested, thereby allowing a personalized drug recommendation for the individual patient.

The same approach can be done for other bone disease such as fracture healing, for example a fracture healing drug may be provided as explained in Example 6 to test its efficacy on fracture healing with or without primary immune cells of the patient. For example, only the one or more drug may be applied (e.g. without immune cells). In general, also the patient derived primary immune cells may be testedas a candidate therapeutic compounds, e.g. their efficacy in fracture healing may be tested as explained in Example 6. Furthermore, their efficacy to combat bone cancer cells may be tested as described for an anti-cancer drug above, with the difference that the cells act as the drug. Such a cell therapy may also comprise allogeneic or autologous cell therapies, such as a CAR-T cell therapy. The efficacy of such cell therapies can readily be tested in the microfluidic device of the present invention.

The outcome is measured as explained in Example 4 and/or 5. For example immune cell-bone cell interactions is verified by RNASequencing analysis and endpoint mineralization. The efficacy of a drug or cell therapy to a bone cancer can be assessed by measuring the total number of cancer cells over time. The number of cancer cells can be quantified e.g. via flow cytometry. For example, apoptosis (cell viability) can be assessed using ELISA, cell imaging, flow cytometry and RNA sequencing. Furthermore, new drugs can be tested using the microfluidic chip of the present invention. Such new drugs may be obtained from conventional libraries (e.g. the bioactive compound series or natural product series from MedChemExpress) to investigate immune cell-bone cell interactions and endpoint mineralization. Their efficacy to different bone diseases such as cancer or fracture healing can be assessed together or without the respective primary immune cells of a patient. This has the potential to investigate novel enhancers of bone formation and bone regeneration. Furthermore, this allows for personalized medicine. Patient derived cells may be directly seeded into the chip or may be seeded on a chip already comprising other bone cells as described above. The efficacy of the patient derived cells and/or drugs can then be assessed specifically for the cells of the individual patient. This allows the medical practitioner to make an informed decision about the most promising treatment for their individual patent.

One example how this can be realized is provided herein. The medical practitioner provides a diagnosis of a disease and provides the relevant patient derived cells to be studied. The cells are cultured and seeded within the chip as explained above alone or in co-culture with other relevant cell lines. A drug or a cell therapy which can comprise an autologous or allogeneic cell therapy, such as a CAR-T cell therapy can then be tested on the cells within the microfluidic device. The efficacy of the drug or cell therapy is assessed and reported back the medical practitioner which based on the results can provide the therapy with the highest efficacy to the individual patient resulting in an improved treatment.

The above examples illustrate an exemplary way of carrying out the invention. They are not to be construed as limiting the scope of the invention. The skilled person considering the present invention is capable to use the microfluidic device of the present invention for seeding with any cell line of interest or any patient derived cells. Furthermore, the skilled person is capable of selecting appropriate substances and/or drugs to be tested in the advantageous environment of microfluidic device of the present invention.

### Material and methods

### 1. Statistical analysis

Statistical analysis is performed using the SPSS program. Student's t-test and ANOVA for multiple groups are used in analysis to evaluate the similarity between experiments. P <0.05 ae used as the statistical significance level. Statistical evaluation; Conducting statistical analysis using SPSS and ANOVA - Contribution to success is 5% (i.e. quantifiable portion (5%) of the project's success or outcome to the statistical analysis conducted in SPSS, implying that it was a helpful but relatively small part of the broader effort).

### 2. Deep learning and AI algorithm

The image database containing β-catenin, nucleus, and merged fluorescent images acquired from the bone-on-a-chip platform is used for deep learning-based image analysis. There are two labeled groups in the image data: the drug-treated group (420 images) and the nondrug group (424 images). For data augmentation, one image is segmented into four images, and each image is randomly transformed using random zoom (zoom range of 0.5) and horizontal flip. For an algorithm classifying the images by group, a convolutional neural network (CNN) is used with fully connected layers. The entire network architecture consists of three convolution layers, three pooling layers, a dropout layer, a flattened layer, and two fully connected layers. The 2D convolution layer (Conv2D) extracted features from the input images and generated a feature map. Conv2D is followed by rectified linear unit (ReLU) activation. For feature extraction, 2D max-pooling layers (MaxPooling2D) extract the maximum value from the feature map. In addition, dropout layers are applied randomly to a neural network during the training process to prevent overfitting. The flattened layer converts the features of the extracted data into 1D data. Finally, the classification algorithm is completed by connecting all the virtual neurons of the previous layers through two fully connected layers. Images corresponding to 10% of the total data are randomly selected and treated as the test set. The remaining images are used as a training set. The test set is used to evaluate the final performance of the classification algorithm. During the training process, a 10-fold cross-validation method is used to test network performance. The accuracy and area under the receiver operating characteristic curve (AUC ROC) are measured to evaluate the diagnostic ability of this algorithm using the dataset. ROC curves are created by plotting the sensitivity and the false positive rate (1-specificity) based on the detection probability obtained by the classification algorithm.

### 3. Design, preparation, modification and manufacturing of the microfluidic device (chip/BONEoC platform) as well as Characterization of physicochemical properties of hBONEoC platform

The microfluidic device is designed and prepared according to Example 1.

3D-printed chip is observed by a inverted microscope (EVOS kern XL- Thermo Fisher), and the ImageJ software is used to analyze the photos for calculation of pore size and fiber diameter.

### 4. Cell culture

### 4.1 HUVEC culture

HUVECs purchase from Lonza and expanded in monolayer culture with endothelial medium (EM; endothelial growth basal medium 2 supplemented with an EGM-2 Bullet kit (Lonza)). The EM change every 3 d until 80% confluence. Cells are used at passage 5.

### 4.2 Saos-2 culture

The base medium for this cell line is ATCC-formulated McCoy's 5a Medium Modified, Catalog No. 30-2007. To make the complete growth medium, add the following components to the base medium: fetal bovine serum to a final concentration of 10%., penicillin/streptomycin (1%), and Glutamax^{™} (1%). Cells between passages 3 and 5 are used in the experiments.

### 4.3 Human CD34⁺ HSPCs

Cells test via flow cytometry-as described below-and the cells are only used if a minimum of 95% of the cells are positively stained for CD34⁺. HSPCs are maintained in serum-free HPC Expansion Medium XF (PromoCell GmbH, Heidelberg, Germany) supplemented with Cytokine-Mix E (containing TPO, SCF, Flt-3 ligand and IL-3) and 1% (v/v) penicillin/streptomycin.

### 4.4 Hematopoietic immune cells

Hematopoietic CD45RA+ immune cells are extracted from the peripheral blood according to previously published isolation protocols and injected into the chips with the ambient flotation system. Differentiation of the CD45RA+ cells into the different immune cell populations can be modified by adding pro-inflammatory cytokines.

### 4.5 PBMC Isolation

### PBMC Isolation and Characterization for Cell Sorting

1. **Isolation**
   **∘ Blood Collection:** Use heparinized tubes.
   **∘ Density Gradient Centrifugation:**
      ▪ Dilute blood with PBS (1:1).
      ▪ Layer over *Ficoll-Paque* or similar medium.
      ▪ Centrifuge at 400-500 g for 30-40 min at room temperature (no brake).
      ▪ Collect PBMC layer between plasma and Ficoll.
   ∘ **Wash:** Rinse cells with PBS or RPMI 1640, centrifuge at 300 g to remove debris and platelets.
2. **Culture Medium**
   ∘ **RPMI 1640** with 10% FBS, 1% Penicillin-Streptomycin, and 2 mM L-glutamine.
3. **Characterization for Sorting**
   ∘ **Staining:** Label cells with fluorescent antibodies for specific markers:
      ▪ *T Cells:* CD3, CD4, CD8
      ▪ *B Cells*: CD19
      ▪ *NK Cells:* CD16, CD56
   o Flow Cytometry: Sort and analyze based on fluorescence markers.

### 5. BONEoC platform cell seeding and mineralisation protocol / Cell seeding and culture

BONEoC are sterilised prior to cell seeding as described in Example 1. All the following steps are performed in a class II biological safety cabinet. Briefly, scaffolds are immersed in 70% v/v ethanol (aq.) and incubated for 20 min at RT; scaffolds are washed three times with 1× PBS. The chips are placed to further exposed to UV-C light for 30 min each side. After sterilisation, 1× 10⁵ Saos-2 cells are suspended in 50 µL with 50 µL matrigel volume of complete media and gently pipetted on the top of each chip. Scaffolds are incubated for 30 min (37 °C, 5% CO₂) allowing cell adhesion, then the chips are incubated for 24 hours. Afterwards, the chip are connected to the peristaltic pump with the flow rate of 4µl/min. Cell culture media are replaced after 4 days, and after 7 days of culture (37 °C, 5% CO₂), the culture media are changed to Osteoblast mineralisation media (C-27020, PromoCell) to induce mineralisation and changed thereafter every 4 days and until the end point (i.e. day 28).

### 6. In vitro cytotoxicity for Resin chip fabrication

The Calcein-AM/PI double staining kit (Sigma-Aldrich) is applied to evaluate the viability of hBMSCs seeded on chip for 96 h. Fluorescence images are acquired using by Automated Live Cell Imaging System (AutoLCI).

### 7. Live cell imaging

AutoLCI is an automated live cell imaging system that is equipped with an advanced fluorescence and bright field microscopy, autofocusing and real time multi-position imaging technology for a ^{h}BONEoC.

This device allows to ^{h}BONEoC positioning in an incubator providing improved cell viability as there is less disturbances over the course of your experiment reducing chances of cellular abnormality. AutoLCI Scan to control the actual operation of AutoLCI device which includes real-time cell monitoring and time-lapse imaging functions.

### 8. Cell viability: (Live/Dead assay)

To evaluate the biocompatibility of scaffolds for a long-time incubation, Saos-2 cells are seeded at the concentration of 1 × 10⁴ cell/ mL and cultured for 1, 4, 7 and 10 days. Afterwards, the staining solution of Calcein AM/PI (Beyotime, China) prepared in supplement-free media is added into each well and further incubated for 30 min at 37 ^{°C} in the dark. The live (green) and dead (red) cells are observed via an AutoLCI system.

### 9. Cell adhesion: (Adhesion/Cell Spreading assay)

SaoS-2 cells are seeded at the concentration of 1 × 10⁴ cell /mL and cultured for 4, 12 and 24 h. Then the chip is treated with 10% CCK-8 solution (Dojindo, China) at 37 ^{°C}. After 2 h incubation, CCK-8 included medium is collected and its absorbance is measured following the manufacturer's manual and normalized to surface area of chip.

### 10. Cellular morphology analysis

After incubation of a desired period or treatment, Saos-2 cells are fixed with 4% paraformaldehyde and washed several times with PBS. After permeabilization with 0.1% of Triton X-100 for 30 min, they are blocked by 0.1% BSA for 1 h, stained by Actin Tracker (Beyotime, China) or Dil Stain (Thermo Fisher) for 1 h, and the nuclei are stained with DAPI. All samples are protected by antifade reagent and fluorescence images are taken by an AutoLCI system. ImageJ software is used for analysis of cell area and circularity.

### 11. Gene Expression Profiling RNA isolation/ RT-qPCR

For total RNA extraction, samples were first pulverized by hammering in a liquid nitrogen-cooled biopulverizer (59012MS, BioSpec Products), and the powder obtained is added to Trizol (Invitrogen). RNA is then purified using the RNeasy Plus mini kit (Qiagen, Hilden) following the manufacturer's protocol. Reverse transcription with the SuperScript III kit (Invitrogen) and random hexamer primers is used to prepare cDNA. Quantitative real-time PCR is performed on the StepOnePlus thermocycler (Applied Biosystems) with SYBR Green Reaction Mix (Applied Biosystems). Gene expression levels of alkaline phosphatase (ALP), runt-related transcription factor 2 (RUNX2), osteocalcin (OCN), bone sialoprotein II (BSP2), osteopontin (OPN), VEGF, and collagen type I (COL1) are analyzed. 18S rRNA is used as a housekeeping gene. Exemplary primer sequences are reported in table 1 or 2. Gene expression fold of change is calculated by the comparative cycle threshold (CT) method, using expression levels at day 0 as the reference for the 2-ΔΔCT calculation. A two-way ANOVA test with Tukey's post hoc analysis (multiple comparison) is used to pinpoint statistical differences of gene expression.

**Table 1**

| Gene | Forward sequence 5' - 3' | Reverse sequence 5' - 3' |
|---|---|---|
| ALP | ATCTTTGGTCTGGCCCCCATG | AGTCCACCATGGAG CATTCT CTC |
| RUNX2 | CAACCACAGAACCACAAGTGCG | TGTTTGATGCCATAGTCCCTCC |
| OCN | TCACACTCCTCGCCCTATTG | GAAGAGGAAAGAAGGGTGCC |
| BSP2 | CGAATACACGGGCGTCAATG | GTAGCTGTACTCATCTTCAT GGC |
| OPN | TCACCAGTCTGATGAGTCTCACCATTC | TAGCATCAGGGTACTGGATGTCAGGT |
| VEGF | AGGGCAGAATCATCACGAAGT | AGGGTCTCGATTGGATGGCA |
| COL1 | TAA AGG GTC ACC GTG GCT | CCA ACC ACA TTG CCA TCA |
| 18S | CCATCCAATCGGTAGTAGCG | GTAACCCGTTGAACCCCATT |

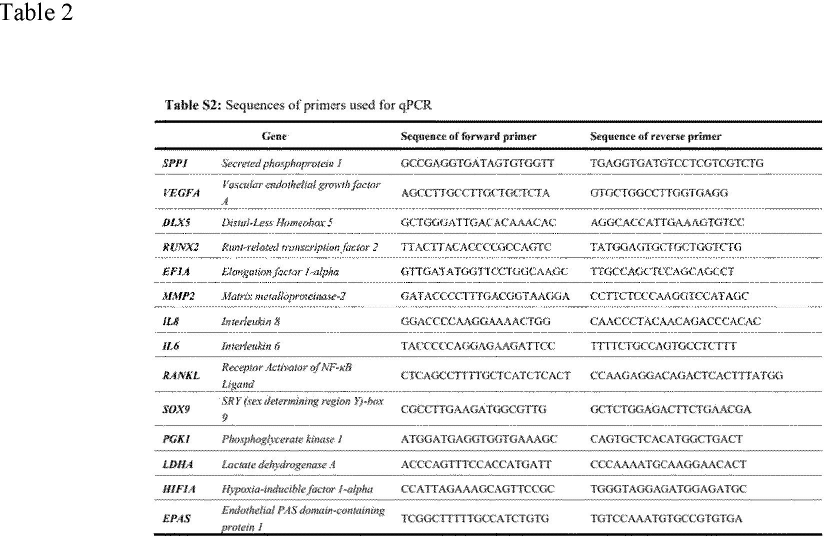

### 12. Alizarin-red staining and calcium detection quantification

Saos-2 are continuously cultured in chips in an osteogenic medium for 21 days to induce osteogenesis. Saos-2 are washed three times with PBS, after which 10% tissue, fixative was used to fix the cells. Then, 0.5% alizarin red staining dye is applied to stain cells. And the red mineralized nodules were observed using a charge-coupled device microscope and the absorbance is evaluated at 570 nm.

### 13. Alkaline phosphatase activity (ALP)

### Cell osteogenic differentiation

### ALP staining

An ALP staining kit is used for Saos-2 staining. Saos-2 are washed twice, and 10% 10% tissue fixative is used to fix the cells. BCIP/NBT substrate is applied to incubate cells for 24 h. Colorimetric alteration is calculated with a charge-coupled microscope and absorbance was evaluated at 405 nm.

### 14. Vascularized bone construct fabrication

A 2:1 ratio HUVECs:Saos-2 suspension is prepared at a final concentration of 25*10³ cells ml⁻¹ in a 1:1 solution of matrigel and cell mix. The macropores of each chipwere filled with 50 µl of HUVECS: Saos-2- and 50 µl of matrigel.

### 15. Enzyme-Linked Immunosorbent Assay (ELISA) Analysis

Measurement of cytokine levels by ELISA is conducted on primed macrophage cultures (after 24 h) and macrophage coculture stimulations (after 72 h). Supernatants are collected and stored at - 20 °C for cytokine measurements. The concentrations of TNF-α, IL-6, OSM, SDF-1α (human DuoSet^{®} kits, R&D Systems), and bone morphogenetic protein 2 (BMP-2, BGK8C060, Peprotech) are measured using ELISA kits, according to the manufacturer's instructions. Culture consisting of non-primed macrophages are used as control.

### 16. Immunofluorescence staining and image acquisition

The morphology of Saos-2 cells on chip is analysed by immunofluorescence staining using DAPI (Thermofisher, UK) and Alexa Fluor^{™} 488 Phalloidin (A12379, Thermofisher, UK) for nucleus and F-actin, respectively. Briefly, each scaffold was fixed with 4% v/v paraformaldehyde for 10 min and permeabilised with 0.1% Triton-X for 10 min at RT. After washes the scaffolds are incubated with a mix of DAPI (1 µg/mL) and Phalloidin (1:80) in 1× PBS for 30 min at RT and in the dark. Samples are washed three times with 1× PBS and stored immersed in 1× PBS at 4 °C in the dark. Collagen I and osteocalcin immunofluorescence staining are performed on the chip to detect any deposition of extracellular matrix after 28 days of culture. For this staining, chips are fixed with 4% v/v paraformaldehyde for 5 min at RT, washed three times with 1× PBS and then incubated with blocking buffer (1% w/v BSA in 1× PBS) for 1 h at RT to avoid non-specific antibody binding. Chips are washed with 1× PBS, and then incubated with Osteocalcin monoclonal antibody (1:500 dilution in 1× PBS, MA1-82975, Thermofisher, UK) and Collagen-I polyclonal antibody (1:250 dilution in 1× PBS, PA5-95137, Thermofisher, UK) overnight (16 h) at 4 °C. After three washes with blocking buffer, samples are incubated with a solution of secondary antibodies Goat anti-rabbit 488 (1 :2000 dilution in 1 × PBS, A-11008, Thermofisher, UK) and Goat anti-mouse 594 (1:2000 dilution in 1× PBS, A-11005, Thermofisher, UK) for 30 min at RT following manufacturers instruction. Samples are washed three times with blocking buffer and stored immersed in 1× PBS at 4 °C in the dark.

Images of chips are acquired using the fluorescent inverted microscope (Leica DMI6000, Leica Microsystems, UK) coupled with a 5.5 Neo sCMOS camera (Andor, UK). The µManager software (v.1.46, Vale Lab, UCSF, USA) is used to control both microscope and camera, as well as to capture images. For acquisitions, a dry 10x objective (PL 10×/0.3 PH1, Leica), a dry 20× objective (PL 20×/0.5 PH2, Leica) and a dry 63× objective (PL 63×/0.9 PH2, Leica) with filter cubes (A4, I3 and N2.1) is used. All images are post-processed to remove background noise using ImageJ v1.49p.

### 17. Cytokine and chemokine quantification in outlet mediums

Supernatants are immediately stored at -80°C after 24 and 48 hours of co-cultivation. The concentration (pg/mL) of cytokines and chemokines is determined using multiplex suspension assay (Bio-Rad Laboratories, Hercules, CA, USA) following the manufacturer's instructions. The following cytokines and chemokines (lower detection limit) are measured: IL-1β (7.55 pg/mL), IL-2 (18.99 pg/mL), IL-4 (4.13 pg/mL), IL-5 (20.29 pg/mL), IL-6 (25.94 pg/mL), IL-7 (16.05 pg/mL), IL-8 (37.9 pg/mL), IL-10 (37.9 pg/mL), IL-13 (7.21 pg/mL), IL-17 (24.44 pg/mL), interferon-gamma (IFNy, 56.32 pg/mL), tumor necrosis factor-alpha (TNF-α, 59.53 pg/mL), monocyte chemotactic protein-1 (MCP-1, 27.02 pg/mL), macrophage inflammatory protein MIP-1β (6.27 pg/mL), granulocyte colony-stimulating factor (G-CSF, 50.98 pg/mL), and granulocyte-macrophage colony-stimulating factor (GM-CSF, 11.82 pg/mL).(1)

### 18. Analysis of cytokine expression

Bone marrow supernatants from naïve and pinned femurs and from contralateral and fractured femurs harvested at 2 hours, days 1, 2, 4, and 7 postfracture are analyzed for G-CSF, Eotaxin, IL1α, IL-1β, IL-2, IL-6, IL-17, VEGF-a, MCP-1, LIF, and KC cytokine expression. Cytokine levels of bone marrow supernatants are measured using an MCYTOMAG-70K-11 Mouse Cytokine Magnetic Multiplex Assay Kit (Milliplex) on a Bio-Plex MAGPIX Multiplex Reader (Bio-Rad) according to manufacturer's instructions (Luminex).

### 19. Flow Cytometry

Bone fragments used for cell analysis are first manually minced using a rongeur, thoroughly washed with PBS and then placed in low glucose modified Dulbecco's eagle medium (Life Technologies, Paisley, UK) containing 20% fetal calf serum and collagenase (3000 units per gram of bone, Worthington Biochemical Corporation, NJ, USA) for 4 h at 37 °C as previously described. After completion of the bone digest, the fraction containing cell suspension is recovered. To recover the remaining cells, bone fragments are further washed with PBS which is added to the digest cell fraction. After pelleting at 100× g for 1 min to eliminate bone debris, supernatant was spun again at 300× g for 10 min to pellet cells. For flow cytometry, cells are re-suspended in 30 µL of FACS buffer (PBS + 0.01% Sodium Azide) containing 1% Bovine serum albumin (as blocking agent) and incubated with varying combination of antibodies at 4 °C for 30 min (wrapped in aluminium foil). Cells are then washed with 200 µL cold FACS buffer. In total, 5 µL of live cell 7-aminoactinomycin D (7AAD) die (Abcam, Cambridge) are added and cells analysed by flow cytometry. Antibodies used are for Panel-1: anti-CD45, anti-CD3, anti-CD4, anti-CD8, anti-CD56, anti-CD19, anti-CD14 and for Panel-2: anti-CD45, anti-CD271, anti-CD90 and anti-CD73. All analyses are performed on LSRII 4 laser flow cytometer (BD-biosciences). Data analysis is done by using Attune^{™} NxT Flow Cytometer.

### 20. Metabolism study:Cell Proliferation Assay

Alamar blue assay (Deep Blue Cell Viability Kit, 424701, Biolegend) is used to analyze the proliferation of MDA-MB 231 and MDA-IV cells in hBONEoC at days 1, 3, and 7.Briefly, cell culture media is gently removed from each well, 400 µL of deep blue solution (10% v/v Deep blue viability reagent in complete cell culture media) is added to each well and incubated for 2 h (37 °C, 5% CO₂). After incubation, solution (200 µL) is taken from each well, transferred to a 96- well plate, and immediately measured with Synergy-2 (Biotek) plate reader (Ex 530-570 nm/Em 590-620 nm). The measurements are carried in triplicates (n = 3) for each experiment and values are plotted as mean ± standard deviation (SD) of N = 3 independent experiments.

### 21. In Vitro Fracture Model

A pro-inflammatory cocktail is used which is described to mimic the early fracture healing environment. This cocktail comprises 500 ng/mL C3a, 10ng/mL C5a, 250 pg/mL Il-1beta, 500 pg/mL IL-6, 150 pg/mL IL-8, 10 ng/mL TNF-α. Also, the single cytokines should be included as controls.

### 22. Immunoenzymatic Assays

Proinflammatory cytokines and protein analysis of main bone markers produced by cells within the chip model are measured by ELISA colorimetric tests for the quantification of Interleukin 6 (IL6, SEA079Hu Cloud-Clone Corp, USA), Interleukin 1ß (IL1ß, EK0392, Boster Bio, Pleasanton, CA, USA), Tumor Necrosis Factor α (TNF-α, SEA133Hu, Cloud-Clone Corp., Katy, TX, USA), Collagen alpha-1(I)chain (COLL1a1, SEA350Hu Cloud-Clone Corp., Katy, TX, USA) and Alkaline Phosphatase (ALP, SEB472Hu Cloud-Clone Corp., Katy, TX, USA). Supernatants are collected from all wells at all experiment times and maintained at -20 °C until used.

## Claims

1. A microfluidic device comprising
a main chamber configured to have a ring-like shape;
at least one cell introduction port in fluidic connection with the main chamber; and
at least one test port in fluidic connection with the main chamber.

2. The microfluidic device according to claim 1, wherein the main chamber comprises four or more subchambers, the subchambers being arranged to form the ring-like shape and wherein each of the subchambers has a polygonal shape, preferably wherein each of the subchambers has a hexagonal shape.

3. The microfluidic device of claim 2, wherein boundaries between adjacent subchambers are partially open allowing fluid and particle flow between adjacent subchambers.

4. The microfluidic device of claim 2 or 3, wherein each subchamber comprises a diameter of between 2 mm and 6 mm, preferably between 2.5 mm and 4 mm, most preferably between 3 mm and 3.5 mm and/or
wherein each subchamber comprises a volume of between 10 mm³ and 94 mm³, preferably a volume of between 16 mm³ and 42 mm³, more preferably a volume of between 23 mm³ and 32 mm³.

5. The microfluidic device of any one of claims 2 to 4, wherein each of the subchambers comprises multiple scaffold structures, each of the scaffold structures extending between a bottom surface of the subchamber to a top surface of the subchamber,
wherein each of the scaffold structures is shaped as a pillar having a polygonal cross-section, preferably wherein each of the scaffold structures is shaped as a pillar having a hexagonal cross-section,
wherein each subchamber comprises multiple scaffold structures having different cross-sectional diameters, preferably at least six scaffold structures comprising at least two different cross-sectional diameters, preferably at least nine scaffold structures comprising at least three different cross-sectional diameters,
wherein each of the scaffold structures has a cross-sectional diameter within the range of 150 micrometers to 800 micrometers, and
wherein each subchamber comprises between 3-18 scaffold structures

6. The microfluidic device of any one of claims 2 to 5, wherein each subchamber comprises at least one scaffold structure having a cross-sectional diameter within each of the following ranges: 150-250 µm, 250-450 µm, 450-650 µm; and 650-800 µm.

7. A method of producing a microfluidic device, wherein the microfluidic device comprises a main chamber configured to have a ring-like shape, at least one cell introduction port in fluidic connection with the main chamber, and at least one test port in fluidic connection with the main chamber;
preferably wherein the method includes 3D printing the microfluidic device using digital light processing (DLP) 3D printing.

8. A method for culturing cells in the microfluidic device of any one of claims 1 to 7.

9. The method of claim 8, wherein the cells are cells of the bone tissue and/or cells which are capable of differentiating into cells of the bone tissue, preferably wherein the cells which are capable of differentiating into cells of the bone tissue are stem cells; and/or
wherein the cells are endothelial cells or cells which are capable of differentiating into endothelial cells; and/or
wherein the cells are hematopoietic cells or cells which are capable of differentiating into hematopoietic cells.

10. The method of claim 8 or 9, comprising the following steps in the order (A) to (C):
(A) seeding the microfluidic device of any one of claims 1-7 with cells as defined in claim 9;
(B) culturing the cells; and
optionally (C) recovering the cells/cell culture and/or bone micro tissue or a cell culture having characteristics of bone micro tissue from the microfluidic device.

11. The method of claim 10, wherein about 5,000-50,000 cells are introduced into the microfluidic device, thereby seeding the microfluidic device, preferably 25,000 cells.

12. The method of any one of claims 8 to 11,
wherein cells which are capable of differentiating into bone cells are seeded/cultured;
wherein cells which are capable of differentiating into endothelial cells are seeded/cultured; and optionally,
wherein cells which are capable of differentiating into hematopoietic cells are seeded/cultured,
wherein the cells are cultured in the microfluidic device under conditions and/or for a time allowing the formation of bone micro tissue or a cell culture having characteristics of bone micro tissue.

13. The method of claim 12, wherein the cells which are capable of differentiating into bone cells are sarcoma osteogenic-2 (Saos-2) cells, preferably wherein the Saos-2 cells are capable of differentiating into osteocytes; or
wherein the cells which are capable of differentiating into bone cells are stem cells, preferably mesenchymal stem cells (MSCs); and
wherein the cells which are capable of differentiating into endothelial cells human umbilical vein endothelial cells (HUVECs); and/or
wherein the cells which are capable of differentiating into hematopoietic cells are hematopoietic stem cells, preferably hematopoietic stem and progenitor cells (HSPCs), more preferably CD34⁺ HSPCs.

14. The method of any one of claims 8 to 13, the method further comprising (preferably as step (CC)) contacting the cells/cell culture and/or bone micro tissue with a pro-inflammatory composition, comprising culturing the cells/cell culture and/or bone micro tissue under conditions and/or for a time allowing producing cells, bone micro tissue and/or a cell culture having characteristics of a bone fracture.

15. A method for analyzing a bone fracture and/or bone fracture healing comprising the method of any one of claims 8 to 14, the method further comprising (preferably as step (D)) assessing the efficacy of a therapeutic agent or a candidate therapeutic agent comprising contacting the cells, bone micro tissue and/or cell culture with a therapeutic agent or a candidate therapeutic agent; or
a method comprising assessing the efficacy of a therapeutic agent or a candidate therapeutic agent, the method comprising contacting the cells, bone micro tissue and/or a cell culture obtained or obtainable by the method of claim 14 with a therapeutic agent or a candidate therapeutic agent.

## Amended claims

### Amended claims in accordance with Rule 137(2) EPC.

1. A microfluidic device comprising
a main chamber configured to have a ring-like shape;
at least one cell introduction port in fluidic connection with the main chamber; and
at least one test port in fluidic connection with the main chamber.

2. The microfluidic device according to claim 1, wherein the main chamber comprises four or more subchambers, the subchambers being arranged to form the ring-like shape and wherein each of the subchambers has a polygonal shape, preferably wherein each of the subchambers has a hexagonal shape.

3. The microfluidic device of claim 2, wherein boundaries between adjacent subchambers are partially open allowing fluid and particle flow between adjacent subchambers.

4. The microfluidic device of claim 2 or 3, wherein each subchamber comprises a diameter of between 2 mm and 6 mm, preferably between 2.5 mm and 4 mm, most preferably between 3 mm and 3.5 mm and/or
wherein each subchamber comprises a volume of between 10 mm³ and 94 mm³, preferably a volume of between 16 mm³ and 42 mm³, more preferably a volume of between 23 mm³ and 32 mm³.

5. The microfluidic device of any one of claims 2 to 4, wherein each of the subchambers comprises multiple scaffold structures, each of the scaffold structures extending between a bottom surface of the subchamber to a top surface of the subchamber,
wherein each of the scaffold structures is shaped as a pillar having a polygonal cross-section, preferably wherein each of the scaffold structures is shaped as a pillar having a hexagonal cross-section,
wherein each subchamber comprises multiple scaffold structures having different cross-sectional diameters, preferably at least six scaffold structures comprising at least two different cross-sectional diameters, preferably at least nine scaffold structures comprising at least three different cross-sectional diameters,
wherein each of the scaffold structures has a cross-sectional diameter within the range of 150 micrometers to 800 micrometers, and
wherein each subchamber comprises between 3-18 scaffold structures

6. The microfluidic device of any one of claims 2 to 5, wherein each subchamber comprises at least one scaffold structure having a cross-sectional diameter within each of the following ranges: 150-250 µm, 250-450 µm, 450-650 µm; and 650-800 µm.

7. A method of producing a microfluidic device, wherein the microfluidic device comprises a main chamber configured to have a ring-like shape, at least one cell introduction port in fluidic connection with the main chamber, and at least one test port in fluidic connection with the main chamber;
preferably wherein the method includes 3D printing the microfluidic device using digital light processing (DLP) 3D printing.

8. A method for culturing cells in the microfluidic device of any one of claims 1 to 7.

9. The method of claim 8, wherein the cells are cells of the bone tissue and/or cells which are capable of differentiating into cells of the bone tissue, preferably wherein the cells which are capable of differentiating into cells of the bone tissue are stem cells; and/or wherein the cells are endothelial cells or cells which are capable of differentiating into endothelial cells; and/or
wherein the cells are hematopoietic cells or cells which are capable of differentiating into hematopoietic cells.

10. The method of claim 8 or 9, comprising the following steps in the order (A) to (C):
(A) seeding the microfluidic device of any one of claims 1-7 with cells as defined in claim 9;
(B) culturing the cells; and
optionally (C) recovering the cells/cell culture and/or bone micro tissue from the microfluidic device.

11. The method of claim 10, wherein about 5,000-50,000 cells are introduced into the microfluidic device, thereby seeding the microfluidic device, preferably 25,000 cells.

12. The method of any one of claims 8 to 11,
wherein cells which are capable of differentiating into bone cells are seeded/cultured; wherein cells which are capable of differentiating into endothelial cells are seeded/cultured; and optionally,
wherein cells which are capable of differentiating into hematopoietic cells are seeded/cultured,
wherein the cells are cultured in the microfluidic device for about 12 to about 48 hours so as to allow the formation of bone micro tissue or a cell culture having characteristics of bone micro tissue.

13. The method of claim 12, wherein the cells which are capable of differentiating into bone cells are sarcoma osteogenic-2 (Saos-2) cells, preferably wherein the Saos-2 cells are capable of differentiating into osteocytes; or
wherein the cells which are capable of differentiating into bone cells are stem cells, preferably mesenchymal stem cells (MSCs); and
wherein the cells which are capable of differentiating into endothelial cells human umbilical vein endothelial cells (HUVECs); and/or
wherein the cells which are capable of differentiating into hematopoietic cells are hematopoietic stem cells, preferably hematopoietic stem and progenitor cells (HSPCs), more preferably CD34⁺ HSPCs.

14. The method of any one of claims 8 to 13, the method further comprising (preferably as step (CC)) contacting the cells/cell culture and/or bone micro tissue with a pro-inflammatory composition, comprising culturing the cells/cell culture and/or bone micro tissue under conditions and/or for a time allowing producing cells, bone micro tissue and/or a cell culture having characteristics of a bone fracture.

15. A method for analyzing a bone fracture and/or bone fracture healing comprising the method of any one of claims 8 to 14, the method further comprising (preferably as step (D)) assessing the efficacy of a therapeutic agent or a candidate therapeutic agent comprising contacting the cells, bone micro tissue and/or cell culture with a therapeutic agent or a candidate therapeutic agent; or
a method comprising assessing the efficacy of a therapeutic agent or a candidate therapeutic agent, the method comprising contacting the cells, bone micro tissue and/or a cell culture obtained or obtainable by the method of claim 14 with a therapeutic agent or a candidate therapeutic agent.
